# EUROPEAN PATENT SPECIFICATION

(11) **EP 2 387 583 B1**
(45) Date of publication and mention of the grant of the patent: **19.09.2018**
(21) Application number: 10700549.8
(22) Date of filing: 14.01.2010
(51) Int. Cl.: C07K 16/10, C07K 16/18, A61P 31/12

(54) **PULMONARY ADMINISTRATION OF IMMUNOGLOBULIN SINGLE VARIABLE DOMAINS AND CONSTRUCTS THEREOF**
PULMONALE VERABREICHUNG VON VARIABLEN IMMUNGLOBULIN EINZELDOMÄNEN UND KONSTRUKTEN DAVON
ADMINISTRATION PULMONAIRE DES ANTICORPS À DOMAINE UNIQUE ET SES CONSTRUCTIONS

(30) Priority: 14.01.2009 US 144586 P; 15.10.2009 US 251879 P
(43) Date of publication of application: 23.11.2011
(73) Proprietor: Ablynx N.V., 9052 Ghent-Zwijnaarde (BE)
(72) Inventor: BOUCHE, Marie-Paule Lucienne Armanda, B-9050 Gentbrugge (BE); VANLANDSCHOOT, Peter, B-9881 Bellem (BE); SABLON, Erwin, B-1785 Merchtem (BE); DEPLA, Erik, B-9070 Destelbergen (BE); DE BUCK, Stefan, CH-4125 Riehen (CH); SAELENS, Xavier, B-8900 Ieper (BE); SCHEPENS, Bert, B-9031 Drongen (BE)
(74) Representative: Hoffmann Eitle
(86) International application number: PCT/EP2010/050414
(87) International publication number: WO 2010/081856

(56) References cited:
- WO-A1-2008/049897
- WO-A2-2006/059108
- WO-A2-2006/122257
- WO-A2-2007/049017
- Anonymous: "Alexion PharmaceuticalsTM antibody therapy shown effective in model for sever allergic asthma"[Online] 15 May 2006 (2006-05-15), XP002581082 Alexion Pharmaceuticals, Inc. News Retrieved from the Internet: URL:http://www.alxn.com/News/article.aspx? relid=216307> [retrieved on 2010-05-04]
- Graeme O'Neill (Australian Life Scientist): "Domantis lung study nets 'exciting' results"[Online] 27 October 2005 (2005-10-27), XP002581083 Australian Life Scientist Retrieved from the Internet: URL:http://www.lifescientist.com.au/articl e/142818/domantis_lung_study_nets_exciting _results/?fp=&fpid=&pf=1> [retrieved on 2010-05-04]
- DEONARAIN MAHENDRA P: "Recombinant antibodies for cancer therapy." EXPERT OPINION ON BIOLOGICAL THERAPY AUG 2008 LNKD- PUBMED:18613764, vol. 8, no. 8, August 2008 (2008-08), pages 1123-1141, XP009133149 ISSN: 1744-7682
- Robert Connelly: "Fully Human DomainAntibody Therapeutics:The Best of Both Worlds"[Online] 2005, pages 42-45, XP002581084 Drug Discovery Retrieved from the Internet: URL:http://www.iptonline.com/articles/publ ic/IPT_17_2005_p42_45.pdf> [retrieved on 2010-05-04]
- CHOUGULE MAHAVIR B ET AL: "Development of dry powder inhalers." RECENT PATENTS ON DRUG DELIVERY & FORMULATION 2007 LNKD- PUBMED:19075871, vol. 1, no. 1, 2007, pages 11-21, XP002581085 ISSN: 1872-2113
- HILL JIM ET AL: "Administration of antibody to the lung protects mice against pneumonic plague." INFECTION AND IMMUNITY MAY 2006 LNKD- PUBMED:16622253, vol. 74, no. 5, May 2006 (2006-05), pages 3068-3070, XP002581086 ISSN: 0019-9567
- AGNÃS MAILLET ET AL: "Aerodynamical, Immunological and Pharmacological Properties of the Anticancer Antibody Cetuximab Following Nebulization" PHARMACEUTICAL RESEARCH, KLUWER ACADEMIC PUBLISHERS-PLENUM PUBLISHERS, NE, vol. 25, no. 6, 21 November 2007 (2007-11-21), pages 1318-1326, XP019613059 ISSN: 1573-904X
- WEERS JEFFRY G ET AL: "Design of fine particles for pulmonary drug delivery." EXPERT OPINION ON DRUG DELIVERY MAY 2007 LNKD- PUBMED:17489656, vol. 4, no. 3, May 2007 (2007-05), pages 297-313, XP008092541 ISSN: 1742-5247

## Description

### Field of the invention

The present invention relates to an immunoglobulin single variable domain and/or construct thereof for use in a method wherein an immunoglobulin single variable domain (such as a Nanobody) and/or construct thereof are absorbed in pulmonary tissue. More particularly, the invention provides systemic delivery of an immunoglobulin single variable domain and/or construct thereof via the pulmonary route.

### Technological Background

Inhalation is an attractive delivery route to administer pulmonary local-acting agents in respiratory diseases (i.e. asthma, infections). Its use is also being adopted for the delivery of systemic-acting therapeutics whether they are small molecules or macromolecules (A. J. Bitonti and J. A. Dumont. Pulmonary administration of therapeutic proteins using an immunoglobulin transport pathway. Adv. Drug Deliv. Rev. 58:1106-1118 (2006).). As a hallmark of success, the first inhaled insulin powder, Exubera®, has recently been approved in Europe and US for the treatment of adult patients with type 1 or type 2 diabetes (L. Fabbri. Pulmonary safety of inhaled insulins: a review of the current data. Curr. Med. Res. Opin. 22 (Suppl 3) 21-28 (2006).).

For example, the systemic delivery of a conventional antibody, Cetuximab, a chimeric conventional antibody targeting the epidermal growth factor receptor (EGFR), is described in Maillet et al. (Maillet et al. Pharmaceutical Research, Vol. 25, No. 6, June 2008). Cetuximab was nebulized using three types of delivery devices and the immunological and pharmacological properties of cetuximab were evaluated. It was found that the conventional antibody aggregates and although they conclude that the antibody resists to physical constraints of nebulization as it remains biologically active, it is thought that the aggregated IgG will be lost for systemic uptake.

Furthermore, inhaled immunoglobulin single variable domain for local pulmonary delivery has been suggested for therapeutic use in lung diseases (see e.g. WO2007049017). However, the lung as a portal of entry for systemic drug delivery of immunoglobulin single variable domain and in particular Nanobodies and construct thereof has never been described in any details. Most immunoglobulin single variable domains for use as a biotherapeutic are still only developed as an intravenous injection delivery form. The use of these intravenous injection delivery forms is associated often with low patient compliance and high costs (application of injection often only by medical staff) in clinical practice. To improve compliance and a cost effect application, the development of non-invasive, easy to use delivery strategies such as pulmonary absorption of pharmaceuticals in particular biopharmaceuticals, e.g. such as immunoglobulin single variable domain, is clearly a medical need.

### Summary of the invention

The systemic exposure of immunoglobulin single variable domains such as a Nanobody and/or constructs thereof is often short as they are cleared from the systemic circulation rapidly. For example the *in vivo* half life of a monovalent Nanobody is about 45 minutes in mouse (Expert Opinion on Biological Therapy, Volume 5, Number 1, 1 January 2005 , pp. 111-124(14)). EP 1517921 proposes a strategy to prolong systemic exposure by making a construct that comprises an immunoglobulin variable domain against a antigen and an immunoglobulin variable domain against a serum protein with increased half-life. However, there is a clear need for alternative and/or improved strategies to prolong the half life of immunoglobulin single variable domains.

The generation of immunoglobulin variable domains, such as Nanobodies, has been described extensively in various publications, among which WO 94/04678, Hamers-Casterman et al. (Nature. 1993 Jun 3;363(6428):446-8) and S. Muyldermans (J Biotechnol. 2001 Jun;74(4):277-302 Review) can be exemplified. In these methods, camelids such as lamas are immunized with the target antigen in order to induce an immune response against said target antigen. The repertoire of Nanobodies obtained from said immunization is further screened for Nanobodies that bind the target antigen.

Currently, the art provides no method to systemically deliver immunoglobulin single variable domains and/or constructs thereof (e.g. such as Nanobodies and/or constructs thereof) via pulmonary tissue absorption in an effective amount. WO2007049017 describes an immunoglobulin single variable domain that was administered to the lungs but not delivered systemically in substantial amounts.

It is the objective of the present invention to overcome these shortcomings of the art. In particular it is an objective of the present invention to provide a immunoglobulin single variable domain and/or construct thereof for use in a method for delivering immunoglobulin single variable domains and/or constructs thereof to a mammal, e.g. a human. Furthermore, the methods described herein provide a sustained delivery of said immunoglobulin single variable domains.

The herein mentioned problems are overcome by the present invention. It has been found that administration of immunoglobulin single variable domains and/or constructs thereof can result in a sustained release of said immunoglobulin single variable domains and/or constructs thereof to the systemic circulation in an effective amount i.e. an amount that can have a prophylactic and/or therapeutic effect.

The present invention relates to the following.
An immunoglobulin single variable domain and/or construct thereof directed against at least one antigen for use in a method of treatment by delivering an effective amount of an immunoglobulin single variable domain and/or construct thereof to the systemic circulation of a human via the pulmonary route; wherein the method comprises the step of administering said immunoglobulin single variable domain and/or construct thereof to the pulmonary tissue at dosage intervals of once a day or longer.

The present invention relates to an immunoglobulin single variable domain and/or construct thereof for use as defined in the claims, wherein the process of administering comprises the step of forming an aerosol comprising said immunoglobulin single variable domain and/or construct thereof by an appropriate inhaler device.

The present invention relates to an immunoglobulin single variable domain and/or construct thereof for use as defined in the claims, wherein delivery of said immunoglobulin single variable domain and/or construct thereof to the systemic circulation is achieved with a bioavailability:
a) in case the immunoglobulin single variable domain and/or construct thereof consists of not more than 150 amino acid residues, the delivery of said immunoglobulin single variable domain and/or construct thereof to the systemic circulation is achieved with a bioavailability (compared to i.v. injection) that is at least 10% after administration of a single dose administration of said immunoglobulin single variable domain and/or construct thereof;
b) in case the immunoglobulin single variable domain and/or construct thereof consists of not more than 300 amino acid residues, the delivery of said immunoglobulin single variable domain and/or construct thereof to the systemic circulation is achieved with a bioavailability (compared to i.v. injection) that is at least 7.5% after administration of a single dose of said immunoglobulin single variable domain and/or construct thereof; or
c) in case the immunoglobulin single variable domain and/or construct thereof consists of not more than 450 amino acid residues, the delivery of said immunoglobulin single variable domain and/or construct thereof to the systemic circulation is achieved with a bioavailability (compared to i.v. injection) that is at least 5% after administration of a single dose of said immunoglobulin single variable domain and/or construct thereof.

The present invention relates to an immunoglobulin single variable domain and/or construct thereof for use as defined in the claims, wherein the terminal half life of said immunoglobulin single variable domain and/or construct thereof in the systemic circulation is longer than 5 hours.

The present invention relates to an immunoglobulin single variable domain and/or construct thereof for use as defined in the claims, wherein said immunoglobulin single variable domain and/or construct thereof is a V_{HH}, a humanized V_{HH}, and/or construct thereof.

The present invention relates to an immunoglobulin single variable domain and/or construct thereof for use as defined in the claims, wherein said immunoglobulin single variable domain and/or construct thereof is selected from the group of a V_{HH}, a humanized V_{HH}, a construct consisting of two V_{HH}'s or humanized V_{HH}'s directed against the same or different antigens optionally connected by a linker; and a construct consisting of 3 V_{HH}'s or humanized V_{HH}'s directed against the same or different antigens optionally connected by a linker.

Also disclosed is a method for providing to the systemic circulation of a mammal an effective amount of an immunoglobulin single variable domain and/or construct thereof that can bind to and/or have affinity for at least one antigen; wherein the method comprises the step of:
a) administering the immunoglobulin single variable domain and/or construct thereof to the pulmonary tissue of said mammal.

In a preferred method, the administration in said above mentioned method is performed by inhaling said immunoglobulin single variable domain and/or construct thereof in an aerosol cloud.

According to the invention, the immunoglobulin single variable domain can be a domain antibody, or an amino acid sequence that is suitable for use as a domain antibody, a single domain antibody, or an amino acid sequence that is suitable for use as single domain antibody, a "dAb", or an amino acid sequence that is suitable for use as a dAb, or a Nanobody, including but not limited to a V_{HH} sequence, and preferably is a Nanobody.

According to the invention, the construct comprising at least one immunoglobulin single variable domain can be a construct or polypeptide designed from the above mentioned sequences.

In a preferred embodiment the immunoglobulin single variable domain and/or construct thereof used in the above mentioned method is a Nanobody and/or a construct thereof. In a further similar preferred aspect, i.e. when using a Nanobody and/or a construct thereof, the method includes effective local pulmonary delivery of said Nanobody and/or a construct thereof.

According to the description, inhaling of the aerosol cloud can be performed by an inhaler device. The device should generate from a formulation comprising the immunoglobulin single variable domain and/or construct thereof an aerosol cloud of the desired particle size (distribution) at the appropriate moment of the mammal's inhalation cycle, containing the right dose of the immunoglobulin single variable domain and/or construct thereof ("Pulmonary Drug Delivery", Edited by Karoline Bechtold-Peters, Henrik Luessen, 2007, ISBN 978-3-87193-322-6, page 125).

The application also describes uses, formulations and devices suitable in the performance of the methods of the description.

### Detailed description

The present invention encompasses, but is not limited to, the subject matter of the appended claims and preferred aspects as described herein.

### A) Definitions

Unless indicated or defined otherwise, all terms used have their usual meaning in the art, which will be clear to the skilled person. Reference is for example made to the standard handbooks, such as Sambrook et al, "Molecular Cloning: A Laboratory Manual" (2nd.Ed.), Vols. 1-3, Cold Spring Harbor Laboratory Press (1989); F. Ausubel et al, eds., "Current protocols in molecular biology", Green Publishing and Wiley Interscience, New York (1987); Lewin, "Genes II", John Wiley & Sons, New York, N.Y., (1985); Old et al., "Principles of Gene Manipulation: An Introduction to Genetic Engineering", 2nd edition, University of California Press, Berkeley, CA (1981); Roitt et al., "Immunology" (6th. Ed.), Mosby/Elsevier, Edinburgh (2001); Roitt et al., Roitt's Essential Immunology, 10th Ed. Blackwell Publishing, UK (2001); and Janeway et al., "Immunobiology" (6th Ed.), Garland Science Publishing/Churchill Livingstone, New York (2005), as well as to the general background art cited herein;

Unless indicated otherwise, the term "immunoglobulin single variable domain" - whether used herein to refer to e.g. a Nanobody or to a dAb - is used as a general term to include both the full-size Nanobody or dAb, as well as functional fragments thereof. The terms antigen-binding molecules or antigen-binding protein are used interchangeably with immunoglobulin single variable domain and/or constructs thereof, and include Nanobodies and their constructs. According to the invention, the immunoglobulin single variable domains can be domain antibodies, or amino acid sequences that are suitable for use as domain antibodies, single domain antibodies, or amino acid sequences that are suitable for use as single domain antibodies, "dAbs", or amino acid sequences that are suitable for use as dAbs, or Nanobodies, including but not limited to humanized V_{HH} sequences, affinity matured V_{HH} sequences, chemically stabilized and/or V_{HH} sequences with improved solubilisation and preferably are Nanobodies. The immunoglobulin single variable domains provided by the invention are preferably in essentially isolated form (as defined herein), or form part of a construct, protein or polypeptide of the invention (as defined herein), which may comprise or essentially consist of one or more amino acid sequences of the invention. For example, and without limitation, the one or more amino acid sequences of the invention may be used as a binding unit in such a construct, protein or polypeptide, , so as to provide a monovalent, multivalent or multispecific construct of the invention, respectively, all as described herein. Such a construct may also be in essentially isolated form (as defined herein).

The invention includes immunoglobulin single variable domains of different origin, comprising mouse, rat, rabbit, donkey, human and camelid immunoglobulin sequences. The invention also includes fully human, humanized or chimeric immunoglobulin sequences. For example, the invention comprises camelid immunoglobulin sequences and humanized camelid immunoglobulin sequences, or camelized domain antibodies, e.g. camelized dAb as described by WO 94/04678). Moreover, the invention comprises fused immunoglobulin sequences, e.g. forming a multivalent and/ or multispecific construct (for multivalent and multispecific polypeptides containing one or more V_{HH} domains and their preparation, reference is also made to Conrath et al., J. Biol. Chem., Vol. 276, 10. 7346-7350, 2001, as well as to for example WO 96/34103 and WO 99/23221), and immunoglobulin single variable domains comprising tags or other functional moieties, e.g. toxins, labels, radiochemicals, etc., which are derivable from the immunoglobulin single variable domains of the present invention.

The amino acid sequence and structure of an immunoglobulin single variable domains, in particular a Nanobody can be considered - without however being limited thereto - to be comprised of four framework regions or "FR's", which are referred to in the art and herein as "Framework region 1" or "FR1"; as "Framework region 2" or "FR2"; as "Framework region 3" or "FR3"; and as "Framework region 4" or "FR4", respectively; which framework regions are interrupted by three complementary determining regions or "CDR's", which are referred to in the art as "Complementarity Determining Region 1" or "CDR1"; as "Complementarity Determining Region 2" or "CDR2"; and as "Complementarity Determining Region 3" or "CDR3", respectively.

The total number of amino acid residues in a Nanobody can be in the region of 110-120, is preferably 112-115, and is most preferably 113. It should however be noted that parts, fragments, analogs or derivatives (as further described herein) of a Nanobody are not particularly limited as to their length and/or size, as long as such parts, fragments, analogs or derivatives meet the further requirements outlined herein and are also preferably suitable for the purposes described herein.

As used herein, the term a sequence to the "immunoglobulin single variable domain" may refer to both the nucleic acid sequences coding for said immunoglobulin molecule, and the immunoglobulin polypeptide *per se.* Any more limiting meaning will be apparent from the particular context.

All these molecules are also referred to as "agent(s) of the invention", which is synonymous with "immunoglobulin single variable domain(s) and/or construct(s) thereof" of the invention.

In addition, the term "sequence" as used herein (for example in terms like "immunoglobulin single variable domain sequence", "Nanobody sequence", "V_{HH} sequence" or "polypeptide sequence"), should generally be understood to include both the relevant amino acid sequence as well as nucleic acid sequences or nucleotide sequences encoding the same, unless the context requires a more limited interpretation.

Unless indicated otherwise, all methods, steps, techniques and manipulations that are not specifically described in detail can be performed and have been performed in a manner known per se, as will be clear to the skilled person. Reference is for example again made to the standard handbooks and the general background art mentioned herein and to the further references cited therein; as well as to for example the following review "Pulmonary Drug Delivery" (Bechtold-Peters and Luessen, eds., referenced supra), which describe techniques for pulmonary drug delivery of biopharmaceuticals such as the agent(s) of the invention.

In a specific and preferred aspect, the immunoglobulin single variable domains are Nanobodies against, and in particular Nanobodies against druggable antigen from a mammal, and especially Nanobodies against human druggable antigen; as well as construct(s) comprising at least one such Nanobody.
In particular, the invention provides Nanobodies against druggable antigen, and constructs comprising the same, that have improved therapeutic and/or pharmacological properties and/or other advantageous properties (such as, for example, improved ease of preparation and/or reduced costs of goods), compared to conventional antibodies against druggable antigen or fragments thereof, compared to constructs that could be based on such conventional antibodies or antibody fragments (such as Fab' fragments, F(ab')₂ fragments, ScFv constructs, "diabodies" and other multispecific constructs (see for example the review by Holliger and Hudson, Nat Biotechnol. 2005 Sep;23(9):1126-36)), and also compared to the so-called "dAb's" or similar (single) domain antibodies that may be derived from variable domains of conventional antibodies. These improved and advantageous properties will become clear from the further description herein, and for example include, without limitation, one or more of:
- increased affinity and/or avidity for druggable antigen, either in a monovalent format, in a multivalent format (for example in a bivalent format) and/or in a multispecific format (for example one of the multispecific formats described herein below);
- better suitability for formatting in a multivalent format (for example in a bivalent format);
- better suitability for formatting in a multispecific format (for example one of the multispecific formats described herein below);
- improved suitability or susceptibility for "humanizing" substitutions;
- less immunogenicity, either in a monovalent format, in a multivalent format (for example in a bivalent format) and/or in a multispecific format (for example one of the multispecific formats described herein below);
- increased stability, either in a monovalent format, in a multivalent format (for example in a bivalent format) and/or in a multispecific format (for example one of the multispecific formats described herein below);
- increased specificity towards druggable antigen, either in a monovalent format, in a multivalent format (for example in a bivalent format) and/or in a multispecific format (for example one of the multispecific formats described herein below);
- decreased or where desired increased cross-reactivity with druggable antigen from different species;
   and/or
- one or more other improved properties desirable for pharmaceutical use (including prophylactic use and/or therapeutic use) and/or for diagnostic use (including but not limited to use for imaging purposes), either in a monovalent format, in a multivalent format (for example in a bivalent format) and/or in a multispecific format (for example one of the multispecific formats described herein below).

As generally described herein for the agent of the invention, the Nanobodies and construct thereof of the invention are preferably in essentially isolated form (as defined herein), wherein the constructs may comprise or essentially consist of one or more Nanobodies of the invention. For example, and without limitation, the Nanobody of the invention may be used as a binding unit in such a construct, which may optionally contain one or more further Nanobodies that can serve as a binding unit (i.e. against one or more other druggable antigens), so as to provide a monovalent, multivalent or multispecific construct of the invention, respectively, all as described herein. In particular, such a construct may comprise or essentially consist of one or more Nanobodies of the invention and optionally one or more (other) Nanobodies (i.e. directed against other druggable antigens), all optionally linked via one or more suitable linkers, so as to provide a monovalent, multivalent or multispecific Nanobody constructs, respectively, as further described herein. Such proteins or polypeptides may also be in essentially isolated form (as defined herein).

In a Nanobody of the invention, the binding site for binding against a druggable antigen is preferably formed by the CDR sequences. Optionally, a Nanobody of the invention may also, and in addition to the at least one binding site for binding against druggable antigen, contain one or more further binding sites for binding against other antigens. For methods and positions for introducing such second binding sites, reference is for example made to Keck and Huston, Biophysical Journal, 71, October 1996, 2002-2011; EP 0 640 130; and WO 06/07260.

As generally described herein for the amino acid sequences of the invention, when a Nanobody of the invention (or a polypeptide of the description comprising the same) is intended for administration to a subject (for example for therapeutic, prophylactic and/or diagnostic purpose as described herein), it is preferably directed against a human druggable antigen; whereas for veterinary purposes, it is preferably directed against a druggable antigen from the species to be treated. Also, as with the amino acid sequences of the description, a Nanobody of the invention may or may not be cross-reactive (i.e. directed against druggable antigen from two or more species of mammal, such as against human druggable antigen and druggable antigen from at least one of the species of mammal mentioned herein).

Also, again as generally described herein for the agents of the invention, the Nanobodies of the invention may generally be directed against any antigenic determinant, epitope, part, domain, subunit or confirmation of a druggable antigen.

As already described herein, the amino acid sequence and structure of a Nanobody can be considered - without however being limited thereto - to be comprised of four framework regions or "FR's" (or sometimes also referred to as "FW's"), which are referred to in the art and herein as "Framework region 1" or "FR1"; as "Framework region 2" or "FR2"; as "Framework region 3" or "FR3"; and as "Framework region 4" or "FR4", respectively; which framework regions are interrupted by three complementary determining regions or "CDR's", which are referred to in the art as "Complementarity Determining Region 1" or "CDR1"; as "Complementarity Determining Region 2" or "CDR2"; and as "Complementarity Determining Region 3" or "CDR3", respectively. Some preferred framework sequences and CDR's (and combinations thereof) that are present in the Nanobodies of the invention are as e.g. described on page 146ff of WO2008/074839.

According to a non-limiting but preferred aspect of the invention, (the CDR sequences present in) the Nanobodies of the invention are such that:
- the Nanobodies can bind to a druggable antigen with a dissociation constant (K_{D}) of 10⁻⁵ to 10⁻¹² moles/liter or less, and preferably 10⁻⁷ to 10⁻¹² moles/liter or less and more preferably 10⁻⁸ to 10⁻¹² moles/liter (i.e. with an association constant (K_{A}) of 10⁵ to 10¹² liter/ moles or more, and preferably 10⁷ to 10¹² liter/moles or more and more preferably 10⁸ to 10¹² liter/moles);
and/or such that:
- the Nanobodies can bind to a druggable antigen with a kₒₙ-rate of between 10² M⁻¹s⁻¹ to about 10⁷ M⁻¹s⁻¹, preferably between 10³ M⁻¹s⁻¹ and 10⁷ M⁻¹s⁻¹, more preferably between 10⁴ M⁻¹s⁻¹ and 10⁷ M⁻¹s⁻¹, such as between 10⁵ M⁻¹s⁻¹ and 10⁷ M⁻¹s⁻¹;
and/or such that they:
- the Nanobodies can bind to a druggable antigen with a k_{off} rate between 1 s⁻¹ (t_{1/2}=0.69 s) and 10⁻⁶ s⁻¹ (providing a near irreversible complex with a t_{1/2} of multiple days), preferably between 10⁻² s⁻¹ and 10⁻⁶s⁻¹, more preferably between 10⁻³ s⁻¹ and 10⁻⁶ s⁻¹, such as between 10⁻⁴ s⁻¹ and 10⁻⁶ s⁻¹.

Preferably, (the CDR sequences present in) the Nanobodies of the invention are such that: a monovalent Nanobody of the invention (or a polypeptide that contains only one Nanobody of the invention) is preferably such that it will bind to druggable antigen with an affinity less than 500 nM, preferably less than 200 nM, more preferably less than 10 nM, such as less than 500 pM.

The affinity of the Nanobody of the invention against druggable antigen can be determined in a manner known per se, for example using the general techniques for measuring K_{D}. K_{A}, k_{off} or kₒₙ mentioned herein, as well as some of the specific assays described herein.

Some preferred IC50 values for binding of the Nanobodies of the invention (and of polypeptides comprising the same) to druggable antigen will become clear from the further description and examples herein.

The invention relates to immunoglobulin single variable domains and/or constructs thereof that can bind to and/or have affinity for an antigen as defined herein. In the context of the present invention, "binding to and/or having affinity for" a certain antigen has the usual meaning in the art as understood e.g. in the context of antibodies and their respective antigens.

In particular embodiments of the invention, the term "binds to and/or having affinity for" means that the immunoglobulin single variable domain and/or construct thereof specifically interacts with an antigen, and is used interchangeably with immunoglobulin single variable domains and/or constructs thereof "against" the said antigen.

The term "specificity" refers to the number of different types of antigens or antigenic determinants to which a particular immunoglobulin single variable domains and/or constructs thereof (such as a Nanobody or other agent of the invention) can bind. The specificity of an antigen-binding protein can be determined based on affinity and/or avidity. The affinity, represented by the equilibrium constant for the dissociation of an antigen with an antigen-binding protein (K_{D}), is a measure for the binding strength between an antigenic determinant and an antigen-binding site on the antigen-binding protein: the lesser the value of the K_{D}, the stronger the binding strength between an antigenic determinant and the antigen-binding molecule (alternatively, the affinity can also be expressed as the affinity constant (K_{A}), which is 1/K_{D}). As will be clear to the skilled person (for example on the basis of the further disclosure herein), affinity can be determined in a manner known per se, depending on the specific antigen of interest. Avidity is the measure of the strength of binding between an antigen-binding molecule (such as a Nanobody or other agent of the invention) and the pertinent antigen. Avidity is related to both the affinity between an antigenic determinant and its antigen binding site on the antigen-binding molecule and the number of pertinent binding sites present on the antigen-binding molecule.

Typically, immunoglobulin single variable domains and/or constructs thereof of the present invention (such as the Nanobodies and/or other agents of the invention) will bind to their antigen with a dissociation constant (K_{D}) of 10⁻⁵ to 10⁻¹² moles/liter or less, and preferably 10⁻⁷ to 10⁻¹² moles/liter or less and more preferably 10⁻⁸ to 10⁻¹² moles/liter (i.e. with an association constant (K_{A}) of 10⁵ to 10¹² liter/ moles or more, and preferably 10⁷ to 10¹² liter/moles or more and more preferably 10⁸ to 10¹² liter/moles);
and/or
bind to antigens as e.g. defined herein with a kₒₙ-rate of between 10² M⁻¹s⁻¹ to about 10⁷ M⁻¹s⁻¹, preferably between 10³ M⁻¹s⁻¹ and 10⁷ M⁻¹s⁻¹, more preferably between 10⁴ M⁻¹s⁻¹ and 10⁷ M⁻¹s⁻¹, such as between 10⁵ M⁻¹s⁻¹ and 10⁷ M⁻¹s⁻¹; and/or
bind to the antigens as e.g. defined herein with a k_{off} rate between 1s⁻¹ (t_{1/2}=0.69 s) and 10⁻⁶ s⁻¹ (providing a near irreversible complex with a t_{1/2} of multiple days), preferably between 10⁻² s⁻¹ and 10⁻⁶ s⁻¹, more preferably between 10⁻³ s⁻¹ and 10⁻⁶ s⁻¹, such as between 10⁻⁴ s⁻¹ and 10⁻⁶ s⁻¹.

Any K_{D} value greater than 10⁻⁴ mol/liter (or any K_{A} value lower than 10⁴ M⁻¹) liters/mol is generally considered to indicate non-specific binding.

Preferably, a monovalent immunoglobulin single variable domain of the invention will bind to the desired antigen with an affinity less than 500 nM, preferably less than 200 nM, more preferably less than 10 nM, such as less than 500 pM.

Specific binding of an antigen-binding protein to an antigen or antigenic determinant can be determined in any suitable manner known per se, including, for example, Scatchard analysis and/or competitive binding assays, such as radioimmunoassays (RIA), enzyme immunoassays (EIA) and sandwich competition assays, and the different variants thereof known per se in the art; as well as the other techniques mentioned herein.

The dissociation constant may be the actual or apparent dissociation constant, as will be clear to the skilled person. Methods for determining the dissociation constant will be clear to the skilled person, and for example include the techniques mentioned herein. In this respect, it will also be clear that it may not be possible to measure dissociation constants of more then 10⁻⁴ moles/liter or 10⁻³ moles/liter (e.g. of 10⁻² moles/liter). Optionally, as will also be clear to the skilled person, the (actual or apparent) dissociation constant may be calculated on the basis of the (actual or apparent) association constant (K_{A}), by means of the relationship [K_{D} = 1/K_{A}].

The affinity denotes the strength or stability of a molecular interaction. The affinity is commonly given as by the K_{D}, or dissociation constant, which has units of mol/liter (or M). The affinity can also be expressed as an association constant, K_{A}, which equals 1/K_{D} and has units of (mol/liter)⁻¹ (or M⁻¹). In the present specification, the stability of the interaction between two molecules (such as an agent of the invention and its intended antigen) will mainly be expressed in terms of the K_{D} value of their interaction; it being clear to the skilled person that in view of the relation K_{A} =1/K_{D}, specifying the strength of molecular interaction by its K_{D} value can also be used to calculate the corresponding K_{A} value. The K_{D}-value characterizes the strength of a molecular interaction also in a thermodynamic sense as it is related to the free energy (DG) of binding by the well known relation DG=RT.ln(K_{D}) (equivalently DG=-RT.ln(K_{A})), where R equals the gas constant, T equals the absolute temperature and In denotes the natural logarithm.

The K_{D} for biological interactions, such as the binding of the agents of the invention to the antigens as e.g. defined herein, which are considered meaningful (e.g. specific) are typically in the range of 10⁻¹⁰M (0.1 nM) to 10⁻⁵M (10000 nM). The stronger an interaction is, the lower is its K_{D}.

The K_{D} can also be expressed as the ratio of the dissociation rate constant of a complex, denoted as k_{off}, to the rate of its association, denoted kₒₙ (so that K_{D} =k_{off}/kₒₙ and K_{A} = kₒₙ/k_{off}). The off-rate k_{off} has as unit s⁻¹ (where s is the SI unit notation of second). The on-rate kₒₙ has units M⁻¹s⁻¹.

As regards agents of the invention, the on-rate may vary between 10² M⁻¹s⁻¹ to about 10⁷ M⁻¹s⁻¹, approaching the diffusion-limited association rate constant for bimolecular interactions. The off-rate is related to the half-life of a given molecular interaction by the relation t_{1/2}=ln(2)/k_{off}. The off-rate of immunoglobulin sequences of the invention may vary between 10⁻⁶ s⁻¹ (near irreversible complex with a t_{1/2} of multiple days) to 1 s⁻¹ (t_{1/2}=0.69 s).

The affinity of a molecular interaction between two molecules can be measured via different techniques known per se, such as the well known surface plasmon resonance (SPR) biosensor technique (see for example Ober et al., Intern. Immunology, 13, 1551-1559, 2001) where one molecule is immobilized on the biosensor chip and the other molecule is passed over the immobilized molecule under flow conditions yielding kₒₙ, k_{off} measurements and hence K_{D} (or K_{A}) values. This can for example be performed using the well-known Biacore instruments.

It will also be clear to the skilled person that the measured K_{D} may correspond to the apparent K_{D} if the measuring process somehow influences the intrinsic binding affinity of the implied molecules for example by artefacts related to the coating on the biosensor of one molecule. Also, an apparent K_{D} may be measured if one molecule contains more than one recognition sites for the other molecule. In such situation the measured affinity may be affected by the avidity of the interaction by the two molecules.

Another approach that may be used to assess affinity is the 2-step ELISA (Enzyme-Linked Immunosorbent Assay) procedure of Friguet et al. (J. Immunol. Methods, 77, 305-19, 1985). This method establishes a solution phase binding equilibrium measurement and avoids possible artefacts relating to adsorption of one of the molecules on a support such as plastic.

However, the accurate measurement of K_{D} may be quite labour-intensive and as consequence, often apparent K_{D} values are determined to assess the binding strength of two molecules. It should be noted that as long as all measurements are made in a consistent way (e.g. keeping the assay conditions unchanged) apparent K_{D} measurements can be used as an approximation of the true K_{D} and hence in the present document K_{D} and apparent K_{D} should be treated with equal importance or relevance.

Finally, it should be noted that in many situations the experienced scientist may judge it to be convenient to determine the binding affinity relative to some reference molecule. For example, to assess the binding strength between molecules A and B, one may e.g. use a reference molecule C that is known to bind to B and that is suitably labelled with a fluorophore or chromophore group or other chemical moiety, such as biotin for easy detection in an ELISA or FACS (Fluorescent activated cell sorting) or other format (the fluorophore for fluorescence detection, the chromophore for light absorption detection, the biotin for streptavidin-mediated ELISA detection). Typically, the reference molecule C is kept at a fixed concentration and the concentration of A is varied for a given concentration or amount of B. As a result an IC₅₀ value is obtained corresponding to the concentration of A at which the signal measured for C in absence of A is halved. Provided K_{D ref}, the K_{D} of the reference molecule, is known, as well as the total concentration c_{ref} of the reference molecule, the apparent K_{D} for the interaction A-B can be obtained from following formula: K_{D} =IC₅₀/(1+c_{ref}/ K_{D ref}). Note that if c_{ref} << K_{D ref}, K_{D} ≈ IC₅₀. Provided the measurement of the IC₅₀ is performed in a consistent way (e.g. keeping c_{ref} fixed) for the binders that are compared, the strength or stability of a molecular interaction can be assessed by the IC₅₀ and this measurement is judged as equivalent to K_{D} or to apparent K_{D} throughout this text.

In the context of the present invention, "systemic circulation" denotes the portion of the cardiovascular system which carries oxygenated blood away from the heart, to the body, and returns deoxygenated blood back to the heart (see e.g. Wikipedia).

In the context of the present invention, "pulmonary tissue" is for the purposes of this invention equivalent with lung tissue or lung. The lung comprises 2 distinct zones: a conducting and a respiratory zone, within which the airway and vascular compartments lie (see e.g. "Pulmonary Drug Delivery, Bechtold-Peters and Luessen, eds., supra, pages 16-28).

In the context of the present invention, "aerosol" denotes a suspension of fine solid particles or liquid droplets (or combination thereof) in a gas wherein for the purposes of this invention the particles and/or droplets comprise the agent(s) of the invention.

In the context of the present invention, "half-life" of an agent of the invention can generally be defined as described in paragraph o) on page 57 of WO 08/020079 and as mentioned therein refers to the time taken for the serum concentration of the amino acid sequence, compound or polypeptide to be reduced by 50%, in vivo, for example due to degradation of the sequence or compound and/or clearance or sequestration of the sequence or compound by natural mechanisms. The in vivo half-life of an agent of the invention can be determined in any manner known per se, such as by pharmacokinetic analysis. Suitable techniques will be clear to the person skilled in the art, and may for example generally be as described in paragraph o) on page 57 of WO 08/020079. As also mentioned in paragraph o) on page 57 of WO 08/020079, the half-life can be expressed using parameters such as the t1/2-alpha, t1/2-beta and the area under the curve (AUC). Reference is for example made to the standard handbooks, such as Kenneth, A et al: Chemical Stability of Pharmaceuticals: A Handbook for Pharmacists and Peters et al, Pharmacokinetic analysis: A Practical Approach (1996). Reference is also made to "Pharmacokinetics", M Gibaldi & D Perron, published by Marcel Dekker, 2nd Rev. edition (1982). The terms "increase in half-life" or "increased half-life" as also as defined in paragraph o) on page 57 of WO 08/020079 and in particular refer to an increase in the t1/2-beta, either with or without an increase in the t1/2-alpha and/or the AUC or both. Moreover, in the context of the present invention the term "Terminal plasma half-life" is the time required to divide the plasma concentration by two after reaching pseudo-equilibrium, and not the time required to eliminate half the administered dose. When the process of absorption is not a limiting factor, half-life is a hybrid parameter controlled by plasma clearance and extent of distribution. In contrast, when the process of absorption is a limiting factor, the terminal half-life reflects rate and extent of absorption and not the elimination process (flip-flop pharmacokinetics). The terminal half-life is especially relevant to multiple dosing regimens, because it controls the degree of drug accumulation, concentration fluctuations and the time taken to reach equilibrium.

In the context of the present invention, "bioavailability" of an inhaled aerosol comprising the agent of the invention can be determined using plasma concentration-time profiles by comparing the area under the concentration-time curve after inhalation (referred herein as "AUC-inh") with that obtained after intravenous administration (referred herein as "AUC-iv") wherein an aerosol is a suspension of fine solid particles or liquid droplets in a gas. The "absolute bioavailability" (referred herein as "F-inh") after inhalation can then be calculated by the equation F-inh = AUC-inh divided by AUC-iv (when inhaled and intravenous doses are identical).

In the context of the present invention, "tau" is a time interval and denotes the dosing interval.

In the context of the present invention, "antigen(s)" define all antigens that are druggable to the skilled person in the art and include all druggable interaction sites of an antigen. Particularly preferred antigen(s) are the antigen(s) as e.g. herein described such as human von Willebrand factor, human RANK ligand and /or viral antigen(s) such as RSV and/or avian flu virus.

As used herein, the term "antigen" is intended to include, and also refer to, any part, fragment, subunit, epitope or domain of said antigen. Any subsection of a cell wherein the antigen is associated falls within the scope of the present invention, provided it represents a druggable antigen of interest.
In particular, the present invention relates to immunoglobulin single variable domains directed to antigens in their natural conformation. In the context of the present invention, "natural conformation" means that the antigen exhibits its secondary and/or tertiary structure. In other words, the natural conformation describes the antigen in a non-denatured form, and describes a conformation wherein the conformational or linear epitopes are present. Specifically, the antigen will have the conformation that is present when the antigen is integrated into mammal, e.g. firmly attached to a cell membrane of said mammal. Antigens can be obtained in their natural conformation when present in cells comprising natural or transfected cells expressing the cell-associated antigen, cell derived membrane extracts, vesicles or any other membrane derivative harbouring antigen, liposomes, or virus particles expressing the cell associated antigen. In any of these embodiments, antigen may be enriched by suitable means. Said cell-associated antigen can be expressed on any suitable cell allowing expression of the antigen in its native or natural conformation, encompassing, but not limited to Cho, Cos7, Hek293 or cells of camelid origin.

The skilled person will appreciate that there may be different specific three dimensional conformations that are encompassed by the term "natural conformation". If, for example, a protein has two or more different conformations whilst being in a membrane environment, all these conformations will be considered "natural conformations". This is exemplified by receptors changing their conformation by activation, e.g. the different activation states of rhodopsin induced by light, or ion channels showing a "closed" or "open" conformation. The invention encompasses immunoglobulin sequences to any one of these different natural conformations, i.e. to the different kinds of conformational epitopes that may be present.

The antigen is preferably a druggable interaction site of an antigen that has, when modulated, a prophylactic and/or therapeutic effect in a mammal, e.g. a human, preferably in a mammal, e.g. human, that is at risk and/or has a disease.

In the context of the present invention, the term "interaction site" on the antigen means a site, epitope, antigenic determinant, part, domain or stretch of amino acid residues on the target or antigen that is a site for binding to a ligand, receptor or other binding partner, a catalytic site, a cleavage site, a site for allosteric interaction, a site involved in multimerisation (such as homomerization or heterodimerization) of the antigen; or any other site, epitope, antigenic determinant, part, domain or stretch of amino acid residues on the target or antigen that is involved in a biological action or mechanism of the antigen. More generally, an "interaction site" can be any site, epitope, antigenic determinant, part, domain or stretch of amino acid residues on the antigen to which an agent of the invention can bind such that the antigen (and/or any pathway, interaction, signalling, biological mechanism or biological effect in which the antigen is involved) is modulated.

In the context of the present invention, "modulating" or "to modulate" generally means either reducing or inhibiting the activity of, or alternatively increasing the activity of, a target or antigen, as measured using a suitable in vitro, cellular or in vivo assay. In particular, "modulating" or "to modulate" may mean either reducing or inhibiting the activity of, or alternatively increasing a (relevant or intended) biological activity of, a target or antigen, as measured using a suitable in vitro, cellular or in vivo assay (which will usually depend on the target or antigen involved), by at least 1%, preferably at least 5%, such as at least 10% or at least 25%, for example by at least 50%, at least 60%, at least 70%, at least 80%, or 90% or more, compared to activity of the target or antigen in the same assay under the same conditions but without the presence of the construct of the invention.

As will be clear to the skilled person, "modulating" may also involve effecting a change (which may either be an increase or a decrease) in affinity, avidity, specificity and/or selectivity of a target or antigen for one or more of its ligands, binding partners, partners for association into a homomultimeric or heteromultimeric form, or substrates; and/or effecting a change (which may either be an increase or a decrease) in the sensitivity of the target or antigen for one or more conditions in the medium or surroundings in which the target or antigen is present (such as pH, ion strength, the presence of co-factors, etc.), compared to the same conditions but without the presence of the construct of the invention. As will be clear to the skilled person, this may again be determined in any suitable manner and/or using any suitable assay known per se, depending on the target or antigen involved.

"Modulating" may also mean effecting a change (i.e. an activity as an agonist, as an antagonist or as a reverse agonist, respectively, depending on the target or antigen and the desired biological or physiological effect) with respect to one or more biological or physiological mechanisms, effects, responses, functions, pathways or activities in which the target or antigen (or in which its substrate(s), ligand(s) or pathway(s) are involved, such as its signalling pathway or metabolic pathway and their associated biological or physiological effects) is involved. Again, as will be clear to the skilled person, such an action as an agonist or an antagonist may be determined in any suitable manner and/or using any suitable (in vitro and usually cellular or in assay) assay known per se, depending on the target or antigen involved. In particular, an action as an agonist or antagonist may be such that an intended biological or physiological activity is increased or decreased, respectively, by at least 1%, preferably at least 5%, such as at least 10% or at least 25%, for example by at least 50%, at least 60%, at least 70%, at least 80%, or 90% or more, compared to the biological or physiological activity in the same assay under the same conditions but without the presence of the construct of the invention.

Modulating may for example also involve allosteric modulation of the target or antigen; and/or reducing or inhibiting the binding of the target or antigen to one of its substrates or ligands and/or competing with a natural ligand, substrate for binding to the target or antigen. Modulating may also involve activating the target or antigen or the mechanism or pathway in which it is involved. Modulating may for example also involve effecting a change in respect of the folding or confirmation of the target or antigen, or in respect of the ability of the target or antigen to fold, to change its confirmation (for example, upon binding of a ligand), to associate with other (sub)units, or to disassociate. Modulating may for example also involve effecting a change in the ability of the target or antigen to transport other compounds or to serve as a channel for other compounds (such as ions). Modulating may be reversible or irreversible, but for pharmaceutical and pharmacological purposes will usually be in a reversible manner.

In the context of the present invention, "non-human animal" includes, but is not limited to vertebrate, shark, mammal, lizard, camelid, llama, preferably camelids and most preferably llama or alpaca.

### B) The methods of the present description

The present invention relates to immunoglobulin single variable domains and/or constructs thereof for use in a method for providing to a the systemic circulation of a mammal, an effective amount of an immunoglobulin single variable domain and/or construct thereof that can bind to and/or have affinity for at least one antigen, as defined herein. The method comprises the following step:
a) administering the immunoglobulin single variable domain and/or construct thereof to the pulmonary tissue of said mammal.

Thus, in general terms (and in a preferred way) the method of the present description includes systemic delivery of an immunoglobulin single variable domain and/or construct thereof to a mammal mainly via pulmonary tissue absorption. In one particular embodiment, the mammal is a human. In another particular embodiment, the administration is achieved by inhaling said immunoglobulin single variable domain and/or construct thereof to the pulmonary tissue in an aerosol cloud.

One particular advantage of the present invention resides in the fact that delivery of a immunoglobulin single variable domain and/or construct thereof is widely applicable and results in a long systemic exposure of said immunoglobulin single variable domain and/or construct thereof. The method of the description is not limited to have e.g. serum protein binding properties, e.g. serum albumin binding, of said immunoglobulin single variable domain and/or construct thereof to achieve a long exposure but may well include such constructs. In particular, there is no requirement for extending the immunoglobulin single variable domain and/or construct thereof directed against the antigen to add an additional binding unit directed against a particular antigen, e.g. serum albumin binder, in order to extend exposure time in systemic circulation. Advantageously for some of such constructs (e.g. the Nanobody and the constructs in the experimental part of example 1), the method also implies that relatively simple dose calculation for multiple dosing based on experimentally terminal half life, tau and bioavailability can be performed (based on the assumption that the rate limiting step of the pharmacokinetic properties of immunoglobulin single variable domain and/or construct thereof is absorption controlled). Hence, the method of the present description is broadly applicable to any druggable antigen, in particular interaction side. In particular, e.g. in a preferred embodiment, the present method is applicable to antigens for which a potent (e.g. a sub-nanomolar IC50 in a relevant in vitro assay) immunoglobulin single variable domain and/or construct thereof, in particular Nanobody and/or construct thereof, to said antigen is available.

In a further aspect, the method of systemic delivery via the pulmonary route may be beneficial for constructs of immunoglobulin single variable domains that bind to and/or has a specific affinity for an antigen that has a prophylactic and/or therapeutic effect when modulated and bind to and/or has a specific affinity for serum protein such as e.g. serum albumin, e.g. human serum albumin. For such a construct the lung may not be the rate limiting step anymore, and thus the half life in this case may be driven by clearance and distribution.

Hence, the present invention is advantageous as compared to prior art immunoglobulin single variable domains and/or constructs thereof for use in methods that lack to mention properties as disclosed herein. In particular there is no teaching in the art to what extend in terms of half life and bioavailability such a method for delivery of immunoglobulin single variable domains and /or constructs thereof to the systemic circulation of mammals such as humans is capable.

More specifically, the present invention provides an immunoglobulin single variable domain and/or construct thereof for use in a first-in-class method for delivering an effective amount of immunoglobulin single variable domains and/or constructs thereof to the systemic circulation of mammals via the pulmonary route, which, according to one specific embodiment, is provided by inhaling a pharmaceutical dosage formulation with an inhaler device.

The device should generate from the formulation an aerosol cloud of the desired particle size of the fine solid particles or liquid droplets (distribution) at the appropriate moment of the mammal's inhalation cycle, containing the right dose of the immunoglobulin single variable domains and /or constructs thereof. The following 4 requirements (formulation, particle size, time and dose) should be considered (Pulmonary Drug Delivery, Bechtold-Peters and Luessen, eds., supra, pages 125 and 126):
- The formulations that are used in the devices may vary from aqueous solutions or suspensions used in nebulizers to the propellant-based solutions or suspensions used in metered dose inhaler or even specially engineered powder mixtures for the dry powder inhalers. All these different formulations require different principles for aerosol generation, which emphasizes the mutual dependency of device and formulation (e.g. Nebulizer formulation contain water with co-solvents such as PEG, ethanol or glycine (in "Inhalation Delivery of Therapeutic Peptides and Proteins" (1997), 2 para, page 246);
- Since the site of deposition of aerosol particles depends on their (aerodynamic) size and velocity, the desired particle size of the aerosol cloud varies depending on the desired site of deposition in the lung, which is related to the therapeutic goal of the administration. Preferably the agents of the invention that are to be absorbed into the systemic circulation should be deposited in the alveoli. Hence, preferably the particle size for the agents of the invention for a human may be within the 1 to 5 micrometer range (see also e.g. in particular page 245 in "Inhalation Delivery of Therapeutic Peptides and Proteins" (1997): Mass median diameters normally range from 2 to 5 um in nebulizers);
- As the aerosol cloud can be tuned to be released at different moments during the inhalation cycle generated by the mammal, it is preferred that for the agents of the invention (to be deposited in the peripheral parts of the lung) the aerosol is released at the start of the inhalation cycle;
- The variety of the agents of the invention that is proposed to be delivered via the pulmonary route implies that doses may vary considerably and may e.g. vary e.g. for a human from a few microgram to several hundreds of microgram or even milligrams, e.g. about up to about 10 milligrams.

Various inhalation systems are e.g. described on pages 129 to 148 in the review ("Pulmonary Drug Delivery", Bechtold-Peters and Luessen, eds., supra) and include, but are not limited to, nebulizers such as e.g. vibrating mesh nebulizers, metered dose inhalers, metered dose liquid inhalers, and dry powder inhalers. Devices taking into account optimized and individualized breathing pattern for controlled inhalation manoeuvres may also be used (see e.g. AKITA® technology on page 157 of "Pulmonary Drug Delivery", Bechtold-Peters and Luessen, eds., supra). Traditionally, nebulizers have been classified into two main types: air-jet (pneumatic) and ultrasonic devices. Recently, a third type, vibrating-mesh nebulizers has been commercialized (Newman, S., Gee-Turner, A., 2005. The Omron MicroAir Vibrating mesh technology nebuliser, a 21st century approach to inhalation therapy. J. Appl. Ther. Res. 5, 29-33). Air-jet nebulizers convert liquid into aerosols by means of a high velocity gas passing through a narrow "venturi" nozzle. The fluid in the nebulizer reservoir is drawn up a feed tube and emerges as fine filaments that collapse into aerosol droplets due to surface tension. In ultrasonic nebulizers, a high frequency vibrating piezoelectric crystal is employed to generate the aerosol. A fountain of fluid is produced at the air-fluid interface. Small droplets are generated from the lower regions of the fountain whilst large droplets are generated from the apex. In both air-jet and ultrasonic nebulizers baffles in the nebulizer trap and recycle the large (primary) aerosol droplets, whilst small (secondary) droplets are released for inhalation. In air-jet nebulizers, the aerosol output comprises aerosolized droplets and solvent vapour which saturates the outgoing air. This induces cooling of the nebulizer fluid and increases solute concentration in the residual volume (Cockcroft, D.W., Hurst, T.S., Gore, B.P., 1989. Importance of evaporative water losses during standardized nebulized inhalation provocation tests. Chest 96, 505-508). Ultrasonic nebulizers are generally unsuitable for delivery of suspensions (Taylor, K.M.G., McCallion, O.N.M., 2002. Ultrasonic nebulizers. In: Swarbrick, J., Boylan, J.C. (Eds.), Encyclopedia of Pharmaceutical Technology, 2nd ed. Marcel Dekker, Inc., New York, pp. 2840-2847) and liposomes (Elhissi, A.M.A., Taylor, K.M.G., 2005. Delivery of liposomes generated from proliposomes using air-jet, ultrasonic, and vibrating-mesh nebulisers. J. Drug Deliv. Sci. Technol. 15, 261-265), and due to heat generation during atomization they may degrade labile substances such as proteins (Niven, R.W., Ip, A.Y., Mittelman, S., Prestrelski, S.J., Arakawa, T., 1995. Some factors associated with the ultrasonic nebulization of proteins. Pharm. Res. 12, 53-59).

Vibrating-mesh nebulizers may overcome the drawbacks of air-jet and ultrasonic nebulizers. Vibrating-mesh devices employ perforated plates which vibrate in order to generate the aerosol. These nebulizers do not heat the fluid during atomization and have been shown to be suitable for delivery of suspensions (Fink, J.B., Simmons, B.S., 2004. Nebulization of steroid suspension: an in vitro evaluation of the Aeroneb Go and Pari LC Plus nebulizers. Chest 126, 816S), and delicate structures such as liposomes (Wagner, A., Vorauer-Uhl, K., Katinger, H., 2006. Nebulization of liposomal rh-Cu/Zn-SOD with a novel vibrating membrane nebulizer. J. Liposome Res. 16, 113-125) and nucleic acids (Lentz, Y.K., Anchordoquy, T.J., Lengsfeld, C.S., 2006. Rationale for the selection of an aerosol delivery system for gene delivery. J. Aerosol Med. 19, 372-384). Moreover, the Aeroneb Pro vibrating-mesh nebulizer in particular is recommended for the delivery of drugs during mechanical ventilation (Pedersen, K.M., Handlos, V.N., Heslet, L., Kristensen, H.G.K., 2006. Factors influencing the in vitro deposition of tobramycin aerosol: a comparison of an ultrasonic nebulizer and a high-frequency vibrating mesh nebulizer. J. Aerosol Med. 19, 175-183). Vibrating-mesh nebulizers are divided into passively and actively vibrating-mesh devices (Newman, S., Gee-Turner, A., 2005. The Omron MicroAir Vibrating mesh technology nebuliser, a 21st century approach to inhalation therapy. J. Appl. Ther. Res. 5, 29-33). Passively vibrating-mesh devices (e.g. Omron MicroAir NE-U22 nebulizer) employ a perforated plate having up to 6000 tapered holes, approximately 3_min diameter. A vibrating Piezo-electric crystal attached to a transducer horn induces "passive" vibrations in the perforated plate positioned in front of it, resulting in extrusion of fluid through the holes and generation of the aerosol. Actively vibrating-mesh devices (e.g. Aeroneb Pro nebulizer) may employ a "micropump" system which comprises an aerosol generator consisting of a plate with up to 1000 dome-shaped apertures and a vibrating element which contracts and expands on application of an electric current. This results in upward and downward movements of the mesh by a few micrometres, extruding the fluid and generating the aerosol.

In pulmonary delivery, the generation of particles smaller than approximately 5 or 6 micrometer is considered necessary to achieve deposition as the fine particle fraction (FPF) (i.e. in the respiratory bronchioles and alveolar region) (O'Callaghan, C., Barry, P.W., 1997. The science of nebulised drug delivery. Thorax 52, S31-S44).

However, not only the device is important to systemic delivery via the pulmonary route and/or pulmonary delivery of the agent of the invention but also the right formulation is critical to achieve an effective delivery. This can be in principle achieved by using one of the following approaches:
- Administration of aqueous solutions or suspensions comprising the agent of the invention (e.g. nasal drops) into the nasal cavities;
- Nebulisation of aqueous solutions or suspensions comprising the agent of the invention;
- Atomization by means of liquefied propellants; and
- Dispersion of dry powders.
Hence formulations of the agent of the inventions have to be adopted and adjusted to the chosen inhalation device. Appropriate formulations, i.e. the excipients in addition to the agent of the invention, are e.g. described in chapter IV of "Pulmonary Drug Delivery", Bechtold-Peters and Luessen, eds., supra.

The application also describes, in a specific embodiment, a method for delivery an effective amount of a Nanobody and/or construct thereof that can bind to and/or have affinity for at least one antigen, as defined herein. The method comprises the following step:
a) administering the Nanobody and/or construct thereof to the pulmonary tissue of said mammal.

More particularly, the application describes in a specific embodiment, a method for delivery an effective amount of a Nanobody construct that can bind to and/or has affinity for at least one antigen, as defined herein. The method comprises the following step:
a) administering the Nanobody and/or construct thereof to the pulmonary tissue of said mammal; and wherein the construct comprises at least one Nanobody. The construct may also comprise more than one Nanobody, e.g. two Nanobodies or three Nanobodies.

More particularly, the application describes in a specific embodiment, a method for delivery an effective amount of a Nanobody construct that can bind to and/or have affinity for at least one antigen. The method comprises the following step:
a) administering the Nanobody and/or construct thereof to the pulmonary tissue of said mammal; and wherein the construct comprises at least one Nanobody. The construct may also comprise more than one Nanobody, e.g. two Nanobodies or three Nanobodies. Furthermore, the construct can bind to and/or have affinity for more than one antigen, e.g. two or three antigens wherein optionally one of the antigens is serum albumin, e.g. human serum albumin.

Furthermore, the present invention provides in a specific embodiment, an immunoglobulin single variable domain and/or construct thereof for use in a method for systemic delivery of an immunoglobulin single variable domain and/or construct thereof that can bind to and/or have affinity for at least one antigen; and wherein the immunoglobulin single variable domain and/or construct thereof has a bioavailability comparable (e.g. within 10% to 20% higher or lower) to the equivalent subcutaneous administration. In a further embodiment, the bioavailability is at least about 10%, or 20%, or 30%, or 40% or 50% of the bioavailability of the equivalent intravenous administration (absolute bioavailability).

Another aspect of the invention is the surprisingly long lasting stability of the immunoglobulin single variable domain and/or construct thereof, in particular Nanobody and/or construct thereof. E.g. it has been found that a Nanobody directed against RSV remains functional in the lung for at least 48 hours (see experimental part). Thus, methods of administration of the description with dosage intervals of the agents of the invention such as once a day, once every 2^{nd}, 3^{rd}, 4^{th}, 5^{th}, 6^{th} or once every week, preferably once a day, are thought to be possible taken the estimated long lasting stability, potential bioavailability and half-life in systemic circulation.

It has also been surprisingly found, that in view of the high bioavailability of systemic delivery of the agents of the invention, e.g. as shown in the experimental part of this application, and the long lasting controlled release (e.g. the pseudo-equilibrium pharmacokinetic with relative long terminal half life as shown in the experimental part) into systemic circulation, dosage intervals of once a day, or longer, e.g. every 2^{nd}, 3^{rd}, 4^{th}, 5^{th} 6^{th}, or once a week, preferably once a day may be possible. In particular, dosage intervals of the agent of the invention comprising e.g. a serum albumin binder, e.g. human serum albumin binder, as described in the previous sentence may be feasible. This underlines the particular advantage of the present invention of resulting in an immunoglobulin single variable domain and/or construct thereof for use in an easy to use, non invasive delivery method that provides a long lasting systemic exposure of the agent of the invention allowing for once daily or longer interval dosing, e.g. up to once weekly dosing. It was unforeseeable from the prior art that such advantages can be obtained by using the pulmonary delivery route, in particular as the prior art suggests that systemic delivery via the pulmonary route is minimal (WO2007/049017).

### Dose:

The appropriate dosage will of course vary depending upon, for example, the inhalation/formulation employed, the host, and the nature and severity of the condition being treated. However, in general, satisfactory results in animals are indicated to be obtained at a daily dosage of from about 0.1 to about 10 mg/kg, e.g. about 5mg/kg animal body weight. In larger mammals, for example humans, an indicated daily dosage is in the range from about 1 to about 200 mg, preferably about 1 to about 10 mg of the compound conveniently administered as described herein.

The present description furthermore provides a pharmaceutical composition for pulmonary administration intended for pulmonary but in particular also for systemic delivery comprising an agent of the invention in association with at least one pharmaceutically acceptable diluent or carrier. Such compositions may be formulated in conventional manner as e.g. described and/or referenced herein. Unit dosage forms contain, for example, from about 0.25 to about 10 mg, preferably about 1mg, of an agent according to the invention.

The general principles of the present invention as set forth above will now be exemplified by reference to specific experiments. However, the invention is not to be understood as being limited thereto.

### Brief description of the figures

Figure 1: Individual (i.v.) and mean (i.t.) observed plasma concentration-time plot of ALX-0081 (i.v. 5 mg/kg; i.t. 3.1 mg/kg).
Figure 2: Individual (i.v.) and mean (i.t.) observed plasma concentration-time plot of RANKL008A (i.v. 5 mg/kg; i.t. 3.2 mg/kg).
Figure 3: Individual (i.v.) and mean (i.t.) observed plasma concentration-time plot of RSV NB2 (i.v. 4 mg/kg; i.t. 3.6 mg/kg).
Figure 4: Individual observed plasma concentration-time plot of RSV NB2, ALX-0081, and RANKL008A after a single i.v. bolus dose of RSV NB2 (4 mg/kg), ALX-0081 (5 mg/kg) and RANKL008A (5 mg/kg), respectively to male Wistar rats.
Figure 5: Mean (+SD) observed BALF concentration-time profiles of RSV NB2, ALX-0081, and RANKL008A after a single intratracheal administration of RSV NB2 (3.6 mg/kg), ALX-0081 (3.1 mg/kg) and RANKL008A (3.2 mg/kg) to male rats.
Figure 6: Pulmonary delivered Nanobodies are stable in the lung for at least 24 hrs post-administration.
Figure 7: Bioavailability in plasma of pulmonary administered vs *i.v.* administered Nanobodies.
Figure 8: Intranasal inoculation of bivalent Nanobody 191-D3 (RSV101) prevents in vivo infection and replication of RSV A2 strain. Titers of infectious RSV in the lung homogenates (pfu/lung) prepared three and five days post infection (detection limit below 100 pFU).
Figure 9: Functional Nanobody RSV101 remains detectable for at least 3 days following intranasal inoculation in mice.
Figure 10: Virus neutralizing titers of llama serum after immunization with hemagglutinin.
Figure 11: Binding assay with a dilution series of purified anti-H5 HA Nanobodies.
Figure 12: Competition of periplasmic fractions with fetuin for binding to the hemagglutinin.
Figure 13: Competition of purified nanobodies with fetuin for binding to the hemagglutinin
Figure 14: Identification of the neutralizing Nanobody 202-C8.
Figure 15: Identification of the neutralizing Nanobodies 203-B12 and 203-H9.
Figure 16: Combinations of Nanobodies 202-C8, 203-H9 and 203-B12 do not result in increased neutralization.
Figure 17: Intranasal delivery of Nanobody 202-C8 protects against infection and replication of mouse-adapted NIBRG-14 virus.

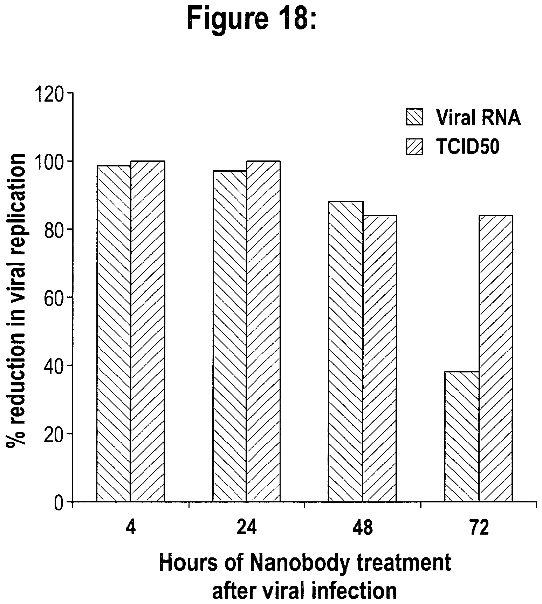

Figure 18: Nanobody (202-c8)2 reduces viral replication when administered up to 72 hours after viral infection. Infectious titers (TCID50/ml) and viral RNA in the lungs were determined 96 hours after viral infection. % reduction was calculated by comparing with infectious titers and RNA levels from mice treated with the control Nanobody (191D3)2.
Figure 19: Nanobody (202-C8)2 prevents viral-induced reduction in body weight when administered up to 48 hours after viral challenge. A comparison of body weights at 96 hours p.i. is shown as % of initial body weight
Figure 20: : Setup of the acute in vivo mouse splenocyte model.
Figure 21: Graph showing the results obtained in Example 7 for the inhibition of the mIL-22 synthesis in a mouse splenocyte assay upon administration of P23IL0075 via different routes of administration, i.e. i.t. and s.c.. (A) basal level, i.e. no induction mIL-22; (B) S.c. administration of PBT; (C) S.c. administration of P23IL0075; (D) I.t. administration of PBT; (E) I.t. administration of P23IL0075 (low dose); (F) I.t. administration of P23IL0075 (high dose); (G) I.t. administration of P23IL0075 (high dose, other buffer)
Figure 22: I.t. and i.p. administration of nanobody construct 4.10-Alb1 in mice. (a) Nanobody construct 4.10-Alb1 in circulation after i.p. and i.t. administration; (b) leptin levels before and after i.t. Nanobody construct 4.10-Alb1 administration; (c) leptin levels before and after i.p. Nanobody construct 4.10-Alb1 administration
Figure 23: Dose dependent increase of circulating leptin levels following i.t. administration of 4 increasing amounts of 4.10-Alb1 Nanobody constructs. (a) Nanobody constructs 4.10-Alb1 and IL6R202 were detected in blood following each i.t. or i.p. inoculation; (b) Leptin levels after injection of 4.10-Alb1 and IL6R202 control; (c) Leptin levels after i.t. administration of 4.10-Alb1 and IL6R202 control.

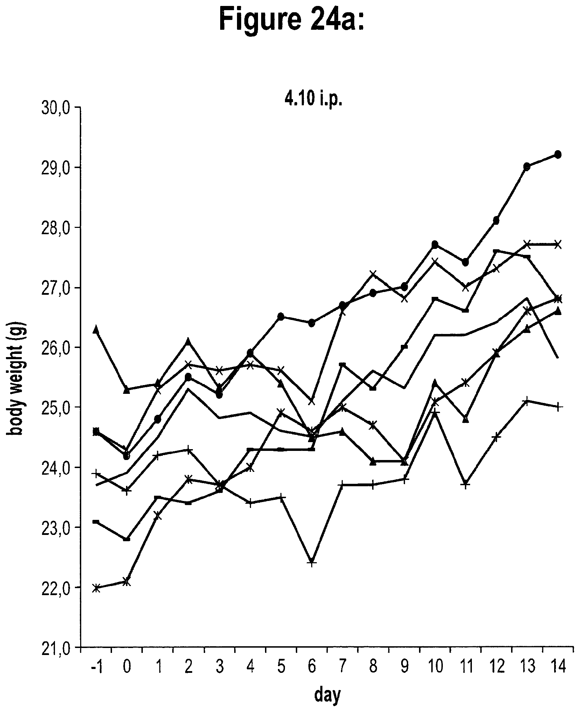

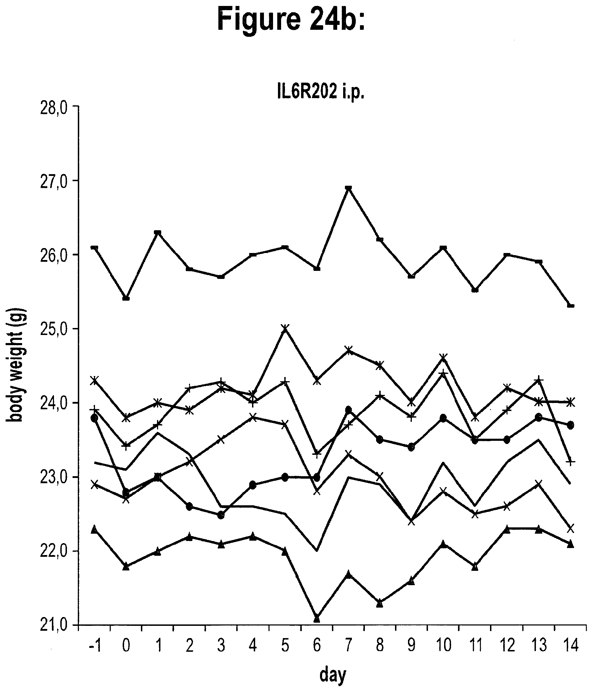

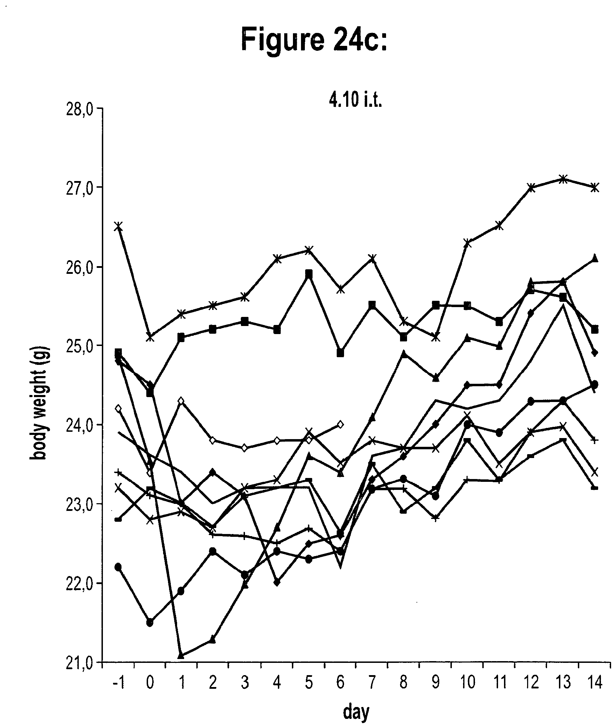

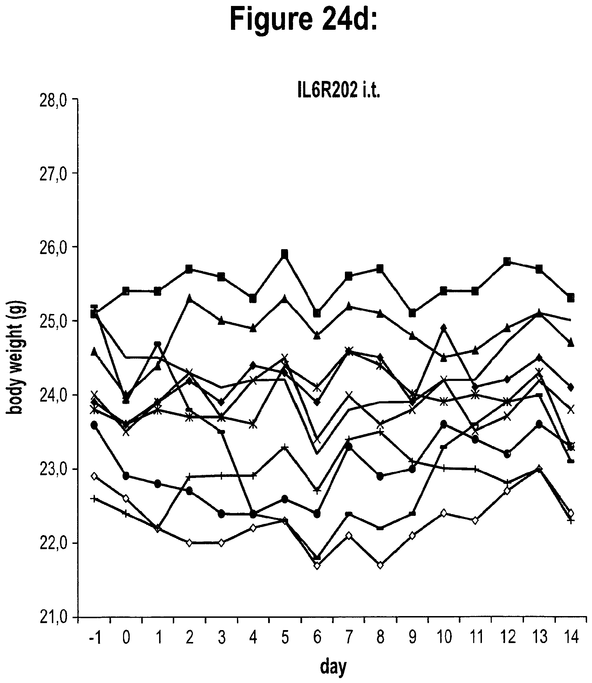

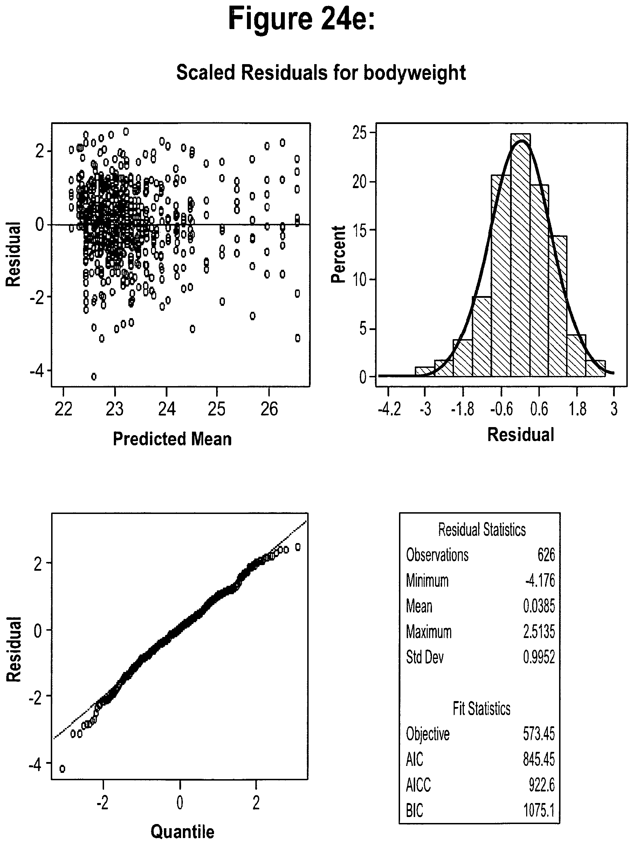

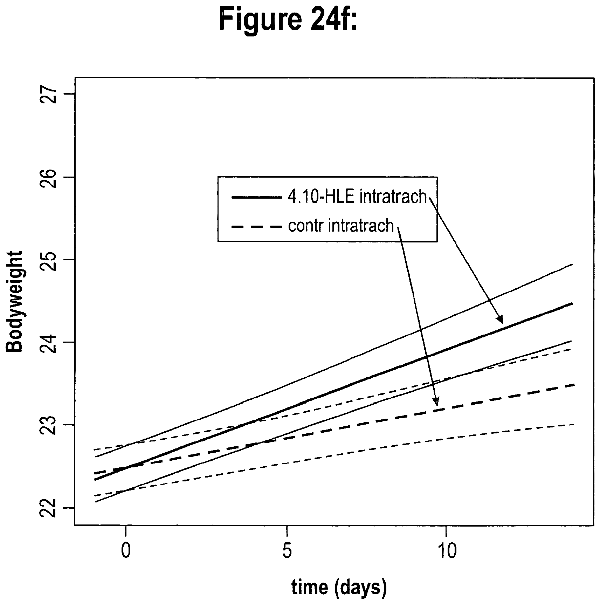

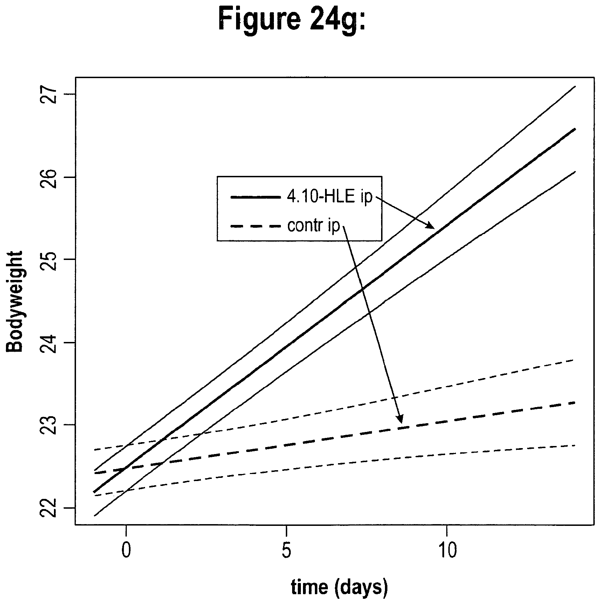

Figure 24: Increase in body weight following i.t. administration of 4 increasing amounts of 4.10 Nanobodies. (a) increase in body weight with 4.10-Alb1 (also referred to as "4.10") via i.p. injections; (b) no increase in body weight with IL6R202 via i.p. injection; (c) increase in body weight with 4.10-Alb1 (also referred to as "4.10") via i.t. administration; (d) no increase in body weight with IL6R202 via i.t. administration; (e) mixed model is a good model for the bodyweight levels; (f) & (g) bodyweight model with corresponding confidence bands (4.10-HLE intratrach = 4.10-Alb1 i.t. administration; contr intratrach = IL6R202 i.t. administration; 4.10-HLE ip = 4.10-Alb1 i.p. injection; contr. Ip = IL6R202 i.p. injection).

### Experimental Part

### Example 1: Pharmacokinetics of RSV NB2, ALX-0081 & RANKL008A in the Male Wistar Rat after Single Intratracheal or Intravenous Administration

**1.1: Table B-1 - test items:**

| Name | Alternative names | SEQ ID NO: | Reference | Amino acid sequence |
|---|---|---|---|---|
| RSV NB2 | 191D3 | 1 | SEQ ID NO:159 in US provisional 61/139,130 | EVQLVESGGGLVQAGGSLRLSCEASGRTYSRYG MGWFRQAPGKEREFVAAVSRLSGPRTVYADSVK GRFTISRDNAENTVYLQMNSLKPEDTAVYTCAAEL TNRNSGAYYYAWAYDYWGQGTQVTVSS |
| ALX-0081 | 12A2H1-3a-12A2H1 | 2 | SEQ ID NO: 98 in WO20061228 25 | EVQLVESGGGLVQPGGSLRLSCAASGRTFSYNP MGWFRQAPGKGRELVAAISRTGGSTYYPDSVEG RFTISRDNAKRMVYLQMNSLRAEDTAVYYCAAAG VRAEDGRVRTLPSEYTFWGQGTQVTVSSAAAEV QLVESGGGLVQPGGSLRLSCAASGRTFSYNPMG WFRQAPGKGRELVAAISRTGGSTYYPDSVEGRFT ISRDNAKRMVYLQMNSLRAEDTAVYYCAAAGVRA EDGRVRTLPSEYTFWGQGTQVTVSS |
| RANKL 008a | | 3 | SEQ ID NO: 759 in WO 2008142164 | EVQLVESGGGLVQPGGSLRLSCAASGFTFSSYPM GWFRQAPGKGREFVSSITGSGGSTYYADSVKGR FTISRDNAKNTLYLQMNSLRPEDTAVYYCAAYIRP DTYLSRDYRKYDYWGQGTLVTVSSGGGGSGGGS EVQLVESGGGLVQPGNSLRLSCAASGFTFSSFGM SWVRQAPGKGLEWVSSISGSGSDTLYADSVKGR FTISRDNAKTTLYLQMNSLRPEDTAVYYCTIGGSL SRSSQGTLVTVSSGGGGSGGGSEVQLVESGGGL VQPGGSLRLSCAASGFTFSSYPMGWFRQAPGKG REFVSSITGSGGSTYYADSVKGRFTISRDNAKNTL YLQMNSLRPEDTAVYYCAAYIRPDTYLSRDYRKY DYWGQGTLVTVSS |

### Animal Model

101 male Wistar rats (approximately 300 gram and 11 weeks old) were used for this study, a strain bred by Charles River Laboratories, Germany. The animals were held for at least 6 days for adaptation. Following the initial health check, the animals were weighed and allocated by means of a computerized randomization program to the test groups; only healthy animals were used.

The sterile test substances were thawed in a water bath at 25°C while swirling gently for 10 minutes. For intratracheal dosing, no further dilutions were required. For intravenous administration, the required amount of test substance was diluted aseptically in sterile DPBS ((Dulbecco's modified) Phosphate Buffered Saline) down to the desired concentrations. The test item formulations were freshly prepared within 4 hours prior to dosing.

### Dose and Route of Administration

The different test groups and the dose levels are given in Table B-2. The i.v. bolus dose was given into a tail vein. The amount of test item for i.v. administration was adjusted to each animal's current body weight. The i.t. dose was administered intratracheally with a syringe with a blunt stainless steel dosing needle, after deep anaesthetization with isoflurane. The amount of test item for i.t. administration was set to 100 µL/animal, irrespective of body weight. The average body weight of intratraceally dosed animals was on average 0.315 kg (RSV NB2 group), 0.317 kg (ALX-0081 group), 0.323 kg (RANKL008A group), corresponding to a mean dose per b.w. were calculated at 3.6 mg/kg (RSV NB2 group), 3.1 mg/kg (ALX-0081 group), 3.2 mg/kg (RANKL008A group),

**Table B-2: Study design**

| Group | Substance | Route | Single Dose (mg/kg) | Number of animals |
|---|---|---|---|---|
| 1 | RSV NB2 | i.v. | 4 | 3 |
| 2 | ALX-0081 | i.v. | 5 | 3 |
| 3 | RANKL008A | i.v. | 5 | 3 |
| 4 | RSV NB2 | i.t. | 3.6 | 28 |
| 5 | ALX-0081 | i.t. | 3.1 | 28 |
| 6 | RANKL008A | i.t. | 3.2 | 28 |
| 7 | - | - | - | 8 |

### Blood and BALF sampling and processing.

After i.v. dosing, blood was sampled (approximately 300 µL) at 0.05, 0.25, 0.5, 1, 2, 4, 6, and 24 hours from the tail vein of RSV NB2- and ALX-0081-dosed animals and at 0.05, 0.25, 0.5, 1, 2, 4, 8, 24, and 48 hours from RANKL008A-dosed animals. All blood samples were placed on melting ice. Within approximately 30 minutes after sampling, the blood samples were centrifuged at 5°C for 10 minutes (1500 g). Citrated plasma was stored in polypropylene tubes at approximately ≤ - 75°C until dispatch on dry ice to the Sponsor.

After intratracheal dosing, blood, lungs, and BALF were collected (at necropsy following deep anaesthesia with isoflurane) at 0.05, 0.333, 1, 2, 4, 6, and 24 hours from RSV NB2-dosed rats and ALX-0081-dosed rats and at 0.05, 0.333, 1, 2, 4, 8 and 24 hours from animals dosed with RANKL008A. By means of an aorta punction 4 mL of blood was withdrawn. Within 42 minutes after sampling, the blood samples were centrifuged at 5°C for 10 minutes (1500 g). Citrated plasma was stored in polypropylene tubes at approximately ≤ -75°C until dispatch on dry ice to the Sponsor. Following the removal of blood, lungs were harvested. First, the lungs including trachea were rinsed with iced DPBS and weighed. Then, BALF was collected. Five mL lavage fluid (DPBS) was carefully put into the lungs. After approximately 10 seconds, as much fluid as possible was returned to the syringe. BALF was transferred to an empty tube and directly stored on melting ice. This procedure was repeated. The second collection of BALF was added to the first collection. The volume of BALF that was collected was documented and reported. Subsequently, BALF was stored at approximately ≤ -75°C until dispatch on dry ice to the Sponsor.

### Determination of RSV NB2 in rat plasma or BALF

96-well microtiter plates (Maxisorp, Nunc-) were coated overnight at 4°C with 100 µL hRSV (12.5 µg/mL, Hytest). Thereafter wells were aspirated, blocked (RT, 1h, PBS-0.1% casein) and washed. The standards, QC, and predilutions of the test samples were prepared in a non-coated (polypropylene) plate in 100% rat plasma or BALF and incubated for 30 min at RT while shaking at 600 rpm. A 1/10 dilution of the samples in PBS-0.1% casein (final concentration of rat plasma or BALF is 10%) was transferred to the coated plate and incubated for 1 hr at RT while shaking at 600 rpm. After three washing steps with PBS-0.05% Tween20, the plates were incubated with polyclonal rabbit anti-Nanobody monoclonal K1 (1/2000 in PBS-0.1% casein, in-house) for 1 hr at RT while shaking at 600 rpm. After 3 washing steps with PBS-0.05% Tween20, 100 µl horseradish peroxidase (HRP) labeled polyclonal goat anti-rabbit (1/2000 in PBS-0.1% casein, DakoCytomation) was incubated for 1 hr at RT while shaking at 600 rpm. Visualization was performed covered from light for 20 min with 100 µL 3,3',5,5'-tetramethylbenzidine (esTMB, SDT, diluted 1/3). After 20 min, the colouring reaction was stopped with 100 µL 1N HCI. The absorbance was determined at 450 nm and corrected for background absorbance at 620 nm. Concentration in each sample was determined based on a sigmoidal standard curve. The lower limit of quantification (LLOQ) and upper limit of quantification (ULOQ) of the different assays are listed in Table B-3.

**Table B-3: LLOQ and ULOQ for determination of RSV NB2 in rat plasma and BALF samples**

| | **LLOQ (ng/ml)** | | **ULOQ (ng/ml)** | |
|---|---|---|---|---|
| **PK ELISA** | Plate level | Plasma/BALF level | Plate level | Plasma/BALF level |
| RSV NB2 | 0.4 | 4.0 | 20.0 | 200.0 |

### Determination of ALX-0081 in rat plasma or BALF

96-well microtiter plates (Maxisorp, Nunc) were coated overnight at 4°C with 100 µL vWF in PBS (2.5 µg/mL, Haemate P1200/500 - ZLB Behring). Thereafter wells were aspirated, blocked (RT, 1h, PBS-0.1% casein) and washed. The standards, QC, and predilutions of the test samples were prepared in a non-coated (polypropylene) plate in 100% rat plasma or BALF and incubated for 30 min at RT while shaking at 600 rpm. A 1/5 dilution of the samples in PBS-0.1% casein (final concentration of rat plasma or BALF is 20%) was transferred to the coated plate and incubated for 1 hr at RT while shaking at 600 rpm. After three washing steps with PBS-0.05% Tween20, the plates were incubated with the anti-ALX0081 NB vWF12B2-GS9-12B2-BIO (1 µg/ml in PBS-0.1% casein, in-house) for 30 min at RT while shaking at 600 rpm. After 3 washing steps with PBS-0.05% Tween20, 100 µl streptavidin-HRP (1/2000 in PBS-0.1% casein, DakoCytomation) was incubated for 30 min at RT while shaking at 600 rpm. Visualization was performed covered from light for 15 min with 100 µL 3,3',5,5'-tetramethylbenzidine (esTMB, SDT, diluted 1/3). After 15 min, the coloring reaction was stopped with 100 µL 1N HCI. The absorbance was determined at 450 nm and corrected for background absorbance at 620 nm. Concentration in each sample was determined based on a sigmoidal standard curve. The LLOQ and ULOQ of the different assays are listed in Table B-4.

**Table B-4: LLOQ and ULOQ for determination of ALX-0081 in rat plasma and BALF samples**

| | **LLOQ (ng/ml)** | | **ULOQ (ng/ml)** | |
|---|---|---|---|---|
| **PK ELISA** | Plate level | Plasma/BALF | Plate level | Plasma/BALF |
| ALX-0081 | 0.75 | 3.75 | 40.0 | 200.0 |

### Determination of RANKL008A in rat plasma or BALF

96-well microtiter plates (Maxisorp, Nunc) were coated overnight at 4°C with 100 µL neutravidin in PBS (2 µg/mL, Pierce,). Wells were aspirated and blocked.. After 3 washing steps with PBS-0.05% Tween20, biotinylated RANKL (0.5 µg/mL in PBS-0.1% casein, in-house,) was captured by incubating 100 µL for 1 hr at RT while shaking at 600 rpm. After this incubation step, wells were washed. The standards, QC, and predilutions of the test samples were prepared in a non-coated (polypropylene) plate in 100% rat plasma or BALF and incubated for 30 min at RT while shaking at 600 rpm. A 1/10 dilution of the samples in PBS-0.1% casein (final concentration of rat plasma or BALF is 10%) was transferred to the coated plate and incubated for 1 hr at RT while shaking at 600 rpm. After three washing steps with PBS-0.05% Tween20, the plates were incubated with polyclonal rabbit anti-Nanobody® monoclonal R23 (1/2000 in PBS-0.1% casein, in-house) for 1 hr at RT while shaking at 600 rpm. After 3 washing steps with PBS-0.05% Tween20, 100 µl horseradish peroxidase (HRP) labelled polyclonal goat anti-rabbit (1/5000 in PBS-0.1% casein, DakoCytomation) was incubated for 1 hr at RT while shaking at 600 rpm. Visualization was performed covered from light for 10 min with 100 µL 3,3',5,5'-tetramethylbenzidine (esTMB, SDT, diluted 1/3). After 10 min, the coloring reaction was stopped with 100 µL 1N HCI. The absorbance was determined at 450 nm and corrected for background absorbance at 620 nm. Concentration in each sample was determined based on a sigmoidal standard curve. The LLOQ and ULOQ of the different assays are listed in Table B-5.

**Table B-5: LLOQ and ULOQ for determination of RANKL008A in rat plasma and BALF samples**

| | **LLOQ (ng/ml)** | | **ULOQ (ng/ml)** | |
|---|---|---|---|---|
| **PK ELISA** | Plate level | Plasma/BALF level | Plate level | Plasma/BALF level |
| RANKL008A | 0.1 | 1.0 | 7.5 | 75.0 |

### Non-Compartmental Pharmacokinetic Data Analysis

Individual plasma and mean BALF concentration-time profiles of all rats were subjected to a non-compartmental pharmacokinetic analysis (NCA) using WinNonlin Professional Software Version 5.1 (Pharsight Corporation, Mountain View California, USA). The pre-programmed Models 200 and 201 were used to analyse the intratracheal and intravenous data, respectively. The linear-up/log down trapezoidal rule was used to calculate the area under the concentration-time data. Nominal times were considered except when the actual time deviated more than 5% of the nominal, the actual time was used. In the calculation of the t1/2, at least 3 data points were considered except where indicated. When at least three individual values were available, mean and SD was calculated.

### 1.3 Results

### Plasma Concentrations of RSV NB2, ALX-0081 and RANKL008A

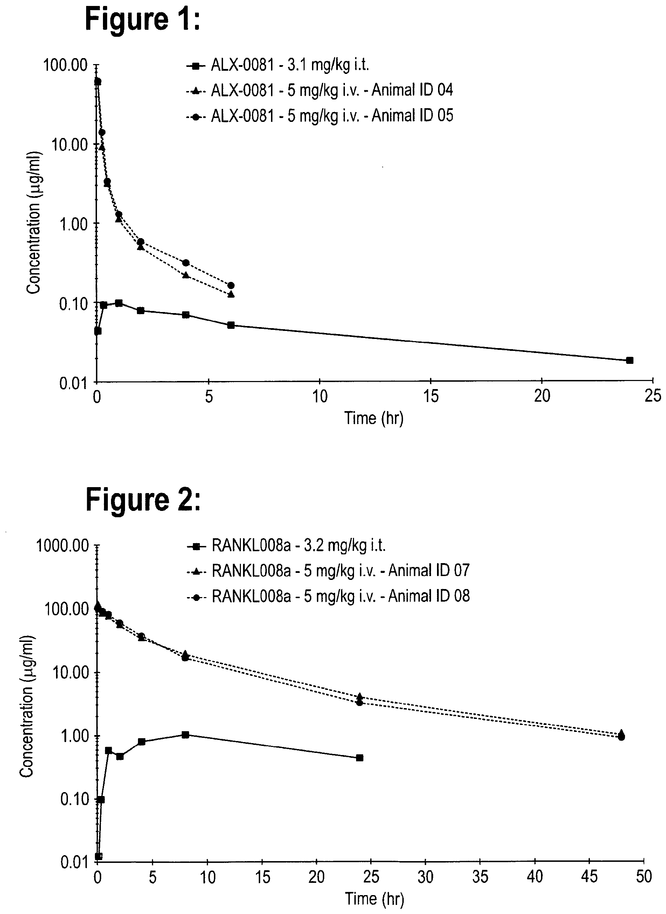

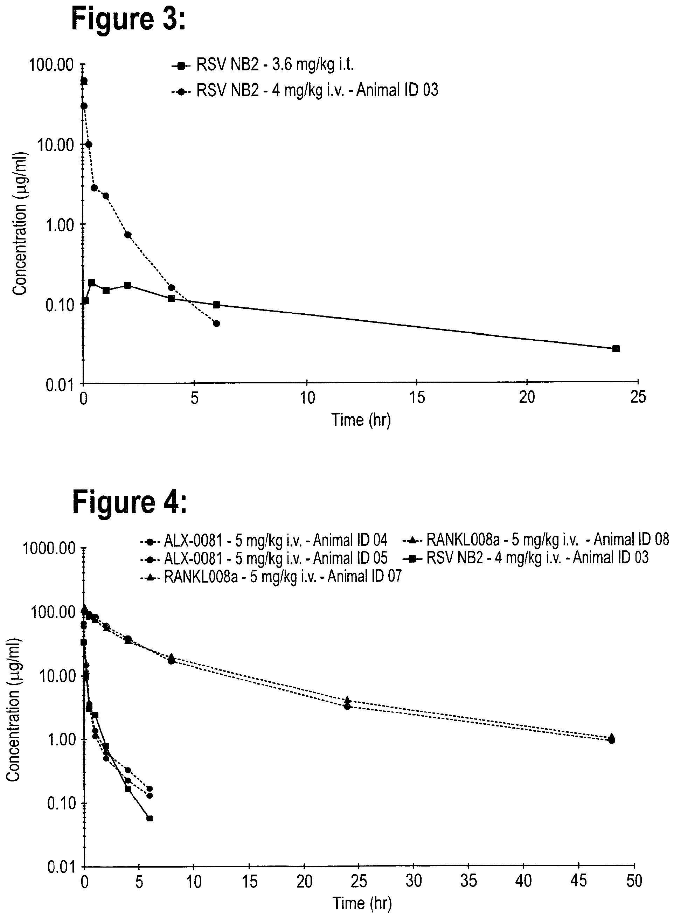

The observed plasma concentration-time data of the individual animals after a single i.v. administration and of the mean (n=4 animals/time-point; destructive sampling) plasma concentration-time data after a single i.t. administration of RSV NB2, ALX-0081, and RANKL008A are shown in Figure 4 (i.v. data for all compounds), Figure 3 (RSV NB2 i.v. and i.t. data), Figure 1 (ALX-0081 i.v. and i.t. data), and Figure 1 (RANKL008A i.v. and i.t. data). The individual (i.v.) and both individual and mean plasma concentrations (i.t.) are listed in Tables B-6, B-7 and B-8, respectively.

**Table B-6: Individual plasma concentration-time data of RSV NB2, ALX-0081, and RANKL008A after a single i.v. bolus dose of RSV NB2 (4 mg/kg), ALX-0081 (5 mg/kg), and RANKL008A (5 mg/kg), respectively, to male Wistar rats.**

| | Plasma concentration after i.v. Administration (µg/mL) | | | | | | | | |
|---|---|---|---|---|---|---|---|---|---|
| | RSV NB2 | | | ALX-0081 | | | RANKL008A | | |
| Nominal Time | ID 1 | ID 2 | ID 3 | ID 4 | ID 5 | ID 6 | ID 7 | ID 8 | ID 9 |
| 3 min | 23.6 | 34.5 | 32.1 | 60.4 | 63.2 | NS | 94.3(1) | 107 | 100 |
| 15 min | 5.16 | 10.7 | 10.6 | 9.18 | 14.1 | NS | 95.7 | 94.8 | 92.8 |
| 30 min | 3.61 | 5.91 | 3 | 3.15 | 3.37 | 4.55 | 88.4 | 85.9 | 74.1 |
| 1 hr | NS(2) | 5.12 | 2.36 | 1.09 | 1.31 | 1.84 | 81.5 | 73.8 | NS |
| 2 hr | NS | NS | 0.763 | 0.498 | 0.594 | NS | 58.7 | 55.9 | NS |
| 4 hr | NS | NS | 0.161 | 0.219 | 0.315 | 0.328 | 35.8 | 35.1 | NS |
| 6 hr | NS | NS | 0.056 | 0.125 | 0.161 | 0.116 | / | / | / |
| 8 hr | /(3) | / | / | / | / | / | 17.1 | 18.8 | NS |
| 24 hr | BQL(4) | NS | BQL | BQL | BQL | BQL | 3.17 | 3.94 | NS |
| 48 hr | / | / | / | / | / | / | 0.902 | 0.988 | NS |
| (1) 5 min instead of 3 min | | | | | | | | | |
| (2) NS: No sample could be obtained due to technical difficulties) | | | | | | | | | |
| (3) No sampling per protocol | | | | | | | | | |
| (4) BQL: Below | | | | | | | | | |
| Quantification Limit | | | | | | | | | |

**Table B-7: Individual plasma concentration-time data of RSV NB2, ALX-0081, and RANKL008A after a single i.t. dose of RSV NB2 (3.6 mg/kg), ALX-0081 (3.1 mg/kg) and RANKL008A (3.2 mg/kg), respectively, to male Wistar rats.**

| | Plasma concentration after i.t. Administration (µg/mL) | | | | | |
|---|---|---|---|---|---|---|
| | RSV NB2 | | ALX-0081 | | RANKL008A | |
| Nominal Time | ID | Concentration | ID | Concentration | ID | Concentration |
| 3 min(1) | 10 | 0.158 | 38 | 0.056 | 66 | 0.004 |
| | 11 | 0.085 | 39 | 0.013 | 67 | 0.030 |
| | 12 | 0.081 | 40 | 0.029 | 68 | 0.006 |
| | 13 | 0.127 | 41 | 0.077 | 69 | 0.005 |
| 20 min | 14 | 0.204 | 42 | 0.102 | 70 | 0.072 |
| | 15 | 0.167 | 43 | 0.102 | 71 | 0.081 |
| | 16 | 0.131 | 44 | 0.097 | 72 | 0.151 |
| | 17 | 0.267 | 45 | 0.070 | 73 | 0.083 |
| 1 hr | 18 | 0.202 | 46 | 0.122 | 74 | 0.401 |
| | 19 | 0.167 | 47 | 0.112 | 75 | 0.541 |
| | 20 | 0.120 | 48 | 0.049 | 76 | 0.305 |
| | 21 | 0.120 | 49 | 0.109 | 77 | 1.077 |
| 2 hr | 22 | BQL | 50 | 0.041 | 78 | 0.279 |
| | 23 | 0.230 | 51 | 0.100 | 79 | 0.389 |
| | 24 | 0.091 | 52 | 0.084 | 80 | 0.705 |
| | 25 | 0.202 | 53 | 0.091 | 81 | 0.489 |
| 4 hr | 26 | 0.113 | 54 | 0.069 | 82 | 0.965 |
| | 27 | 0.150 | 55 | 0.077 | 83 | 0.601 |
| | 28 | 0.080 | 56 | 0.053 | 84 | 0.934 |
| | 29 | 0.129 | 57 | 0.085 | 85 | 0.672 |
| 6/8 hr(3) | 30 | 0.125 | 58 | 0.034 | 86 | 0.869 |
| | 31 | 0.071 | 59 | 0.048 | 87 | 1.42 |
| | 32 | 0.108 | 60 | 0.070 | 88 | 1.16 |
| | 33 | 0.091 | 61 | 0.059 | 89 | 0.606 |
| 24 hr | 34 | 0.024 | 62 | 0.014 | 90 | 0.493 |
| | 35 | 0.024 | 63 | 0.022 | 91 | 0.450 |
| | 36 | 0.025 | 64 | 0.014 | 92 | 0.434 |
| | 37 | 0.036 | 65 | 0.020 | 93 | 0.342 |

| | | | | | | |
|---|---|---|---|---|---|---|
| (1) 4 min instead of 3 min (2) BQL: below the limit of quantification (3) 6 hr for RSV NB2 and ALX-0081, 8 hr for RANKL008A. | | | | | | |

**Table B-8: Mean (n=4) plasma concentration-time data of RSV NB2, ALX-0081, and RANKL008A after a single i.t. dose of RSV NB2 (3.6 mg/kg), ALX-0081 (3.1 mg/kg) and RANKL008A (3.2 mg/kg), respectively, to male Wistar rats.**

| | Plasma concentration after i.t. Administration (µg/mL) | | | | | |
|---|---|---|---|---|---|---|
| | RSV NB2 (ID 10-37) | | ALX-0081 (ID 38-65) | | RANKL008A (ID 66-93) | |
| Nominal Time | Average | SD | Average | SD | Average | SD |
| 3 min | 0.113 | 0.037 | 0.044 | 0.028 | 0.012 | 0.013 |
| 20 min | 0.192 | 0.058 | 0.093 | 0.015 | 0.097 | 0.037 |
| 1 hr | 0.152 | 0.040 | 0.098 | 0.033 | 0.581 | 0.345 |
| 2 hr | 0.175(1) | 0.074 | 0.079 | 0.026 | 0.465 | 0.181 |
| 4 hr | 0.118 | 0.030 | 0.071 | 0.014 | 0.793 | 0.184 |
| 6 hr | 0.099 | 0.023 | 0.052 | 0.015 | /(1) | / |
| 8 hr | / | / | / | / | 1.01 | 0.35 |
| 24 hr | 0.027 | 0.006 | 0.018 | 0.004 | 0.430 | 0.063 |

| | | | | | | |
|---|---|---|---|---|---|---|
| (1) N=3 (2) No sampling planned per protocol | | | | | | |

### Plasma Pharmacokinetic Analysis of RSV NB2, ALX-0081, and RANKL008A

An overview of the basic pharmacokinetic parameters obtained by non-compartmental PK analysis of RSV NB2 (4 mg/kg i.v. & 3.6 mg/kg i.t.), ALX-0081 (5 mg/kg i.v. & 3.1 mg/kg i.t.) and RANKL008A (5 mg/kg i.v. & 3.2 mg/kg i.t.) is given in Tables B9-B11.

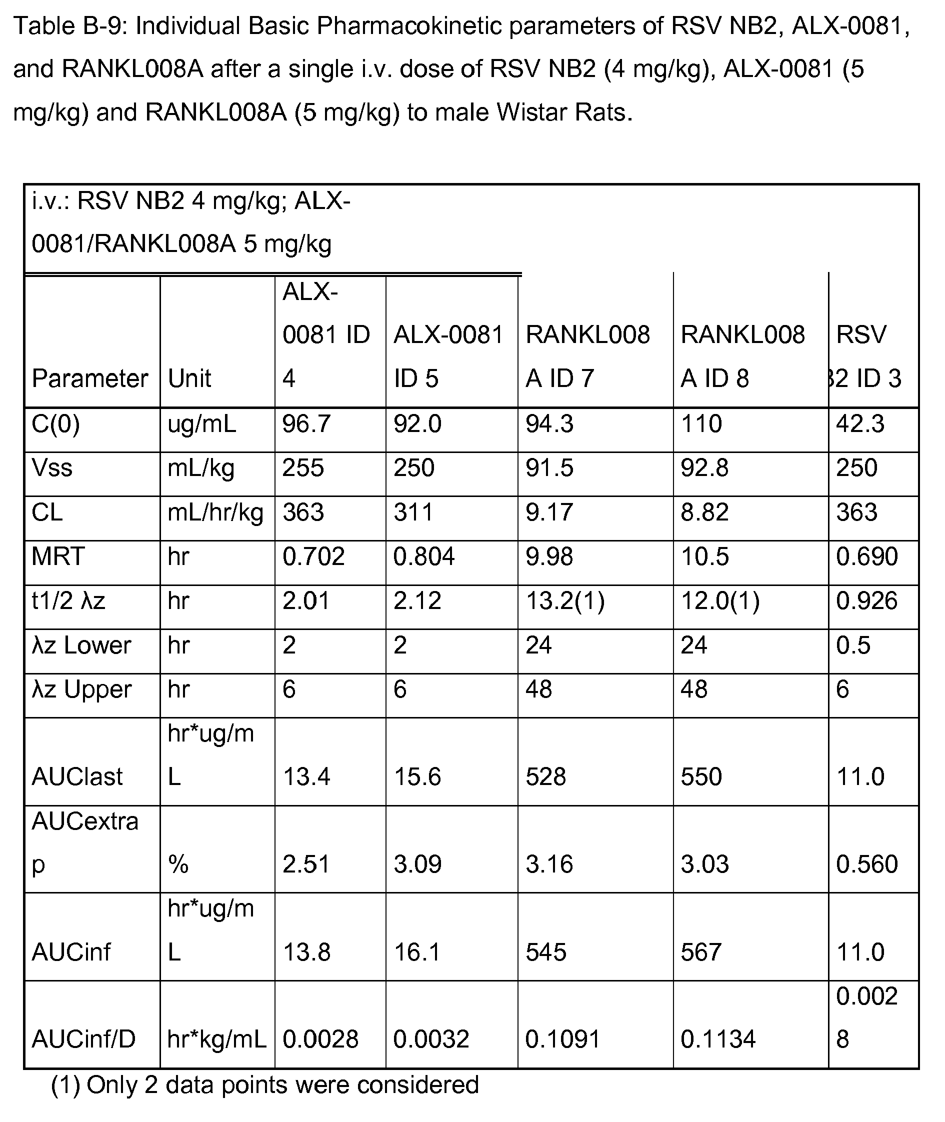

**Table B-10: Mean Basic Pharmacokinetic parameters of RSV NB2, ALX-0081, and RANKL008A after a single i.v. dose of RSV NB2 (4 mg/kg), ALX-0081 (5 mg/kg) and RANKL008A (5 mg/kg) to Wistar Rats.**

| i.v.: RSV NB2 4 mg/kg; ALX- | | | | | | |
|---|---|---|---|---|---|---|
| 0081/RAN KL008A 5 mg/kg | | | | | | |
| Parameter | Unit | ALX-0081 | | RANKL008A | | RSV NB2 |
| | | Average | CV% | Average | CV% | |
| C(0) | ug/mL | 94.3 | 4 | 102 | 11 | 42.3 |
| Vss | mL/kg | 252 | 1 | 92.1 | 1 | 250 |
| CL | mL/hr/kg | 337 | 11 | 9.00 | 3 | 363 |
| MRT | hr | 0.753 | 10 | 10.2 | 4 | 0.690 |
| t1/2 λz | hr | 2.06 | 4 | 12.6(1) | 7 | 0.926 |
| λz Lower | hr | 2 | 0 | 24 | 0 | 0.5 |
| λz Upper | hr | 6 | 0 | 48 | 0 | 6 |
| AUClast | hr*ug/mL | 14.5 | 10 | 539 | 3 | 11.0 |
| AUCextrap | % | 2.80 | 15 | 3.09 | 3 | 0.560 |
| AUCinf | hr*ug/mL | 14.9 | 11 | 556 | 3 | 11.0 |
| AUCinf/D | hr*kg/mL | 0.003 | 9 | 0.111 | 3 | 0.003 |

| | | | | | | |
|---|---|---|---|---|---|---|
| (1) Only 2 data points were considered | | | | | | |

**Table B-11: Basic Pharmacokinetic parameters of RSV NB2, ALX-0081, and RANKL008A after a single i.v. dose of RSV NB2 (3.6 mg/kg), ALX-0081 (3.1 mg/kg) and RANKL008A (3.2 mg/kg) to Wistar Rats.**

| i.t. administration | | | | |
|---|---|---|---|---|
| Parameter | Unit | ALX-0081 3.1 mg/kg | RANKL008A 3.2 mg/kg | RSV NB2 3.6 mg/kg |
| Vss/F | mL/kg | 36339 | 2833 | 21853 |
| CL/F | mL/hr/kg | 2407 | 130 | 1641 |
| MRT | hr | 15.1 | 21.7 | 13.3 |
| t1/2 λz | hr | 10.5 | 13.0(1) | 9.48 |
| λz Lower | hr | 2 | 8 | 4 |
| λz Upper | hr | 24 | 24 | 24 |
| | | | | |
| AUClast | hr*ug/mL | 1.02 | 16.5 | 1.83 |
| AUCextrap | % | 20.8 | 32.8 | 16.8 |
| AUCinf | hr*ug/mL | 1.29 | 24.6(2) | 2.19 |
| tmax | hr | 1 | 8 | 0.330 |
| Cmax | ug/ml | 0.098 | 1.01 | 0.192 |
| AUCinf/D | hr*kg/mL | 0.0004 | 0.0077 | 0.0006 |
| F | % | 13.9 | 6.90 | 22.1 |
| (1) Only 2 data points were considered | | | | |
| (2) Interpret with caution due to high % extrapolated AUC | | | | |
| *Vss*/*F* = *MRT*CL (MRT not corrected for MAT)* | | | | |
| *Estimation F incorrect if CL i.v. and CL i.t. are different;* | | | | |
| *Note dose i.v.* ≠ *i.t.* | | | | |

The PK parameters discussed herein were obtained using non-compartmental analysis (NCA). For rat 1 and 2 (RSV NB2 i.v.), rat 6 (ALX-0081 i.v.) and rat 9 (RANKL008A i.v.) difficulties in blood sampling occurred, and due to the limited data, these animals were excluded from subsequent pharmacokinetic calculations. The terminal parameters for some of the animals were calculated based on only two data-points (R² indicated in red in the tables) in the terminal phase, and should thus be interpreted with caution.

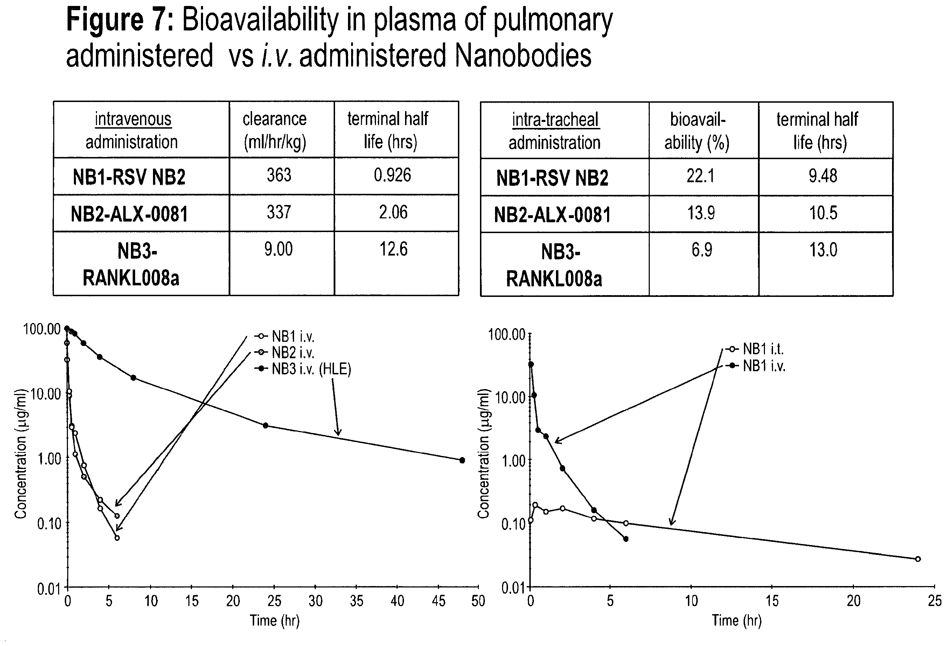

After i.v. administration of RSV NB2 (4 mg/kg) and ALX-0081 (5 mg/kg) comparable plasma PK profiles were observed (Figure 7). This was also reflected in similar pharmacokinetic parameters for the monovalent RSV NB2 and bivalent ALX-0081. The mean clearance was estimated at 363 mL/hr/kg and 337 mL/hr/kg for RSV NB2- and ALX-0081-dosed rats. The corresponding mean Vss values were 250 mL/kg (RSV NB2) and 252 mL/kg (ALX-0081). The plasma concentrations of these Nanobodies® were only detectable up to six hours (detection limits of ca 4 ng/mL) and the terminal half-lives were calculated at 0.926 hours for RSV NB2 and 2.06 hours for ALX-0081. For the trivalent RANKL008A administered intravenously (5 mg/kg), substantially lower mean clearance (9.00 mL/hr/kg) and Vdss values (92.1 mL/kg) were calculated. The terminal half-lives was appreciably longer (12.6 hours). This is explained by the fact that RANKL008A is a half-life extended Nanobody (through binding of the ALB8 component) which is cross reactive with rat albumin, albeit with lower affinity relative to human serum albumin.

After i.t. administration of RSV NB2 (3.6 mg/kg), ALX-0081 (3.1 mg/kg) and RANKL008A (3.2 mg/kg), comparable terminal half-lives in the plasma were observed for the three Nanobodies® (RSV NB2: 9.48 hr, ALX-0081: 10.5 hr and RANKL008A: 13.0 hr). For RSV NB2 and ALX-0081 the half-lives were longer after i.t. administration than after i.v. administration. It is conceivable that for these rapidly cleared compounds, the absorption is the rate limiting step resulting in flip-flop kinetics (i.e. kinetics are absorption rate controlled and the terminal phase is driven by the slow absorption from the site of administration (the lung) to the systemic circulation).

The exposure after i.t. administration was lower for all Nanobodies as compared to that after i.v. administration. This resulting bioavailabilities were 22.1%, 13.9%, and 6.9% for RSV NB2 (16.6 kD), ALX-0081 (27.9 kD), and RANKL008A (40.9 kD), respectively. The bioavailability seems to decrease with increasing molecular weight, but this trend needs to be confirmed when more data become available.

For lung topical applications (RSV NB2), a high pulmonary exposure is desired. It could be expected that a faster and more complete absorption (resulting in a higher bioavailability) would not benefit pulmonary exposure. Therefore, RSV Nanobodies with a higher molecular weight (e.g. a trivalent RSV Nanobody) could possibly lead to enhanced local (pulmonary) exposures and reduced systemic exposures.

The current data indicate that systemic exposure to Nanobodies can be achieved after intratracheal administration, suggesting that the pulmonary route may be viable as non-invasive method of delivery of Nanobodies. In addition, the use of specific delivery formulations and/or devices could significantly improve bioavailability after pulmonary application. It is suggested that the bioavailability may be improved around 5 times in animals (i.t. vs. aerosol - see e.g. table 2 in Patton J., Fishburn S., Weers J. The Lung as a Portal of Entry for Systemic Drug Delivery. 2004. Proc Am Thorac Soc Vol 1. pp 338-344).

### BALF Concentrations of RSV NB2, ALX-0081 and RANKL008A

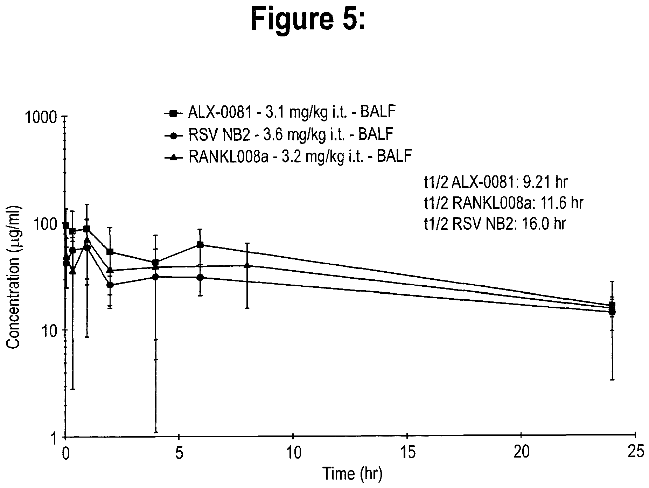

The mean observed BALF concentration-time profiles after a single intratracheal administration of RSV NB2, ALX-0081 and RANKL008A to male rats is shown in Figure 5. Individual and mean BALF concentrations are listed in Tables B-12 and B-13, respectively.

**Table B-12: Individual observed BALF concentrations of RSV NB2, ALX-0081, and RANKL008A after a single i.t. administration of RSV NB2 (3.6 mg/kg), ALX-0081 (3.1 mg/kg) and RANKL008A (3.2 mg/kg) to male rats.**

| BALF concentrations after i.t. Administration (µg/mL) | | | | | | |
|---|---|---|---|---|---|---|
| Nominal Time | RSV NB2 | | ALX-0081 | | RANKL008A | |
| | ID | Concentration | ID | Concentration | ID | Concentration |
| 3 min(1) | 10 | 46.2 | 38 | 145 | 66 | 32.3 |
| | 11 | 65.0 | 39 | 57.9 | 67 | 56.1 |
| | 12 | 23.0 | 40 | 69.2 | 68 | 27.0 |
| | 13 | 36.7 | 41 | 115 | 69 | 80.2 |
| 20 min | 14 | 32.8 | 42 | 40.4 | 70 | 14.4 |
| | 15 | 54.8 | 43 | 148 | 71 | 87.9 |
| | 16 | 70.2 | 44 | 93.4 | 72 | 43.3 |
| | 17 | 68.1 | 45 | 55.7 | 73 | 22.4 |
| 1 hr | 18 | 134 | 46 | 179 | 74 | 124 |
| | 19 | 50.7 | 47 | 80.6 | 75 | 70.3 |
| | 20 | 35.8 | 48 | 62.4 | 76 | 33.8 |
| | 21 | 18.4 | 49 | 35.8 | 77 | 49.8 |
| 2 hr | 22 | BQL(2) | 50 | 33.7 | 78 | 16.1 |
| | 23 | 22.1 | 51 | 36.9 | 79 | 58.3 |
| | 24 | 26.1 | 52 | 111 | 80 | 49.0 |
| | 25 | 32.6 | 53 | 37.1 | 81 | 22.3 |
| 4 hr | 26 | 14.9 | 54 | 32.7 | 82 | 24.8 |
| | 27 | 60.9 | 55 | 2.44 | 83 | 11.4 |
| | 28 | 45.0 | 56 | 85.1 | 84 | 95.0 |
| | 29 | 4.81 | 57 | 50.5 | 85 | 24.9 |
| 6/8 hr(3) | 30 | 24.4 | 58 | 36.2 | 86 | 15.6 |
| | 31 | 43.6 | 59 | 90.1 | 87 | 42.1 |
| | 32 | 21.6 | 60 | 51.9 | 88 | 72.4 |
| | 33 | 33.1 | 61 | 74.6 | 89 | 30.2 |
| 24 hr | 34 | 9.53 | 62 | 20.9 | 90 | 32.7 |
| | 35 | 19.1 | 63 | 13.2 | 91 | 14.6 |
| | 36 | 10.7 | 64 | 16.5 | 92 | 7.48 |
| | 37 | 17.0 | 65 | 14.6 | 93 | 6.91 |

| | | | | | | |
|---|---|---|---|---|---|---|
| (1) 4 min instead of 3 min (2) Below the quantification limit (3) 6 h for RSV NB2 and ALX-0081; 8 h for RANKL008A | | | | | | |

**Table B-13: Mean observed BALF concentrations of RSV NB2, ALX-0081, and RANKL008A after a single i.t. administration of RSV NB2 (3.6 mg/kg), ALX-0081 (3.1 mg/kg) and RANKL008A (3.2 mg/kg) to male rats.**

| BALF concentration after i.t. Administration (µg/mL) | | | | | | |
|---|---|---|---|---|---|---|
| | ALX-0081 (ID 38-65) | | RANKL008A (ID 66-93) | | RSV NB2 (ID 10-37) | |
| Nominal Time | Average | SD | Average | SD | Average | SD |
| 3 min | 96.8 | 40.4 | 48.9 | 24.4 | 42.7 | 17.6 |
| 20 min | 84.3 | 47.9 | 35.7 | 32.9 | 56.5 | 17.2 |
| 1 hr | 89.4 | 62.4 | 69.4 | 39.2 | 59.7 | 51.1 |
| 2 hr | 54.6 | 37.5 | 36.4 | 20.4 | 26.9 | 5.3 |
| 4 hr | 42.7 | 34.6 | 39 | 37.9 | 31.4 | 26.1 |
| 6 hr | 63.2 | 23.9 | 40.1 | 24.1 | /(2) | / |
| 8 hr | / | / | / | / | 30.7 | 9.9 |
| 24 hr | 16.3 | 3.4 | 15.4 | 12.1 | 14.1 | 4.7 |

| | | | | | | |
|---|---|---|---|---|---|---|
| (1) 4 min instead of 3 min (2) No sampling scheduled | | | | | | |

The terminal half-lives of the three Nanobodies in BALF were based on the two last data-points only, and should therefore be interpreted with caution. Of note is also that there was quite some inter-individual variability as indicated by the large standard deviations (see Table B-13). After i.t. administration, comparable terminal half-lives were observed in plasma (RSV NB2 9.48 hr, ALX-0081 10.5 hr and RANKL008A 13.0 hr) and in BALF (RSV NB2 16.0 hr, ALX-0081 9.21 hr and RANKL008A 11.6 hr), supporting the notion that the plasma kinetics are likely absorption rate controlled.

Following intratracheal administration, exposure to the RSV NB2, ALX-0081, RANKL008A Nanobodies exposure was observed for at least 24 hours in BALF (i.e. the last sampling time for BALF).

### Amounts of RSV NB2, ALX-0081 and RANKL008A in BALF

After intratracheal dosing broncho-alveolar lavage fluid (BALF) was collected at necropsy as described above.
Theoretically, the amount of Nanobody in the lung at a given time-point can be obtained by multiplying the measured concentration of each BALF sample by the volume of DPBS added (10 mL), provided that the Nanobody® is efficiently washed out. These individual calculated amounts and their corresponding mean (+SD) values are listed in Table B-14 and B-15, respectively.

**Table B-14: Individual theoretical amount (BALF Concentration x 10 mL) of RSV NB2, ALX-0081, and RANKL008A in BALF after single i.t. administration of RSV NB2 (3.6 mg/kg), ALX-0081 (3.1 mg/kg) and RANKL008A (3.2 mg/kg) to male Wistar rats.**

| BALF Theoretical Amount after i.t. Administration (µg) | | | | | | |
|---|---|---|---|---|---|---|
| Nominal Time | RSV NB2 | | ALX-0081 | | RANKL008A | |
| | ID | Amount | ID | Amount | ID | Amount |
| 3 min(1) | 10 | 462 | 38 | 1446 | 66 | 323 |
| | 11 | 650 | 39 | 579 | 67 | 561 |
| | 12 | 230 | 40 | 692 | 68 | 270 |
| | 13 | 367 | 41 | 1155 | 69 | 802 |
| 20 min | 14 | 328 | 42 | 404 | 70 | 144 |
| | 15 | 548 | 43 | 1479 | 71 | 879 |
| | 16 | 702 | 44 | 934 | 72 | 433 |
| | 17 | 681 | 45 | 557 | 73 | 224 |
| 1 hr | 18 | 1338 | 46 | 1788 | 74 | 1238 |
| | 19 | 507 | 47 | 806 | 75 | 703 |
| | 20 | 358 | 48 | 624 | 76 | 338 |
| | 21 | 184 | 49 | 358 | 77 | 498 |
| 2 hr | 22 | BQL(2) | 50 | 337 | 78 | 161 |
| | 23 | 221 | 51 | 369 | 79 | 583 |
| | 24 | 261 | 52 | 1109 | 80 | 490 |
| | 25 | 326 | 53 | 371 | 81 | 223 |
| 4 hr | 26 | 149 | 54 | 327 | 82 | 248 |
| | 27 | 609 | 55 | 24.4 | 83 | 114 |
| | 28 | 450 | 56 | 851 | 84 | 950 |
| | 29 | 48.1 | 57 | 505 | 85 | 249 |
| 6/8 hr(3) | 30 | 244 | 58 | 362 | 86 | 156 |
| | 31 | 436 | 59 | 901 | 87 | 421 |
| | 32 | 216 | 60 | 519 | 88 | 724 |
| | 33 | 331 | 61 | 746 | 89 | 302 |
| 24 hr | 34 | 95.3 | 62 | 209 | 90 | 327 |
| | 35 | 191 | 63 | 132 | 91 | 146 |
| | 36 | 107 | 64 | 165 | 92 | 74.8 |
| | 37 | 170 | 65 | 146 | 93 | 69.1 |

| | | | | | | |
|---|---|---|---|---|---|---|
| (1) 4 min instead of 3 min (2) Below the quantification limit (3) 6 h for RSV NB2 and ALX-0081; 8 h for RANKL008A | | | | | | |

**Table B-15: Mean (+/- SD; n=4) theoretical amount (BALF Concentration x 10 mL) of RSV NB2, ALX-0081, and RANKL008A in BALF after single i.t. administration of RSV NB2 (3.6 mg/kg), ALX-0081 (3.1 mg/kg) and RANKL008A (3.2 mg/kg) to male Wistar rats.**

| | BALF theoretical amount after i.t. Administration (µg) | | | | | |
|---|---|---|---|---|---|---|
| | RSV NB2 (ID 10-37) | | ALX-0081 (ID 38-65) | | RANKL008A (ID 66-93) | |
| Nominal Time | Average | SD | Average | SD | Average | SD |
| 3 min(1) | 427 | 176 | 968 | 404 | 489 | 244 |
| 20 min | 565 | 172 | 843 | 479 | 420 | 329 |
| 1 hr | 597 | 511 | 894 | 624 | 694 | 392 |
| 2 hr | 269 | 53 | 546 | 375 | 364 | 204 |
| 4 hr | 314 | 261 | 427 | 346 | 390 | 379 |
| 6 hr | 307 | 99 | 632 | 239 | /(2) | / |
| 8 hr | / | / | / | / | 401 | 241 |
| 24 hr | 141.0 | 47.2 | 163 | 34 | 154 | 121 |

| | | | | | | |
|---|---|---|---|---|---|---|
| (1) 4 min instead of 3 min (2) No sampling scheduled | | | | | | |

Note however that large variations occurred in the recovery of the BALF. For some animals it was possible to recover 9.5 mL fluid after injecting 10 mL DPBS, while for other animals only 3 mL was recovered. Furthermore, since the lavage is performed twice and combined in a single vial, it is impossible to determine how much volume was recovered from the first or second lavage separately. In addition, it is also unknown whether there are differences in the concentration of the first and second lavage.
The result is that overestimations of the true amount of Nanobody may occur when the measured BALF concentrations are simply multiplied with the theoretical volume of 10 mL DPBS.
Alternatively, if the amount of Nanobody is estimated by multiplying the measured concentration of each BALF sample by the actual recovered volume of BALF, this may result in underestimations of the actual amount of Nanobody in case significant amounts of Nanobody are present in unrecovered BALF.
Therefore, the true amount of Nanobody in BALF should theoretically be comprised between the amount calculated via the theoretical BALF volume and the actual BALF volume. It is important to note that the larger the recovered volume, the more accurate the calculations are expected to be. Since the average recovered volume is on average ca. 7 mL (Table B-16), both calculation methods should not provide very different results. The individual calculated amounts and mean (+SD) values based on actual recovered volumes are listed in Table B-17 and B-18, respectively.

**Table B-16: Individual recovered volume of BALF after two lavages with DPBS (2x5mL) after a single i.t. administration of RSV NB2 (3.6 mg/kg), ALX-0081 (3.1 mg/kg) and RANKL008A (3.2 mg/kg) to male Wistar rats.**

| | Recovered Volume of BALF after lavages | | | | | |
|---|---|---|---|---|---|---|
| | RSV NB2 | | ALX-0081 | | RANKL008A | |
| Nominal Time | ID | BALF (mL) | ID | BALF (mL) | ID | BALF (mL) |
| 3 min(1) | 10 | 5.5 | 38 | 7.5 | 66 | 8.0 |
| | 11 | 6.5 | 39 | 6.5 | 67 | 8.0 |
| | 12 | 8.5 | 40 | 8.5 | 68 | 4.0 |
| | 13 | 7.5 | 41 | 7.5 | 69 | 8.5 |
| 20 min | 14 | 8.0 | 42 | 7.0 | 70 | 7.5 |
| | 15 | 6.0 | 43 | 8.0 | 71 | 3.0 |
| | 16 | 6.5 | 44 | 8.0 | 72 | 6.0 |
| | 17 | 8.5 | 45 | 7.5 | 73 | 8.0 |
| 1 hr | 18 | 6.5 | 46 | 8.0 | 74 | 7.0 |
| | 19 | 6.5 | 47 | 7.5 | 75 | 6.0 |
| | 20 | 7.5 | 48 | 8.0 | 76 | 7.5 |
| | 21 | 7.5 | 49 | 7.0 | 77 | 8.0 |
| 2 hr | 22 | 5.5 | 50 | 8.0 | 78 | 6.0 |
| | 23 | 6.0 | 51 | 8.0 | 79 | 7.5 |
| | 24 | 6.5 | 52 | 6.5 | 80 | 8.0 |
| | 25 | 7.0 | 53 | 7.5 | 81 | 8.0 |
| 4 hr | 26 | 5.5 | 54 | 8.0 | 82 | 7.0 |
| | 27 | 5.0 | 55 | 8.0 | 83 | 6.5 |
| | 28 | 9.5 | 56 | 9.0 | 84 | 7.0 |
| | 29 | 8.0 | 57 | 7.5 | 85 | 7.5 |
| 6/8 | | | | | | |
| hr(2) | 30 | 7.0 | 58 | 8.0 | 86 | 7.0 |
| | 31 | 7.0 | 59 | 9.0 | 87 | 6.5 |
| | 32 | 7.0 | 60 | 6.0 | 88 | 7.5 |
| | 33 | 8.5 | 61 | 8.5 | 89 | 9.0 |
| 24 hr | 34 | 6.5 | 62 | 7.5 | 90 | 8.0 |
| | 35 | 6.5 | 63 | 7.5 | 91 | 7.5 |
| | 36 | 7.5 | 64 | 8.5 | 92 | 8.0 |
| | 37 | 7.0 | 65 | 6.5 | 93 | 5.5 |

| | | | | | | |
|---|---|---|---|---|---|---|
| (1) 4 min instead of 3 min (2) 6 h for RSV NB2 and ALX-0081; 8h for RANKL008A | | | | | | |

**Table B-17: Individual actual amount (BALF Concentration x recovered volume) of RSV NB2, ALX-0081, and RANKL008A in BALF after a single intratracheal administration of RSV NB2 (3.6 mg/kg), ALX-0081 (3.1 mg/kg) and RANKL008A (3.2 mg/kg) to male rats.**

| | BALF Actual Amount after i.t. Administration (µg) | | | | | |
|---|---|---|---|---|---|---|
| | RSV NB2 | | ALX-0081 | | RANKL008A | |
| Nominal Time | ID | Amount | ID | Amount | ID | Amount |
| 3 min(1) | 10 | 254 | 38 | 1084 | 66 | 258 |
| | 11 | 422 | 39 | 377 | 67 | 449 |
| | 12 | 195 | 40 | 588 | 68 | 108 |
| | 13 | 275 | 41 | 866 | 69 | 682 |
| 20 min | 14 | 262 | 42 | 283 | 70 | 108 |
| | 15 | 329 | 43 | 1183 | 71 | 264 |
| | 16 | 456 | 44 | 747 | 72 | 260 |
| | 17 | 579 | 45 | 418 | 73 | 179 |
| 1 hr | 18 | 869 | 46 | 1430 | 74 | 867 |
| | 19 | 330 | 47 | 605 | 75 | 422 |
| | 20 | 269 | 48 | 499 | 76 | 254 |
| | 21 | 138 | 49 | 250 | 77 | 399 |
| 2 hr | 22 | BDL | 50 | 270 | 78 | 96.4 |
| | 23 | 132 | 51 | 295 | 79 | 438 |
| | 24 | 170 | 52 | 721 | 80 | 392 |
| | 25 | 228 | 53 | 278 | 81 | 179 |
| 4 hr | 26 | 81.9 | 54 | 262 | 82 | 174 |
| | 27 | 305 | 55 | 19.5 | 83 | 74.3 |
| | 28 | 428 | 56 | 766 | 84 | 665 |
| | 29 | 38.5 | 57 | 379 | 85 | 187 |
| 6/8 hr(2) | 30 | 171 | 58 | 289 | 86 | 109 |
| | 31 | 305 | 59 | 811 | 87 | 274 |
| | 32 | 151 | 60 | 311 | 88 | 543 |
| | 33 | 281 | 61 | 634 | 89 | 272 |
| 24 hr | 34 | 62.0 | 62 | 157 | 90 | 262 |
| | 35 | 124 | 63 | 98.7 | 91 | 110 |
| | 36 | 80.0 | 64 | 140 | 92 | 59.9 |
| | 37 | 119 | 65 | 95.2 | 93 | 38.0 |

| | | | | | | |
|---|---|---|---|---|---|---|
| (1) 4 min instead of 3 min (2) 6 h for RSV NB2 and ALX-0081; 8h for RANKL008A | | | | | | |

**Table B-18: Mean actual amount (BALF Concentration x recovered volume) of RSV NB2, ALX-0081, and RANKL008A in BALF after a single intratracheal administration RSV NB2 (3.6 mg/kg), ALX-0081 (3.1 mg/kg) and RANKL008A (3.2 mg/kg) to male rats.**

| | BALF actual amount after i.t. Administration (µg) | | | | | |
|---|---|---|---|---|---|---|
| | RSV NB2 (ID 10-37) | | ALX-0081 (ID 38-65) | | RANKL008A (ID 66-93) | |
| Nominal Time | Average | SD | Average | SD | Average | SD |
| 3 min(1) | 287 | 97 | 729 | 310 | 374 | 248 |
| 20 min | 406 | 140 | 658 | 401 | 203 | 74 |
| 1 hr | 401 | 322 | 696 | 512 | 485 | 265 |
| 2 hr | 177 | 48 | 391 | 220 | 276 | 165 |
| 4 hr | 213 | 185 | 357 | 311 | 275 | 265 |
| 6 hr | 227 | 77 | 512 | 254 | /(2) | / |
| 8 hr | / | / | / | / | 299 | 180 |
| 24 hr | 96.5 | 30.4 | 123 | 30 | 117 | 101 |

| | | | | | | |
|---|---|---|---|---|---|---|
| (1) 4 min instead of 3 min (2) No sampling scheduled per protocol | | | | | | |

By dividing the calculated amount of Nanobody® by the actual amount dosed (RSV NB2: 1.14 mg, ALX-0081: 0.985 mg, RANKL008A: 1.03 mg), the recovered fraction of the dose (expressed as %) was calculated. Individual amounts and their corresponding mean (+SD) values, expressed as % of the administered dose, and based on the theoretical BALF volume (10 mL) and actual recovered volumes are listed in Tables B-19 to B-22.

**Table B-19: Individual theoretical amount (BALF Concentration x 10 mL) expressed as % of the dose of RSV NB2, ALX-0081, and RANKL008A in BALF after a single i.t. administration of RSV NB2 (3.6 mg/kg), ALX-0081 (3.1 mg/kg) and RANKL008A (3.2 mg/kg) to male Wistar rats.**

| | BALF Theoretical Amount expressed as % of the dose | | | | | |
|---|---|---|---|---|---|---|
| | RSV NB2 | | ALX-0081 | | RANKL008A | |
| Nominal Time | ID | Amount/D (%) | ID | Amount/D (%) | ID | Amount/D (%) |
| 3 min(1) | 10 | 40.5 | 38 | 147 | 66 | 31.3 |
| | 11 | 57.0 | 39 | 58.8 | 67 | 54.4 |
| | 12 | 20.2 | 40 | 70.2 | 68 | 26.2 |
| | 13 | 32.2 | 41 | 117 | 69 | 77.8 |
| 20 min | 14 | 28.7 | 42 | 41.0 | 70 | 14.0 |
| | 15 | 48.1 | 43 | 150 | 71 | 85.4 |
| | 16 | 61.6 | 44 | 94.8 | 72 | 42.0 |
| | 17 | 59.7 | 45 | 56.5 | 73 | 21.8 |
| 1 hr | 18 | 117.3 | 46 | 182 | 74 | 120 |
| | 19 | 44.5 | 47 | 81.8 | 75 | 68.3 |
| | 20 | 31.4 | 48 | 63.3 | 76 | 32.8 |
| | 21 | 16.2 | 49 | 36.3 | 77 | 48.4 |
| 2 hr | 22 | BQL(2) | 50 | 34.3 | 78 | 15.6 |
| | 23 | 19.3 | 51 | 37.5 | 79 | 56.6 |
| | 24 | 22.9 | 52 | 113 | 80 | 47.6 |
| | 25 | 28.6 | 53 | 37.6 | 81 | 21.7 |
| 4 hr | 26 | 13.1 | 54 | 33.2 | 82 | 24.1 |
| | 27 | 53.4 | 55 | 2.48 | 83 | 11.1 |
| | 28 | 39.5 | 56 | 86.4 | 84 | 92.3 |
| | 29 | 4.22 | 57 | 51.3 | 85 | 24.2 |
| 6/8 hr(3) | 30 | 21.4 | 58 | 36.7 | 86 | 15.1 |
| | 31 | 38.3 | 59 | 91.5 | 87 | 40.9 |
| | 32 | 18.9 | 60 | 52.7 | 88 | 70.3 |
| | 33 | 29.0 | 61 | 75.8 | 89 | 29.3 |
| 24 hr | 34 | 8.36 | 62 | 21.2 | 90 | 31.8 |
| | 35 | 16.8 | 63 | 13.4 | 91 | 14.2 |
| | 36 | 9.36 | 64 | 16.7 | 92 | 7.26 |
| | 37 | 15.0 | 65 | 14.9 | 93 | 6.71 |

| | | | | | | |
|---|---|---|---|---|---|---|
| (1) 4 min instead of 3 min (2) Below the quantification limit (3) 6 h for RSV NB2 and ALX-0081; 8h for RANKL008A | | | | | | |

**Table B-20: Individual actual amount (BALF Concentration x recovered volume) normalized by dose (%) of RSV NB2, ALX-0081, and RANKL008A in BALF after i.t. administration of RSV NB2 (3.6 mg/kg), ALX-0081 (3.1 mg/kg) and RANKL008A (3.2 mg/kg) to male Wistar rats.**

| | BALF Actual Amount expressed as % of the dose | | | | | |
|---|---|---|---|---|---|---|
| | RSV NB2 | | ALX-0081 | | RANKL008A | |
| Time | ID | Amount/D (%) | ID | Amount/D (%) | ID | Amount/D (%) |
| 3 min(1) | 10 | 22.3 | 38 | 110 | 66 | 25.1 |
| | 11 | 37.0 | 39 | 38.2 | 67 | 43.6 |
| | 12 | 17.1 | 40 | 59.7 | 68 | 10.5 |
| | 13 | 24.1 | 41 | 87.9 | 69 | 66.2 |
| 20 min | 14 | 23.0 | 42 | 28.7 | 70 | 10.5 |
| | 15 | 28.8 | 43 | 120 | 71 | 25.6 |
| | 16 | 40.0 | 44 | 75.8 | 72 | 25.2 |
| | 17 | 50.8 | 45 | 42.4 | 73 | 17.4 |
| 1 hr | 18 | 76.3 | 46 | 145 | 74 | 84.1 |
| | 19 | 28.9 | 47 | 61.4 | 75 | 41.0 |
| | 20 | 23.6 | 48 | 50.6 | 76 | 24.6 |
| | 21 | 12.1 | 49 | 25.4 | 77 | 38.7 |
| 2 hr | 22 | BQL(2) | 50 | 27.4 | 78 | 9.4 |
| | 23 | 11.6 | 51 | 30.0 | 79 | 42.5 |
| | 24 | 14.9 | 52 | 73.2 | 80 | 38.1 |
| | 25 | 20.0 | 53 | 28.2 | 81 | 17.3 |
| 4 hr | 26 | 7.19 | 54 | 26.6 | 82 | 16.9 |
| | 27 | 26.7 | 55 | 1.98 | 83 | 7.21 |
| | 28 | 37.5 | 56 | 77.8 | 84 | 64.6 |
| | 29 | 3.37 | 57 | 38.5 | 85 | 18.1 |
| 6/8 hr(3) | 30 | 15.0 | 58 | 29.4 | 86 | 10.6 |
| | 31 | 26.8 | 59 | 82.3 | 87 | 26.6 |
| | 32 | 13.2 | 60 | 31.6 | 88 | 52.7 |
| | 33 | 24.6 | 61 | 64.4 | 89 | 26.4 |
| 24 hr | 34 | 5.44 | 62 | 15.9 | 90 | 25.4 |
| | 35 | 10.9 | 63 | 10.0 | 91 | 10.6 |
| | 36 | 7.02 | 64 | 14.2 | 92 | 5.81 |
| | 37 | 10.5 | 65 | 9.66 | 93 | 3.69 |

| | | | | | | |
|---|---|---|---|---|---|---|
| (1) 4 min instead of 3 min (2) Below the quantification limit (3) 6 h for RSV NB2 and ALX-0081; 8h for RANKL008A | | | | | | |

**Table B-21: Mean (+SD:n=4) theoretical amount (BALF Concentration x 10 mL) normalized by dose (%) of RSV NB2, ALX-0081, and RANKL008A in BALF after i.t. administration of RSV NB2 (3.6 mg/kg), ALX-0081 (3.1 mg/kg) and RANKL008A (3.2 mg/kg) to male Wistar rats.**

| | BALF theoretical amount expressed as % of the dose | | | | | |
|---|---|---|---|---|---|---|
| | RSV NB2 (ID 10-37) | | ALX-0081 (ID 38-65) | | RANKL008A (ID 66-93) | |
| Time | Average | SD | Average | SD | Average | SD |
| 4 min | 37.5 | 15.5 | 98.3 | 41.0 | 47.5 | 23.7 |
| 20 min | 49.5 | 15.1 | 85.6 | 48.6 | 40.8 | 32.0 |
| 1 hr | 52.3 | 44.8 | 90.7 | 63.3 | 67.4 | 38.0 |
| 2 hr | 23.6 | 4.7 | 55.5 | 38.1 | 35.4 | 19.8 |
| 4 hr | 27.6 | 22.9 | 43.4 | 35.1 | 37.9 | 36.8 |
| 6 hr | 26.9 | 8.7 | 64.2 | 24.3 | /(2) | / |
| 8 hr | / | / | / | / | 38.9 | 23.4 |
| 24 hr | 12.4 | 4.1 | 16.5 | 3.4 | 15.0 | 11.7 |

| | | | | | | |
|---|---|---|---|---|---|---|
| (1) 4 min instead of 3 min (2) No sampling scheduled per protocol | | | | | | |

**Table B-22: Mean actual amount (BALF Concentration x recovered volume) normalized by dose (%) of RSV NB2, ALX-0081, and RANKL008A in BALF after i.t. administration of RSV NB2 (3.6 mg/kg), ALX-0081 (3.1 mg/kg) and RANKL008A (3.2 mg/kg) to male Wistar rats.**

| | BALF actual amount expressed as % of the dose | | | | | |
|---|---|---|---|---|---|---|
| | RSV NB2 (ID 10-37) | | ALX-0081 (ID 38-65) | | RANKL008A (ID 66-93) | |
| Time | Average | SD | Average | SD | Average | SD |
| 3 min(1) | 25.1 | 8.5 | 74.0 | 31.5 | 36.3 | 24.1 |
| 20 min | 35.7 | 12.3 | 66.8 | 40.7 | 19.7 | 7.2 |
| 1 hr | 35.2 | 28.2 | 70.7 | 51.9 | 47.1 | 25.7 |
| 2 hr | 15.5 | 4.2 | 39.7 | 22.3 | 26.8 | 16.0 |
| 4 hr | 18.7 | 16.2 | 36.2 | 31.6 | 26.7 | 25.7 |
| 6 hr | 19.9 | 6.8 | 51.9 | 25.8 | /(2) | / |
| 8 hr | / | / | / | / | 29.1 | 17.5 |
| 24 hr | 8.46 | 2.66 | 12.5 | 3.1 | 11.4 | 9.8 |

| | | | | | | |
|---|---|---|---|---|---|---|
| (1) 4 min instead of 3 min (2) No sampling scheduled per protocol | | | | | | |

By dividing the calculated amount of Nanobody by the actual amount dosed, the recovered fraction of the dose could be compared across time: The highest mean amount to dose percentages via actual and theoretical volume are 35.7% and 49.5% for RSV NB2 (After 20 minutes), 74.0% and 98.3% for ALX-0081 (After 4 minutes) and 47.1% and 67.4% for RANKL008A (After 1 hour), respectively. Thus for ALX-0081 almost the total fraction of the dose could be recovered in the BALF, while for RSV NB2 and RANKL008A, the fraction was lower: approximately 50% of the. The highest individual amount to dose percentages via actual and theoretical volume are 76.6% and 117.3% for RSV NB2, 145% and 182% for ALX-0081 and 84.1% and 120% for RANKL008A at time-point 1 hour post-dose. As expected, the variability was appreciable.

After 24 hours, the fraction of the dose recovered in BALF was lower for all Nanobodies than at earlier time-points. The mean fraction recovered ranged from 12.4% to 16.5% via the theoretical volume and ranged from 8.46% to 12.5% via the actual volumes for the three tested Nanobodies.

### 1.3 Conclusions

- After i.v. administration to rats, similar PK characteristics were observed for RSV NB2 and ALX-0081. For RANKL008A, substantially lower clearance values and longer terminal half-lives were observed. This may be explained by binding of the anti-HSA Nanobody of RANKL008A to rat albumin.
- The current data show that systemic exposure to Nanobodies can be achieved after intra-tracheal administration, indicating that the pulmonary route may be viable as non-invasive method for the delivery of Nanobodies. The data also indicate that the systemic bioavailability seems to decrease with increasing molecular weight.

- After i.t. administration comparable terminal plasma half-lives were observed for the three Nanobodies. For RSV NB2 and ALX-0081 the plasma half-lives are longer after i.t. administration than after i.v. administration, indicating that that absorption is the rate limiting (the drug is slowly absorbed from its site of dosing (i.e. the lung) to the systemic circulation). Comparable terminal half-lives were observed both in plasma and in BALF, supporting the notion that the kinetics may be absorption rate controlled.
- Following intra-tracheal administration, the RSV NB2, ALX-0081, RANKL008A Nanobody exposure in BALF was observed for at least 24 hours (i.e. the last sampling time for BALF).
- Following intra-tracheal administration, systemic exposure to the RSV NB2, ALX-0081 Nanobody in plasma was observed for at least 24 hours (i.e. the last sampling time of plasma after intra-tracheal administration. Following i.v. administration both of these Nanobodies without anti-HSA were no longer detectable at 24 hours in plasma.

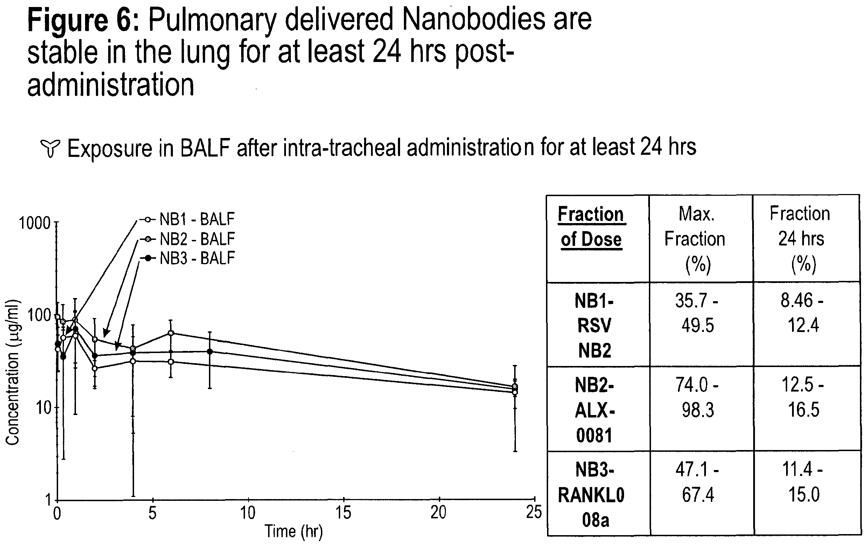

Figure 6 and Figure 7 further illustrate these experimental results.

### Example 2.1: Intranasal delivery of bivalent Nanobody RSV101 protects against infection and replication of Respiratory syncytial virus (RSV) strain A2 in mice

### Compounds:

| Name | Alternative names | SEQ ID NO: | Reference | Amino acid sequence |
|---|---|---|---|---|
| RSV101 | NB2-15GS-NB2 | 4 | a bivalent construct in which two units of NB2 (191D3) are linked by a 15GS linker. This Nanobody is binding to the F-protein of RSV and potently neutralizes RSV in vitro as assessed by the microneutralization assay - see example 4.3 (IC50 of 191D3 for the RSV Long strain is about 250 nM; IC50 of RSV101 for the RSV Long strain is about 0.1 nM). | EVQLVESGGGLVQAGGSLRLSC EASGRTYSRYGMGWFRQAPGK EREFVAAVSRLSGPRTVYADSVK GRFTISRDNAENTVYLQMNSLKP |
| | | | | EDTAVYTCAAELTNRNSGAYYYA WAYDYWGQGTQVTVSSGGGGS GGGGSGGGGSEVQLVESGGGL VQAGGSLRLSCEASGRTYSRYG MGWFRQAPGKEREFVAAVSRLS GPRTVYADSVKGRFTISRDNAEN TVYLQMNSLKPEDTAVYTCAAEL TNRNSGAYYYAWAYDYWGQGT QVTVSSAAAEQKLISEEDLNGAA HHHHHH |
| 12D2biv | | | Bivalent control nanobody construct | Not available |
| Palivizu mab | Synagis | | Medlmmune product; Synagis is indicated for the prevention of serious lower respiratory tract disease caused by RSV in children at high risk of RSV disease (US FDA approved). e.g. American Academy of Pediatrics. "Red Book: 2006 Report of the Committee on Infectious Diseases, 27th ed. "Pp562-565 | |

To test the capacity of Nanobody RSV101 to neutralize virus *in vivo,* a mouse model was used. In this model, female Balb/c mice (9-10 weeks old) were inoculated intranasally with 100 ug of purified RSV101 dissolved in 50 ul PBS. As an irrelevant Nanobody control the bivalent Nanobody 12D2biv was used. In addition, one group of mice received 100 ug Palivizumab (Synagis) and a fourth group received PBS only. Five hours later, 10⁶ infectious units of the RSV A2 strain were administered intra-nasally. Four days and 1 day before virus infection and 1 and 4 days after infection mice were treated with cyclophosphamide (first dosing at 3 mg/kg; subsequent dosing at 2 mg/kg all administered s.c.) to suppress the immune system and as such to increase virus replication.

Three and 5 days after viral challenge, mice were killed; lungs were removed, homogenized and cleared from tissue by centrifugation. Sub-confluent Hep-2 cells, incubated in serum-free medium, were infected with serial dilutions of cleared lung homogenates. Four hours after infection the medium was removed and replaced by fresh medium containing 1% FCS and 0.5% agarose. Two to three days after infection the agarose overlay was removed to allow staining of RSV-plaques by an anti-RSV antibody.

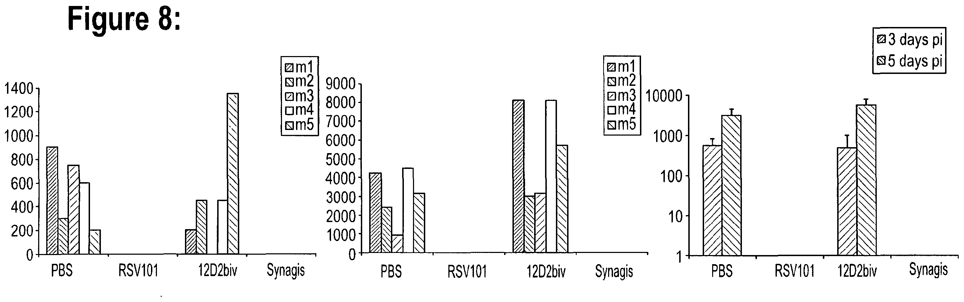

Infectious virus (pfu/lung) was recovered from all animals in the negative control groups (PBS and 12D2biv) in lung homogenates on day 3 (Figure 8, left panel) and 5 after challenge (Figure 8, middle panel). In Figure 8, the right panel the mean of infectious virus titers (pfu/lung) is represented. None of the animals in the RSV101 and Synagis-treated group had detectable infectious virus on day 3 and 5 post challenge.

### Example 2.2: After intranasal administration Nanobodv RSV101 remains functionally active in the lungs for at least 72 hours

In order to test whether nanobodies or palivizumab antibodies might still be present in lungs 3 and 5 days after inoculation, lung homogenates of PBS treated mice were pre-incubated for 1h with the same volume of lung homogenates from the different experimental groups, prepared either three of five days post-infection.

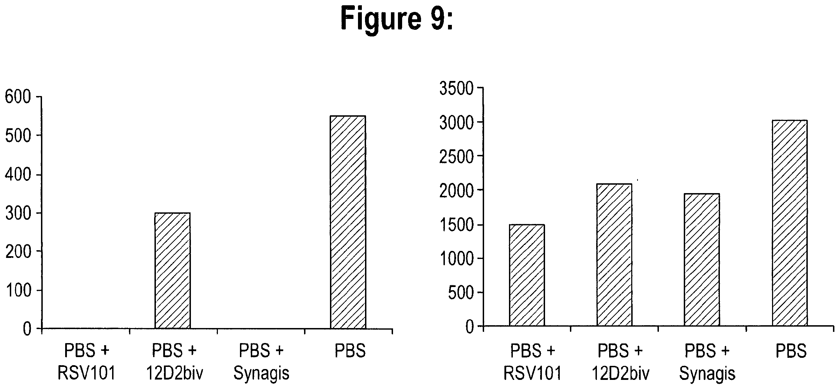

As shown in Figure 9 (left panel), incubation of lung homogenates from PBS treated mice with lung homogenates prepared three days after infection from either RSV101 or palivizumab but not 12D2biv treated mice neutralized the virus present in the lung homogenates from PBS treated mice. In contrast, none of the lung homogenates of mice treated with RSV101 or Synagis prepared five days after infection could severely neutralize the virus present in the lung homogenates of PBS treated mice (Figure 9 right panel).
Taken together, these data show that the functional bivalent Nanobody RSV101 remains present in the lungs for at least 72 hours after administration.

### Example 2.3: Viral RNA is not detected in the lungs of mice pre-treated intranasally with RSV101.

The results described in example 2.1 demonstrated that no infectious virus was present in the lungs of mice treated with RSV101. However, there was still the possibility that virus had infected cells and that viral genomic RNA was replicated with release of non-infectious viral particles or without release of viral particles. To investigate this possibility, the presence of viral RNA was determined by qPCR. RNA was isolated from 100 ul of each long homogenate (1000 ul prepared 5 days post-infection. By the use of an M-gene specific primer RSV genomic RNA specific cDNA was synthesized and quantified by qPCR (in duplicate). The level of viral genomic RNA in each lung homogenate was calculated relative to a lung sample which showed the lowest qRT-PCR signal (normalized to value of 1). As shown in Table B-23, the presence of relative viral genomic RNA in lungs of mice treated with RSV101 and Synagis® was reduced strongly compared to PBS or 12D2biv treated mice.

| Table B-23: Relative viral genomic RNA in lungs of treated mice 5 days post viral inoculation | | | | |
|---|---|---|---|---|
| Mouse | PBS | RSV101 | 12D2biv | Synagis |
| 1 | 170,69 | 16,96 | 214,74 | 4,82 |
| 2 | 53,45 | 10,96 | 466,40 | 4,81 |
| 3 | 471,42 | 3,84 | 350,39 | 7,20 |
| 4 | 404,66 | 5,60 | 418,76 | 6,32 |
| 5 | 342,39 | 2,19 | 193,26 | 4,15 |
| Mean | 288,52 | 7,91 | 328,71 | 5,46 |
| SD | 172,47 | 6,04 | 121,32 | 1,25 |

### Example 3: Pulmonary delivery studies with Nanobodies against HA pseudotyped viruses

The following description of the construction of HA pseudotyped viruses and assays performed taken from (1). A sensitive retroviral pseudotype assay for influenza H5N1-neutralizing antibodies. Influenza and Other Respiratory Viruses 1(3), 105-112)

### References:

(1). Temperton NJ, Hoschler K, Major Det al. A sensitive retroviral pseudotype assay for influenza H5N1-neutralizing antibodies. Influenza and Other Respiratory Viruses 2007 1(3), 105-112
(17) Besnier C, Takeuchi Y, Towers G. Restriction of lentivirus in monkeys. Proc Natl Acad Sci U S A 2002; 9:11920-11925.
(19) Op De Beeck A, Voisset C, Bartosch B et al. Characterization of functional hepatitis C virus envelope glycoproteins. J Virol 2004; 78:2994-3002.
(20) Naldini L, Blomer U, Gallay P et al. In vivo gene delivery and stable transduction of nondividing cells by a lentiviral vector. Science 1996; 272:263-267.

### Example 3.1: The HA-pseudotyped neutralization assay

| Name | Alternative names | SEQ ID NO: | Reference | Amino acid sequence |
|---|---|---|---|---|
| 202-A5 | | 6 | US provisional 61/139,130 | EVQLVESGGDLVQPGGSLRL SCAASGFTFRGYWMTWVRQ APGKGLEWVSSINNIGEEAYY VDSVKGRFTISRDNAKNTLYL QMNSLKSEDTAVYYCVKDW ASDYAGYSPNSQGTQVTVSS |
| 202-A10 | | 7 | US provisional 61/139,130 | EVQLVESGGGLVQAGDSLRL SCIDSGRTFSDYPIGWFRQA PGKEREFVAAIYAIGGDVYYA DSVKGRFTISRDNAKNTVYL QMSSLKPEDTAIYSCAVASG GGSIRSARRYDYWGRGTQV TVSS |
| 202-A12 | | 8 | US provisional 61/139,130 | EVQLVESGGGLVQAGGSLRL |
| | | | | SCAASGGTFSSYAMGWFRQ APGKERDFVSAITWSGGSTY YADSVKGRFTISRDNAKNTV YLQMNSLKPEDTAVYYCAAD DQKYDYIAYAEYEYDYWGQG TQVTVSS |
| 202-B7 | | 9 | US provisional 61/139,130 | EVQLVESGGGLVQPGGSLRL SCAASGFTFRGYWMSWVRQ APGKGLEWVSAINNVGDEVY YADSVKGRFTISRDNAKNTLY LQMNSLKSEDTAVYYCTRDW FDDPNKNEYKGQGTQVTVSS |
| 202-B10 | | 10 | US provisional 61/139,130 | EVQLVESGGGLVQPGGSLRL SCAASGFTFRGYWMSWVRQ APGKGLEWVSAINNVGDEVY YADSVKGRFTISRDNAKNTLY LQMNSLKSEDTAVYYCTRDW YNDPNKNEYKGQGTQVTVS S |
| 202-C1 | | 11 | US provisional 61/139,130 | KVQLVESGGDLVQPGGSLRL SCAASGFTFRGYWMTWVRQ APGKGLEWVSSINNIGEEAYY VDSVKGRFTISRDNAKNTLYL QMNSLKSEDTAVYYCVKDW ASDYAGYSPNSQGTQVTVSS |
| 202-C2 | | 12 | US provisional 61/139,130 | EVQLVESGGDLVQPGGSLRL SCAASGFTFRGYWMTWVRQ APGKGLEWVSSINNIGEEAYY VDSVKGRFTISRDNAKNTLYL QMNSLKSEDTAVYYCVKDW ASDYAGYSPNSQGTQVTVSS |
| 202-C8 | | 13 | US provisional 61/139,130 | EVQLVESGGGLVQPGGSLRL |
| | | | | SCTGSGFTFSSYWMDWVRQ TPGKDLEYVSGISPSGSNTD YADSVKGRFTISRDNAKNTLY LQMNSLKPEDTALYYCRRSL TLTDSPDLRSQGTQVTVSS |
| 202-C9 | | 14 | US provisional 61/139,130 | EVQLVESGGGLVQPGGSLRL SCAASGFTFRGYWMSWVRQ APGKGLEWVSAINNVGGETY YADSVKGRFTISRDNAKNALY LQMNSLKSEDTAVYYCARD WYNDPNKNEYKGQGTQVTV SS |
| 202-D5 | | 15 | US provisional 61/139,130 | EVQLVESGGGLVQAGGSLRL SCAASGSTGSSTAMGWSRQ APGKQREWVASISSAGTIRY VDSVKGRFTISRDNAKNTGY LQMNSLKPEDTAVYYCYVVG NFTTYWGRGTQVTVSS |
| 202-D8 | | 16 | US provisional 61/139,130 | EVQLVESGGGLVQPGGSLRL SCAASGFTFRGYWMSWVRQ APGKGLEWVSAINNVGDEVY YADSVKGRFTISRDNAKNTLY LQMNSLKSEDTAVYYCTRDW YNDPNKNEYKGQGTQVTVS S |
| 202-E4 | | 17 | US provisional 61/139,130 | EVQLVESGGGLVQAGGSLRL SCAASVSAFSEYAMGWYRQ APGKQREFVATINSLGGTSY ADSVKGRFTISRDNAKNTVYL QMNSLKPEDTAVYYCTLYRA NLWGQGTQVTVSS |
| 202-E5 | | 18 | US provisional 61/139,130 | EVQLVESGGDLVQPGGSLRL |
| | | | | SCAASGFTFRGYWMTWVRQ APGKGLEWVSSINNIGEETYY VDSVKGRFTISRDNAKNTLYL QMNSLKSEDTAVYYCVKDW ASDYAGYSPNSQGTQVTVSS |
| 202-E6 | | 19 | US provisional 61/139,130 | EVQLVESGGGLVQAGGSLRL SCAASGRTFSSYAMGWFRQ APGKEREFVAAISWSGRTTY YADFVKGRFTISRDNAKNTVY LQMNSLKPEDTAVYYCAADL SPGNEYGEMMEYEYDYWGE GTQVTVSS |
| 202-E7 | | 20 | US provisional 61/139,130 | EVQLVESGGGLVQPGGSLRL SCAASGFTFRGYWMSWVRQ APGKGLEWVSAINNVGGETY YADSVKGRFTISRDNAKNTLY LQMNSLKSEDTAAYYCARD WYNDPNKNEYKGQGTQVTV SS |
| 202-E11 | | 21 | US provisional 61/139,130 | EVQLVESGGGLVQPGGSLRL SCAASGFTFRGYWMSWVRQ APGKGLEWVSAINNVGDEVY YADSVKGRFTISRDNAKNTLY LQMNSLKSEDTAVYYCTRDW YNDPNKNEYKGQGTQVTVS S |
| 202-F3 | | 22 | US provisional 61/139,130 | EVQLVESGGDLVQPGGSLRL SCAASGFTFRGYWMTWVRQ APGKGLEWVSSINNIGEEAYY VDSVKGRFTISRDNAKNTLYL QMNSLKSEDTAVYYCVKDW ASDYAGYSPNSQGTQVTVSS |
| 202-F4 | | 23 | US provisional 61/139,130 | EVQLVESGGDLVQPGGSLRL SCAASGFTFRGYWMTWVRQ APGKGLEWVSSINNIGEEAYY VDSVKGRFTISRDNAKNTLYL QMNSLKSEDTAVYYCVKDW ASDYAGYSPNSQGTQVTVSS |
| 202-F8 | | 24 | US provisional 61/139,130 | EVQLVESGGGLVQPGGSLRL SCAASGLIFSSYDMGWFRQA PGEERAFVGAISRSGDVRYV DPVKGRFTITRDNAKNTVYLQ MNSLKPEDTAVYYCAADADG WWHRGQAYHWWGQGTQVT VSS |
| 202-G3 | | 25 | US provisional 61/139,130 | EVQLMESGGGLVQAGGSLR LSCAASGRTFSGYTMGWFR QAPGKGREWVAGISWSGDS TYYADSVKGRFTISREDAKNT VYLQMNSLKPGDTADYYCAA ECAMYGSSWPPPCMDWGQ GTQVTVSS |
| 202-G8 | | 26 | US provisional 61/139,130 | EVQLVESGGGSVQPGGSLRL SCAASGFTFRGYWMSWVRQ APGKGLEWVSAINNLGGDTY YADSVKGRFTISRDNAKNML YLQMNSLKAEDTAVYYCARD WYDDPNKNEYKGQGTQVTV SS |
| 202-G11 | | 27 | US provisional 61/139,130 | EVQLVESGGGLVQPGGSLRL SCAASGFTFRGYWMSWVRQ APGKGLEWVSAINNVGGETY YADSVKGRFTISRDNAKNTLY LQMNSLKSEDTAAYYCARD |
| | | | | WYNDPNKNEYKGQGTQVTV SS |
| 203-B1 | | 28 | | EVQLVESGGDLVQPGGSLRL SCAASGFTFRGYWMTWVRQ APGKGLEWVSSINNVGEETY YVDSVKGRFTISRDNAKNTLY LQMNSLKSEDTAVYYCVKDW ESSYAGYSPNSQGTQVTVSS |
| 203-H1 | | 29 | | EVQLVESGGGVVQAGGSLRL SCAASGLTFDIYSMGWFRQQ PGKEREFVASIGRSGNSTNY ASSVKDRFTISRDNAKKLVYL EMNSLTVEDAAVYVCAAKDG PLITHYSTTSMYWGQGTQVT VSS |
| 203-E12 | | 30 | | EVQLVESGGGLVQPGGSLRL SCAASGFTFRGYWMSWVRQ APGKGLEWVSAINNVGDEVY YADSVKGRFTISRDNAKNTLY LQMNSLKSEDTAVYYCTRDW YNDPNKNEYKGQGTQVTVS S |
| 203-H9 | | 31 | | EVQLVESGGGLVQPGGSLRL SCTGSGFTFSSYWMDWVRQ TPGKDLEYVSGISPSGGNTD YADSVKGRFTISRDNAKNTLY LQMNSLQPEDTALYYCRRSL TLTDSPDLRSQGTQVTVSS |
| 203-B12 | | 32 | | EVQLVESGGGLVQPGGSLRL SCAASGFTFSSYAMGWVRR APGEGLEWVSSISSGGALPT YADSVKGRFTISRDNVKNTLY |
| | | | | LQMNSLKPEDTAVYSCEKYA GSMWTSERDAWGQGTQVT VSS |
| 203-A9 | | 33 | | EVQLVESGGGLVQAGDSLRL SCIDSGRTFSDYPIGWFRQA PGKEREFVAAIYPTDDNPTG PNAYYADSVKGRFTISRDNA KNTVYLQMSSLKPEDTAIYSC AVASGGGSIISARRYDYWGQ GTQVTVSS |
| 203-D9 | | 34 | | EVQLVESGGGWVQAGDSLR LSCAASGRTLSSYAMAWFRQ APGKERDFVTGITWNGGSTY YADSVKGRFTISRDNAKNTV YLQMNSLKPEDTAVYYCAAB QNTYGYMDRSDYEYDYWGQ GTQVTVSS |
| 189-E2 | | 35 | | KVQLVESGGGLVQPGGSLRL SCAASGSIFSINAMGWYRQA PGKQRELVAHIASSGSTIYA DSVKGRFTISRDNAKNTVYL QMNSLKPEDTAVYYCNTRGP AAHEVRDYWGQGTQVTVSS |
| 191-D3 | | 41 | | EVQLVESGGGLVQAGGSLRL SCEASGRTYSRYGMGWFRQ APGKEREFVAAVSRLSGPRT VYADSVKGRFTISRDNAENT VYLQMNSLKPEDTAVYTCAA ELTNRNSGAYYYAWAYDYW GQGTQVTVSS |

### Plasmids and cell lines.

Plasmid pl.18/VN1194 HA was constructed at NIBSC (UK). The full-length HA ORF from A/Vietnam/1194/04 was amplified by PCR and cloned into the expression vector pl.18. This backbone plasmid is a pUC-based plasmid incorporating promoter and Intron A elements from human cytomegalovirus. The MLV and HIV gag/pol constructs has been described previously. The luciferase (Luc) reporter construct MLV-Luc has been described (19). Vesicular stomatitis virus envelope protein (VSV-G) expression vector pMDG has been described previously (20). All cell lines were cultured in Dulbecco's modified eagle medium (DMEM) with Glutamax and high glucose (Gibco, Paisley, Scotland, UK), supplemented with 10% fetal calf serum and penicillin/streptomycin, except for 293T cells (15% fetal calf serum).

### Viral vector production and infection of target cells

Confluent plates of 293T cells were split 1:4 the day before transfection. Each plate of 293T cells was transfected with 1 g gag/pol construct, 1.5 ug Luc reporter construct, and 1.5□ug HA- or VSV-G-expressing construct by using the Fugene-6 transfection reagent. At 24 h post-transfection, 1 U of exogenous neuraminidase (Sigma, St. Louis, MO, USA) was added to induce the release of HA-pseudotyped particles from the surface of the producer cells. Supernatant was harvested 48 and 72 h post-transfection, filtered through 0.45-lm filters, and stored at -80°C. MLV vector titers were measured on human 293T, quail QT6, canine MDCK, porcine PK15 and ST-IOWA cells and are presented as infectious units (IU) per milliliter. Briefly, cells were infected with vector, and Luc titers were determined 72 h later by Luc assay. Titers were expressed as RLU for Luc.

### MLV(HA) pseudotype neutralization assay

Serum samples (5 ul) were heat inactivated at 56°C for 30 min, twofold serially diluted in culture medium, and mixed with MLV(HA) virions (10 000 RLU for Luc) at a 1:1 v/v ratio. Purified Nanobodies (10 or 20 ul) were diluted to 100 ul and twofold serially diluted in culture medium, and mixed with MLV(HA) virions (10 000 RLU for Luc) at a 1:1 v/v ratio. After incubation at 37°C for 1 h, 1 x 10⁴ 293T cells were added to each well of a 96-well flat-bottomed plate. Relative light units (RLU) for Luc were evaluated 48 h later by luminometry using the Promega Bright-Glo system (Promega, Madison, WI, USA) according to the manufacturer's instructions. IC90/IC50-neutralizing antibody titers were determined as the highest serum dilution resulting in a 90/50% reduction of infection (as measured by marker gene transfer) compared with a pseudotype virus only control. For Luc, titers <100 are designated negative.

### Example 3.2: Llamas develop high virus-neutralizing antibody titers after immunizations with purified H5 HA.

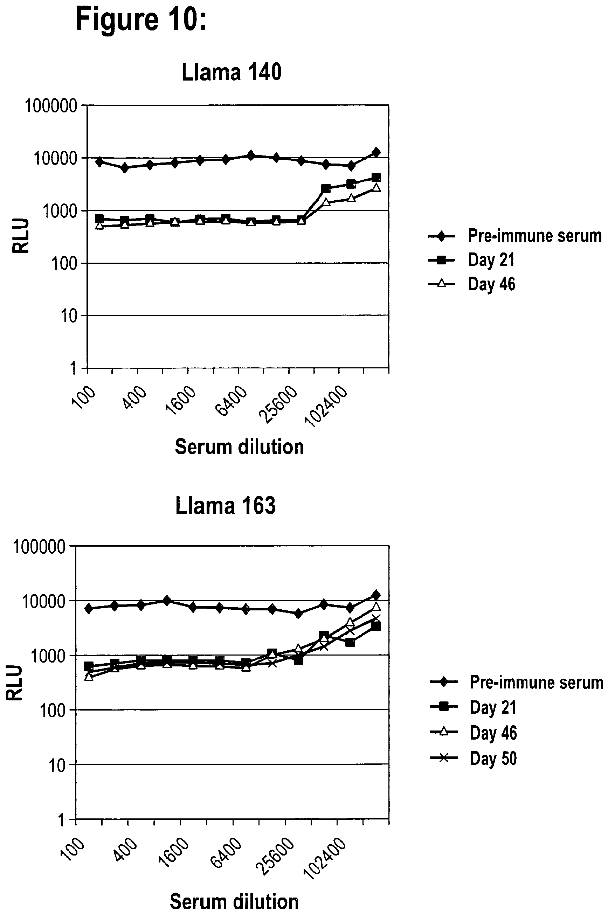

Sera taken from immunized llamas before (pre-immune) and 21 and 48 days after the first immunization was tested in the pseudotyped neutralization assay as described in example 3.1. (Figure 10). Pre-immune serum showed no neutralizing activity, while IC90s of 25600 to 51200 were present in llama 140 and 163, respectively.

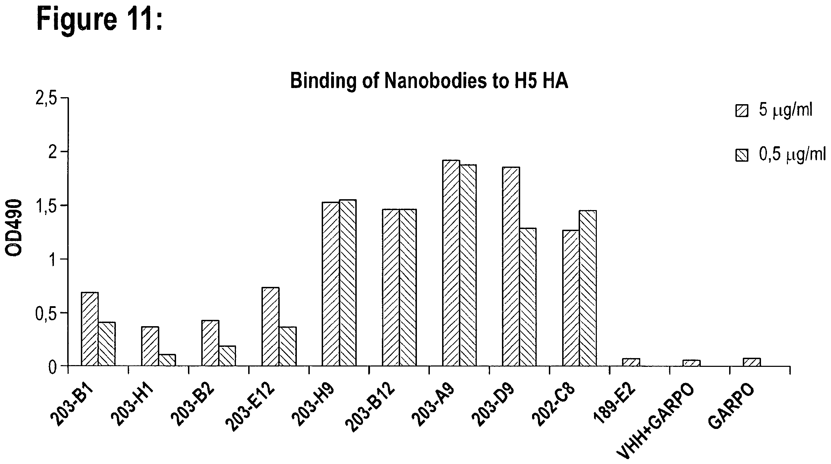

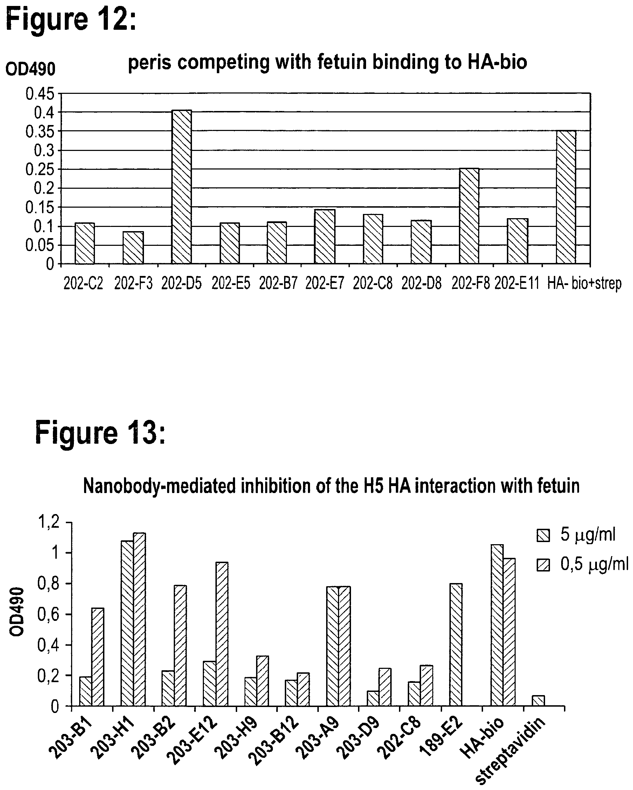

### Example 3.3: Identification of Nanobodies that block the interaction of HA with sialic acid on fetuin (Figures 11 and 12)

Hemagglutinin (HA) on Influenza viruses binds sialic acid on cells during infection. The sialic acid binding site the HA forms a pocket which is conserved between Influenza strains. Most HAs of avian influenza viruses preferentially recognize sialic acid receptors containing the α(2,3) linkage to galactose on carbohydrate side chains (human viruses, the α(2,6) linkage). To increase the chance of isolating neutralizing Nanobodies, a functional selection approach can be used - identify Nanobodies that compete with soluble 2,3 sialic acid (or 2,6 sialic acid for some mutational drift variants). This would select for Nanobodies targeting the sialic acid binding site of HA. These Nanobodies are likely to be the most potent at neutralizing H5N1.

We have selected Nanobodies binding to H5N1 HA and to identify the Nanobodies binding to the sialic acid binding site the following experiments were performed. Fetuin (from fetal calf serum, F2379, Sigma-Aldrich) was coated (10µg/ml) in a 96 well plate and incubated over night at 4°C. The plate was blocked in 2% BSA and then 0.7 µg/ml biotinylated HA (HA-bio) and 10 µl of periplasmic fractions or purified Nanobodies were added for competition. After incubation for 1 hour, HRP conjugated streptavidin was added and incubated for 1 hour. Binding specificity of HA-bio not recognized by periplasmic fractions or purified nanobodies was determined based on OD values compared to controls having received no Nanobody. Results of competition between periplasmic fractions or purified Nanobodies and fetuin for binding to HA-bio is shown in Figures 11, 12 and 13. Several Nanobody clones showed competition which may indicate that the competing Nanobodies recognize the sialic acid binding site on the HA.

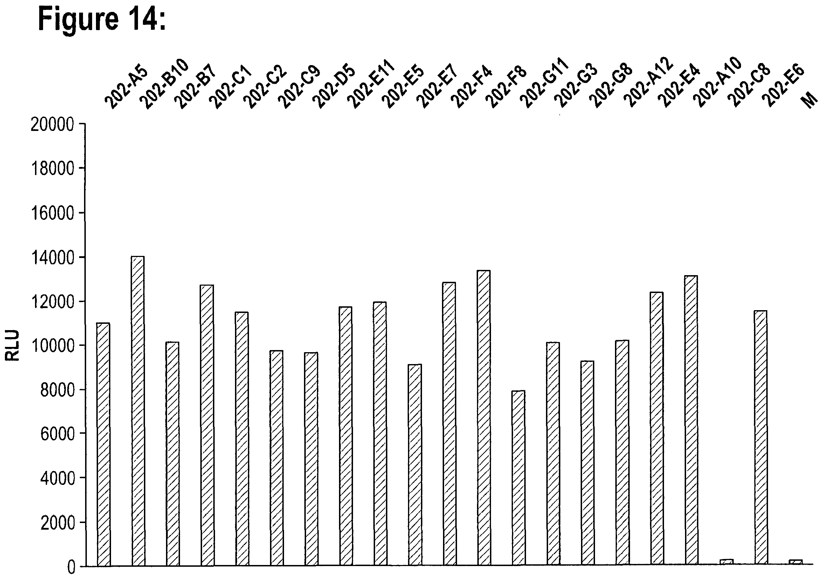

### Example 3.4: Identification of Nanobodies 202-C8, 203-B12 and 203-H9 that neutralize HA pseudotyped virus (Figures 13 and 14)

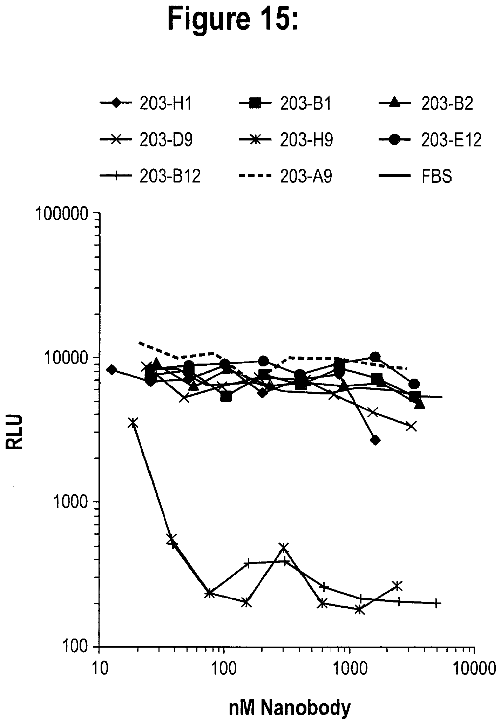

Several purified Nanobodies were tested in the pseudo typed virus neutralization assay described in Example 3.1. In Figure 14, the neutralization of a single 10 fold dilution of different Nanobodies is shown and only Nanobody 202-C8 strongly reduced luciferase activity, indicative for a virus neutralizing activity of this Nanobody. The identification of two more virus-neutralizing Nanobodies 203-B12 and 203-H9 is depicted in Figure 15.

### Example 3.5: Combinations of Nanobodies 202-C8, 203-B12 and 203-H9 do not result in increased neutralization.

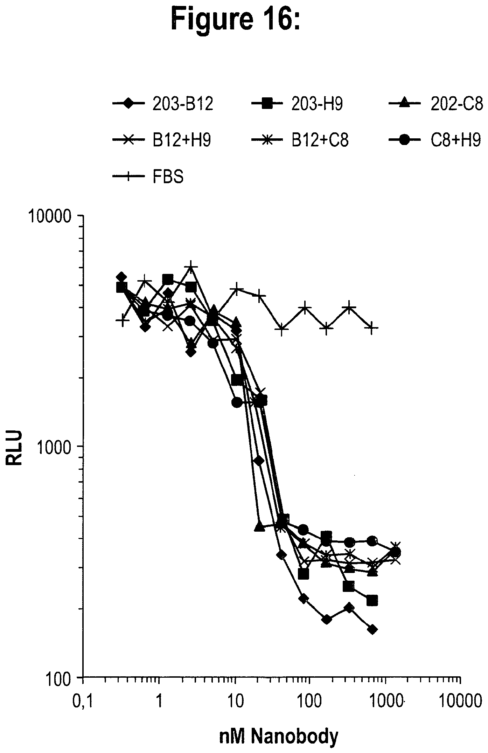

Combined treatment with different virus neutralizing antibodies might results in additive or even synergistic neutralizing effect. However, this was not observed when combinations of 202-C8 with 203-B12 or 202-C8 with 203-H9 or 203-B12 with 203-H9 were tested in the pseudotyped neutralization assay (Figure 16).

### Example 3.6: In vivo neutralization of influenza virus by Nanobody 202-C8

To test the capacity of Nanobody 202-C8 to neutralize virus *in vivo,* a mouse model was used. In this model, female Balb/c mice (6-7 weeks old) were inoculated intranasally with 100 ug of purified 202-C8 dissolved in 50 ul PBS. As an irrelevant Nanobody control the RSV Nanobody 191-D3 was used. In addition, one group of mice received PBS only. Four hours later, 1 LD50 of the mouse adapted NIBRG-14 was administered intranasally. The NIBRG-14 virus contains the HA (with the polybasic cleavage site removed) and the NA of the A/Vietnam/1194*l*/2004 (H5N1) virus. The internal viral genes are of the A/Puerto Rico/8/1934(H1N1).

Four and six days after viral challenge, mice were killed, lungs were removed and homogenized. Viral titers (TCID50) were determined by infection of MDCK cells with serial dilutions of lung homogenates. The presence of virus in cell supernatant was determined by hemagglutination assays. Titers were calculated according the method of Muench and Reed. A value of "0" was entered if no virus was detected. The geometric mean and standard deviation are reported for each group at each time point.

Mice treated with 202-C8 never showed any sign of disease during the whole experiment. The PBS and 191D3-treated mice showed clinical signs, including ruffled fur, inactivity, hunched posture, and depression.

Virus was recovered from all animals in the negative control groups (PBS and 191-D3) in lung homogenates on day 4 and 6 after challenge. None of the animals in the 202-C8-treated group had virus detectable virus titers on day 4 and 6 post challenge (Table B-24).

**Table B-24: Viral titers in mouse 4 and 6 days post inoculation**

| Day 4 lung titers (TCID50/ml lung homogenate) | | | | | |
|---|---|---|---|---|---|
| Group | Mouse 1 | Mouse 2 | Mouse 3 | Geo. Mean | StDev |
| PBS (n=3) | 355656 | 63246 | 63246 | 160716 | 137843 |
| 191D3 (n=3) | 112468 | 112468 | 632456 | 285797 | 245124 |
| 202-C8 (n=3) | 0 | 0 | 0 | 0 | 0 |

| Day 6 lung titers (TCID50/ml lung homogenate) | | | | | |
|---|---|---|---|---|---|
| Group | Mouse 1 | Mouse 2 | Mouse 3 | Geo. Mean | StDev |
| PBS (n=3) | 63426 | 112468 | 112468 | 96121 | 23119 |
| 191-D3 (n=3) | 63246 | 112468 | 112468 | 96061 | 23203 |
| 202-C8 (n=3) | 0 | 0 | 0 | 0 | 0 |

### Example 3.7: After intranasal administration Nanobody 202-C8 remains functionally active in the lungs for at least 48 hours

To test how long Nanobody 202-C8 remains active in the lungs after intranasal inoculation, female Balb/c mice (6-7 weeks old) were inoculated intranasally with 100 ug of purified 202-C8 dissolved in 50 ul PBS. As an irrelevant Nanobody control the RSV Nanobody 191-D3 was used. In addition, one group of mice received PBS only. All mice received 1 LD50 of the mouse adapted NIBRG-14 intranasally, but virus was given 4, 24 or 48 hours after inoculation of the Nanobodies. Four days after viral challenge, mice were killed, lungs were removed and homogenized. Viral titers (TCID50) were determined by infection of MDCK cells with serial dilutions of lung homogenates. The presence of virus in cell supernatant was determined by hemagglutination assays. Titers were calculated according the method of Muench and Reed. A value of "0" was entered if no virus was detected. The geometric mean and standard deviation are reported for each group at each time point (Table B-25).

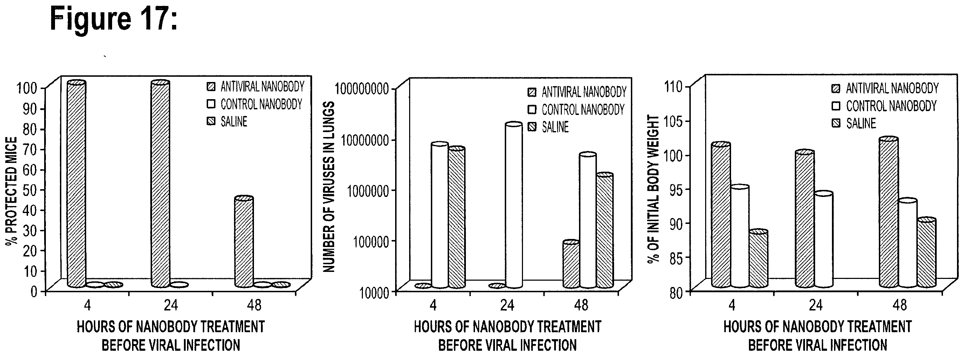

Mice pretreated with 202-C8 never showed any signs of disease during the whole experiment. The PBS and 191D3-treated mice showed clinical signs, including ruffled fur, inactivity, hunched posture, and depression and a reduction in body weight (Figure 17, right panel).

Virus was recovered from all animals pretreated with the control Nanobody 191-D3 or PBS. Virus could not be detected in the lungs of mice that were treated with 202-C8, 4 and 24 hours before virus inoculation. No virus could be detected in lungs of three mice of seven treated with 202-C8 48 hours before virus inoculation (Figure 17, left panel and Table B-25). Viral titers in the remaining 4 mice were on average reduced 50 fold compared to the viral titers found in the lungs of mice treated with 191-D3 48 hours before vial inoculation.

Taken together, these data show that the monovalent Nanobody 202-C8 remains actively present in the lungs for at least 48 hours after administration.

**Table B-25: Note LBG4=202-C8; LBG3= 191-D3**

| | Weight Day 0 | Weight Day 1 | Weight Day 2 | Weight Day 3 | Weight Day 4 | Lung titer Day 4 |
|---|---|---|---|---|---|---|
| LBG4 4h mouse 1 | 18,15 | 18,32 | 17,67 | 18,5 | 18,23 | 0 |
| LBG4 4h mouse 2 | 20,67 | 20,42 | 20,43 | 20,94 | 20,93 | 0 |
| LBG4 4h mouse 3 | 19,72 | 19,67 | 18,97 | 19,68 | 19,77 | 0 |
| average | 19,51 | 19,47 | 19,02 | 19,71 | 19,64 | 0 |
| St. Dev. | 1.27 | 1.06 | 1.38 | 1.22 | 1.35 | 0 |
| LBG4 24h mouse 1 | 18,76 | 18,81 | 18,52 | 18,83 | 18,85 | 0 |
| LBG4 24h mouse 2 | 19,48 | 19,62 | 18,99 | 18,96 | 19,13 | 0 |
| LBG4 24h mouse 3 | 18,73 | 18,55 | 18,18 | 18,34 | 18,32 | 0 |
| LBG4 24h mouse 4 | 19,19 | 19,27 | 18,9 | 19,48 | 19,32 | 0 |
| LBG4 24h mouse 5 | 18,95 | 19,24 | 18,36 | 18,96 | 19,06 | 0 |
| LBG4 24h mouse 6 | 18,99 | 18,81 | 18,21 | 18,66 | 18,91 | 0 |
| average | 19,02 | 19,05 | 18,53 | 18,87 | 18,93 | 0 |
| St. Dev. | 0.28 | 0.39 | 0.35 | 0.38 | 0.34 | 0 |
| LBG4 48h mouse 1 | 17,88 | 17,5 | 17,44 | 17,43 | 17,81 | 9355 |
| LBG4 48h mouse 2 | 17,29 | 17,01 | 16,94 | 17,11 | 17,37 | 355656 |
| LBG4 48h mouse 3 | 19,42 | 19,08 | 19,2 | 19,33 | 19,44 | 93550 |
| LBG4 48h mouse 4 | 19,47 | 19,53 | 18,89 | 19.31 | 19,51 | 0 |
| LBG4 48h mouse 5 | 19,73 | 19,55 | 19,34 | 19,54 | 20,02 | 0 |
| LBG4 48h mouse 6 | 18,92 | 18,84 | 18,72 | 18,47 | 18,91 | 63250 |
| LBG4 48h mouse 7 | 17,94 | 17,65 | 17,82 | 17,74 | 19,49 | 0 |
| average | 18,66 | 18,45 | 18,34 | 18,42 | 18,94 | 74544 |
| St. Dev. | 0.95 | 1.04 | 0.93 | 1.00 | 0.98 | 129378 |
| PBS 4h mouse 1 | 18,97 | 18,89 | 18,69 | 18,05 | 16,95 | 3556500 |
| PBS 4h mouse 2 | 18,15 | 18,36 | 18,13 | 17,32 | 15,95 | 6325000 |
| PBS 4h mouse 3 | 19,54 | 19,9 | 19,68 | 18,11 | 16,87 | 6325000 |
| average | 18,89 | 19,05 | 18,83 | 17,83 | 16,59 | 5402167 |
| St. Dev. | 0.70 | 0.78 | 0.78 | 0.44 | 0.56 | 1598394 |
| PBS 48h mouse 1 | 20,01 | 19,73 | 19,59 | 18,76 | 17,66 | 3556500 |
| PBS 48h mouse 2 | 21,43 | 21,68 | 20,9 | 20,06 | 19,39 | 632500 |
| PBS 48h mouse 3 | 18,78 | 19,02 | 18,74 | 17,67 | 16,8 | 632500 |
| average | 20,07 | 20,14 | 19,74 | 18,83 | 17,95 | 1607167 |
| St. Dev. | 1.33 | 1.38 | 1.09 | 1.20 | 1.32 | 1688172 |
| LBG3 4h mouse 1 | 20,3 | 20,42 | 20,11 | 19,72 | 19,28 | 6324600 |
| LBG3 4h mouse 2 | 18,39 | 18,54 | 18,66 | 18,38 | 18,33 | 9355000 |
| LBG3 4h mouse 3 | 18,39 | 18,82 | 18,44 | 17,77 | 16,3 | 3556500 |
| average | 19,03 | 19,26 | 19,07 | 18,62 | 17,97 | 6412033 |
| St. Dev. | 1.10 | 1.01 | 0.91 | 1.00 | 1.52 | 2900239 |
| LBG3 24h mouse 1 | 18,94 | 18,63 | 18,62 | 18,21 | 18,29 | 6324600 |
| LBG3 24h mouse 2 | 19,46 | 19,62 | 19,4 | 18,48 | 18,09 | 63250000 |
| LBG3 24h mouse 3 | 19,63 | 19,58 | 19,83 | 19,18 | 18,51 | 2000000 |
| LBG3 24h mouse 4 | 19,03 | 18,94 | 19,07 | 18,45 | 17,49 | 6325000 |
| LBG3 24h mouse 5 | 18,91 | 18,72 | 19 | 17,84 | 17,32 | 935500 |
| average | 19,19 | 19,10 | 19,18 | 18,43 | 17,94 | 15767020 |
| St. Dev. | 0.33 | 0.47 | 0.46 | 0.49 | 0.51 | 26657313 |
| LBG3 48h mouse 1 | 19,5 | 19,39 | 18,93 | 19,04 | 18 | 3556500 |
| LBG3 48h mouse 2 | 19,53 | 19,3 | 19,2 | 18,76 | 17,94 | 3556500 |
| LBG3 48h mouse 3 | 20,02 | 20,23 | 20,46 | 19,81 | 19,26 | 9355000 |
| LBG3 48h mouse 4 | 18,21 | 18,09 | 18,12 | 17,75 | 17,29 | 935500 |
| LBG3 48h mouse 5 | 18,38 | 18,17 | 18,32 | 17,92 | 16,53 | 6325000 |
| LBG3 48h mouse 6 | 21,19 | 20,83 | 20,55 | 20,34 | 18,98 | 632460 |
| average | 19,47 | 19,34 | 19,26 | 18,94 | 18,00 | 4060160 |
| St. Dev. | 1.10 | 1.09 | 1.04 | 1.02 | 1.02 | 3322192 |

### Example 4: Further studies with an anti-RSV nanobody construct

### Example 4.1:- prophylactic study with RSV407 in cotton rat

| SEQ ID NO: | Reference | Name | Amino Acid Sequence |
|---|---|---|---|
| 36 | SEQ ID NO: 2415 in PCT/EP2 009/056975 | RSV407 | EVQLVESGGGLVQAGGSLSISCAASGGSLSNYV LGWFRQAPGKEREFVAAINWRGDITIGPPNVEG RFTISRDNAKNTGYLQMNSLAPDDTAVYYCGAG TPLNPGAYIYDWSYDYWGRGTQVTVSSGGGGS GGGGSGGGGSEVQLVESGGGLVQAGGSLSISC AASGGSLSNYVLGWFRQAPGKEREFVAAINWR GDITIGPPNVEGRFTISRDNAKNTGYLQMNSLAP DDTAVYYCGAGTPLNPGAYIYDWSYDYWGRGT QVTVSSGGGGSGGGGSGGGGSEVQLVESGGG LVQAGGSLSISCAASGGSLSNYVLGWFRQAPG KEREFVAAINWRGDITIGPPNVEGRFTISRDNAK NTGYLQMNSLAPDDTAVYYCGAGTPLNPGAYIY DWSYDYWGRGTQVTVSSAAAEQKLISEEDLNG AAHHHHHH |

In this study cotton rats are treated either i.m. or intra-nasally with RSV neutralizing Nanobody constructs (RSV 407) or control (PBS). Viral RSV challenge is administered intranasally 1 hour later. At day 4, animals are sacrificed and RSV titers determined by Q-PCR in nasal and lung washes as well as in nasal and lung tissue.

RSV407 is a trivalent Nanobody construct consisting of 3 identical building blocks linked by 15GS spacers. The building block is binding the F protein of RSV and can neutralize RSV infection of the Long strain with an IC50 of about 50-100 nM. By formatting into a trivalent construct neutralization potency increased to an IC50 of about 100 pM on the RSV Long strain.

### Example 4.2 - therapeutic study with RSV407 in cotton rat

RSV therapeutic studies have been described in the past; e.g. by Crowe and colleagues (PNAS 1994;91:1386-1390) and Prince and colleagues (Journal of Virology 1987;61:1851-1854).

In this study cotton rats are intra-nasally infected with RSV. Twenty-four hours after infection a first group of animals are treated with RSV neutralizing Nanobody constructs (RSV 407) or control (PBS). Treatment is administered to pulmonary tissue by intranasal or aerosol administration. Treatment is repeated at 48 and 72 hours. At day 4 animals are sacrificed and RSV titers determined by Q-PCR in nasal and lung washed as well as in nasal and lung tissue.
In the second group treatment is only initiated 3 days after infection and repeated at day 4 and 5. Finally at day 6 animals are sacrificed and RSV titers determined by Q-PCR in nasal and lung washed as well as in nasal and lung tissue.

### Example 4.3 - Lung to systemic with Nanobody construct against RSV

In this study the lung tissue of rats is exposed to an RSV neutralizing Nanobody (RSV407) by intratracheal or aerosol administration. Serum and BAL samples are taken at regular time points up to 3 days after administration. The Nanobody concentration is measured by means of ELISA and samples are subjected to RSV microneutralization (see below). By combining the information from the ELISA and the neutralization assay the RSV IC50 of each sample can be determined to assess systemic bioavailabilty of functional RSV Nanobody.

Microneutralization: The hRSV micro neutralization assay is used to investigate *in vitro* neutralization capacity of selected purified hRSV Nanobodies. In here, Hep2 cells are seeded at a concentration of 1.5 x 10⁴ cells/well into 96-well plates in DMEM medium containing 10% fetal calf serum (FCS) supplemented with Penicillin and Streptomycin (100 U/ml and 100 µg/ml, respectively) and incubated for 24 hours at 37°C in a 5% CO₂ atmosphere. A standard quantity of hRSV strain Long LM-2 (Accession No. P12568; ATCC VR-26) is pre-incubated with serial dilutions of samples in a total volume of 50 µl for 30 minutes at 37°C. The medium of the Hep2 cells is replaced with the premix to allow infection for 2 hours, after which 0.1 ml of assay medium is added. The assay is performed in DMEM medium supplemented with 2.5 % fetal calf serum and Penicillin and Streptomycin (100U/ml and 100 µg/ml, respectively). Cells are incubated for an additional 72 hours at 37°C in a 5% CO2 atmosphere, after which cells are fixed with 80 % cold acetone (Sigma-Aldrich, St. Louis, MO) in PBS (100 µl/well) for 20 minutes at 4°C and left to dry completely. Next the presence of the F-protein on the cell surface is detected in an ELISA type assay. Thereto, fixed Hep2 cells are blocked with 2% Bovine Serum Albumin (BSA) solution in PBS for 1 hour at room temperature, than incubated for 1 hour with anti-F-protein polyclonal rabbit serum (Corral et al. 2007, BMC Biotech 7: 17) or Synagis® (2 µg/ml). For detection goat Anti-rabbit-HRP conjugated antibodies or goat Anti-Human IgG, Fcγ fragment specific-HRP (Jackson ImmunoResearch, West Grove, PA) is used, after which the ELISA is developed according to standard procedures.

### Example 5- Lung to systemic with Nanobody against RANKL (see WO 2008/142164 for RANKL nanobodies and constructs thereof)

In this study the lung tissue of first group of cynomolgus monkey is exposed to a half-life extended Nanobody construct against RANKL (RANKL 008a or RANKL180 or RANKL010a) by intratracheal or aerosol administration. Urine, serum and BAL samples are taken at regular time points up to 3 month after administration. The Nanobody concentration is measured by means of ELISA to determine the systemic pharmacokinetics of this half-life extended Nanobody. The pharmacodynamic effect of the RANKL Nanobody is assessed by measuring the decline of N-telopeptide (NTx), a biomarker for bone turnover, in serum and urine. A second group of animals is treated and analyzed in exactly the same way, however in this case the HSA binding Nanobody building block is omitted (RANKL13hum5-9GS-RANKL13hum5 or RANKL18-30GS-RANKL18 or RANKL18hum6-30GS-RANKL18hum6) and thus the Nanobody is half-life extended.

### Example 6 - Therapeutic efficacy of intranasal-delivered Nanobody

| Construct name | SEQ ID NO | Ref. SEQ ID NO | Amino Acid Sequence |
|---|---|---|---|
| 202-C8-9GS-202-C8 or (202-c8)2 | 37 | 2423 in PCT/EP20 09/056975 | EVQLVESGGGLVQPGGSLRLSCTGSGFTFSSYW MDWVRQTPGKDLEYVSGISPSGSNTDYADSVKG RFTISRDNAKNTLYLQMNSLKPEDTALYYCRRSLT LTDSPDLRSQGTQVTVSSGGGSGGGGSEVQLVE |
| | | | SGGGLVQPGGSLRLSCTGSGFTFSSYWMDWVR QTPGKDLEYVSGISPSGSNTDYADSVKGRFTISRD NAKNTLYLQMNSLKPEDTALYYCRRSLTLTDSPDL RSQGTQVTVSS |
| 191D3-15GS-191D3 or (191-d3)2 | 38 | 2382 in PCT/EP20 09/056975 | EVQLVESGGGLVQAGGSLRLSCEASGRTYSRYG MGWFRQAPGKEREFVAAVSRLSGPRTVYADSVK GRFTISRDNAENTVYLQMNSLKPEDTAVYTCAAEL TNRNSGAYYYAWAYDYWGQGTQVTVSSGGGGS GGGGSGGGGSEVQLVESGGGLVQAGGSLRLSC EASGRTYSRYGMGWFRQAPGKEREFVAAVSRLS GPRTVYADSVKGRFTISRDNAENTVYLQMNSLKP EDTAVYTCAAELTNRNSGAYYYAWAYDYWGQGT QVTVSSAAAEQKLISEEDLNGAAHHHHHH |

Twelve groups of mice ranging in size from 4 to 6 animals were challenged with 1 LD50 of the NIBRG-14 virus (see Table B-26). The NIBRG-14 virus (Temperton NJ, Hoschler K, Major D et al. A sensitive retroviral pseudotype assay for influenza H5N1-neutralizing antibodies. Influenza and Other Respiratory Viruses 2007 1: 105-112) contains the HA (with the polybasic cleavage site removed) and the NA of the A/Vietnm/1194l/2004 (H5N1) virus. The internal viral genes are of the A/ Puerto Rico/8/1934(H1N1). 4, 24, 48 and 72 after the viral inoculation, mice were inoculated intranasally with 60 µg (202-c8)2 or 60 µg of the irrelevant control Nanobody (191-D3)2. Mice were monitored daily for weight loss (Table B-29) and at day 4 (96 hours) post viral infection, mice were scarified to prepare lung homogenates. Infectious viral titers (Table B-27) and viral RNA (Table B-28) in lung homogenates were determined.

In lungs of 4 mice that received Nanobody (202-c8)2, 4 hours after viral inoculation, no infectious virus could be detected in lung homogenates obtained at 96 hours post infection. A comparison of viral RNA in these lungs with those in the lungs of mice that were treated with (191-d3)2 demonstrated a 98.58% reduction in viral RNA.

In 3 out of 5 mice that received Nanobody (202-c8)2 24 hours after infection, no infectious virus could be detected while titers were very low in the two remaining animals. A comparison of viral RNA in these lungs with those in the lungs of mice that were treated with (191-d3)2 demonstrated a 97.22% reduction in viral RNA. In lungs of mice that received Nanobody (202-c8)2, 48 hours after viral infection, a 84.26% reduction in viral titers was observed when compared to infectious viral titers in mice treated with (191-D3)2 at 48 hours post infections. A comparison of viral RNA in these lungs with those in the lungs of mice that were treated with (191-d3)2 demonstrated a 88.08% reduction in viral RNA.

Even when the Nanobody (202-c8)2 was administered 72 hours after viral challenge, very little infectious virus was detected in lung homogenates (i.e. a 84% reduction compared to (191-d3)2 treated mice). A comparison of viral RNA in these lungs with those in the lungs of mice that were treated with (191-d3)2 demonstrated a 38.21% reduction in viral RNA.

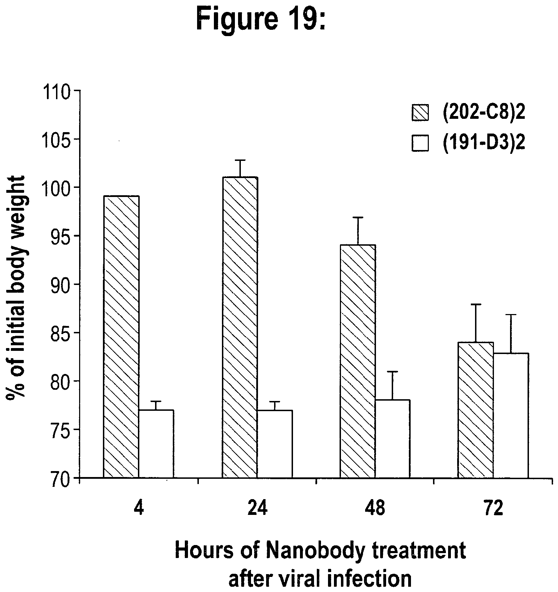

Administration of (202-c8)2 not only inhibited viral replication, it also prevented virus-induced morbidity. As shown in Table B-29 and Figure 19, administration of (202-c8)2 at 4, 24 and 48 hours after the viral challenge protected against viral-induced weight loss. When the Nanobody was administered 72 after infection, this viral-induced reduction in body weight was no longer prevented.

Overall these data demonstrated that a single therapeutic administration of a virus neutralizing Nanobody even up to 72 hours after viral infection inhibited substantially viral replication. In addition, prevention of virus-induced morbidity was prevented by administration of the Nanobody up to 48 hours after viral infection. This demonstrates that pulmonary delivery of Nanobodies is a powerful method to treat viral pulmonary infections.

**Table B-26. Groups and number of mice in each group**

| **h after viral inoculation** | | | | |
|---|---|---|---|---|
| | **4** | **24** | **48** | **72** |
| **(202-C8)2** | 4 | 5 | 6 | 6 |
| **(191-D3)2** | 4 | 5 | 6 | 6 |

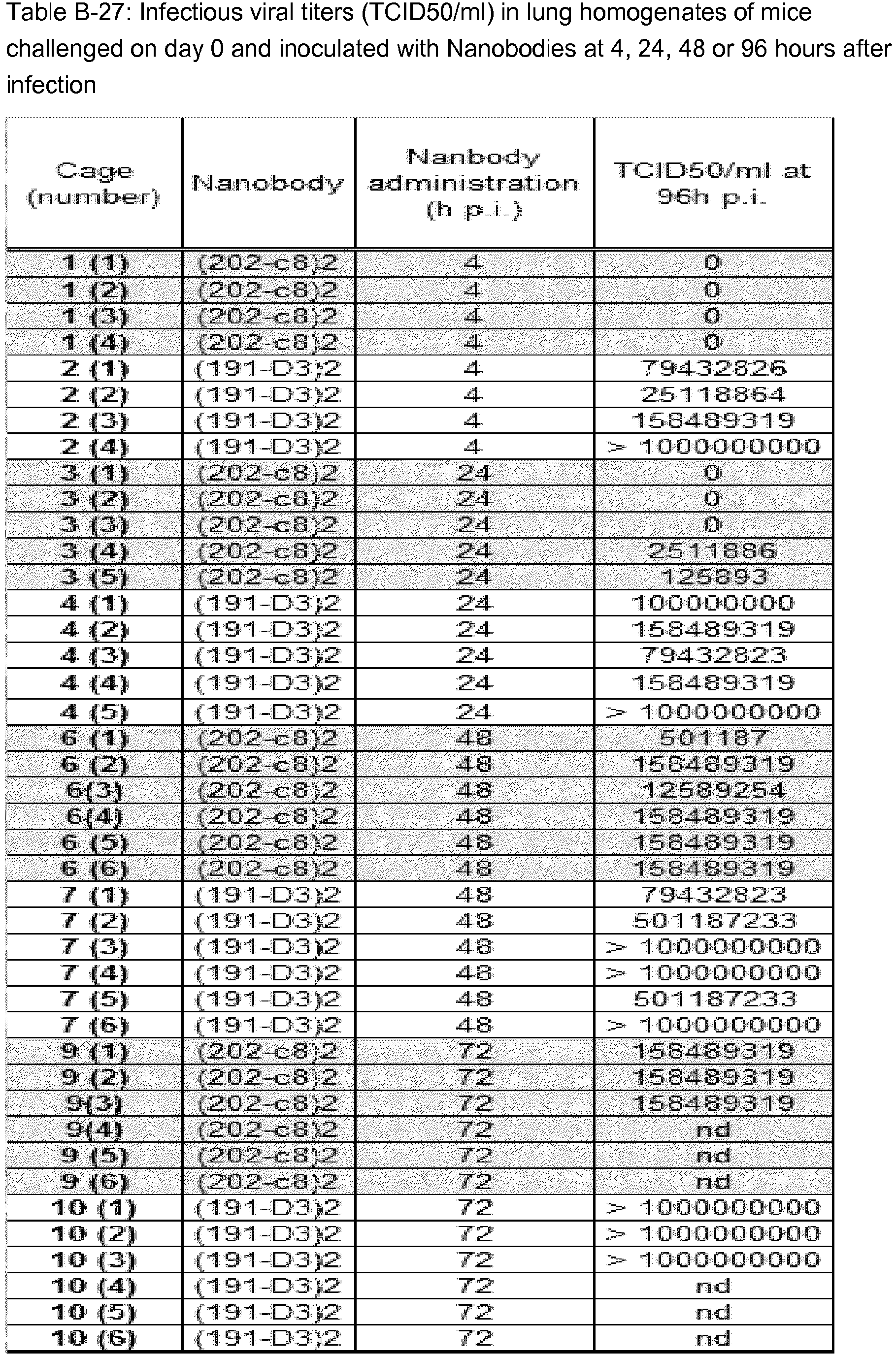

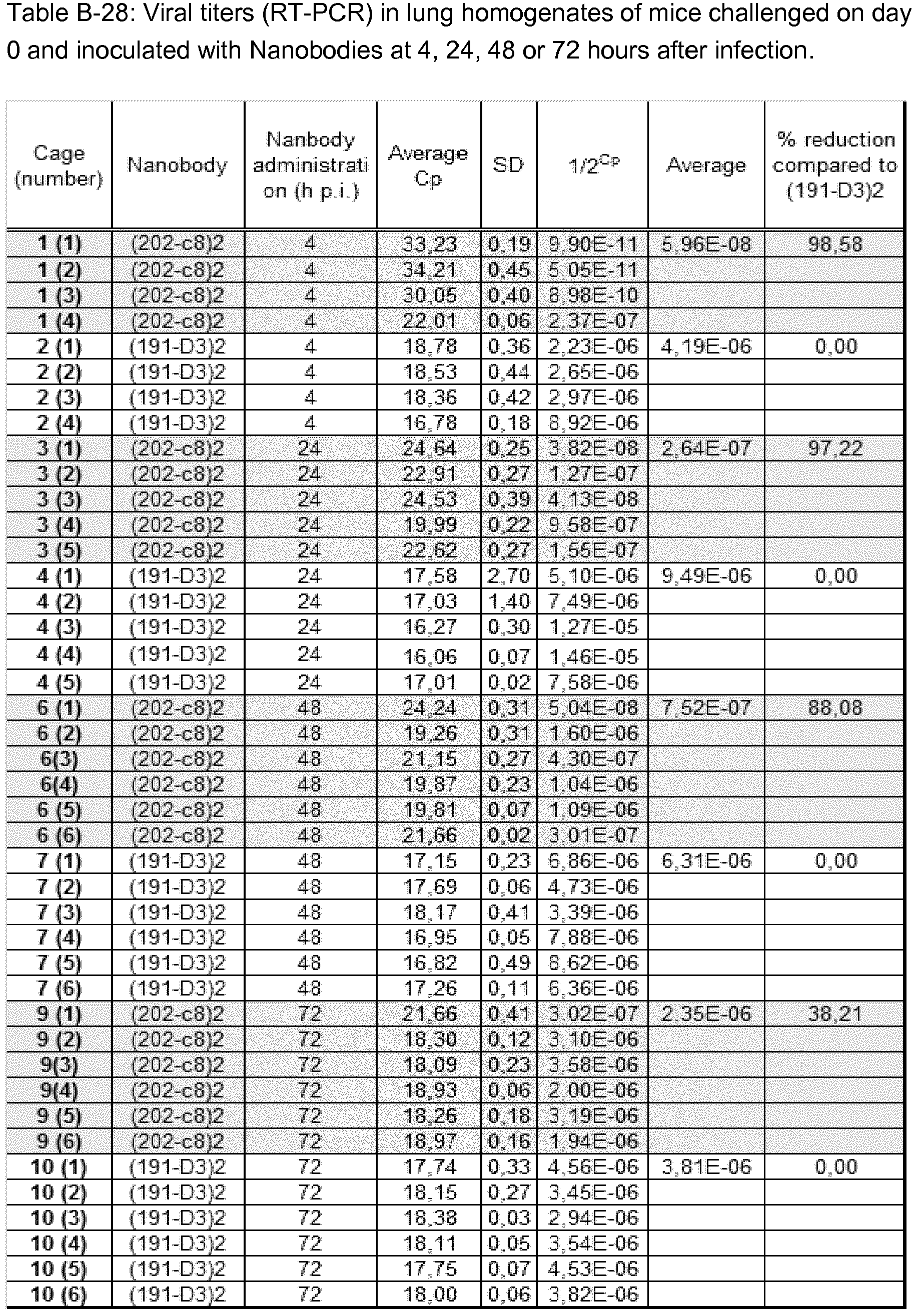

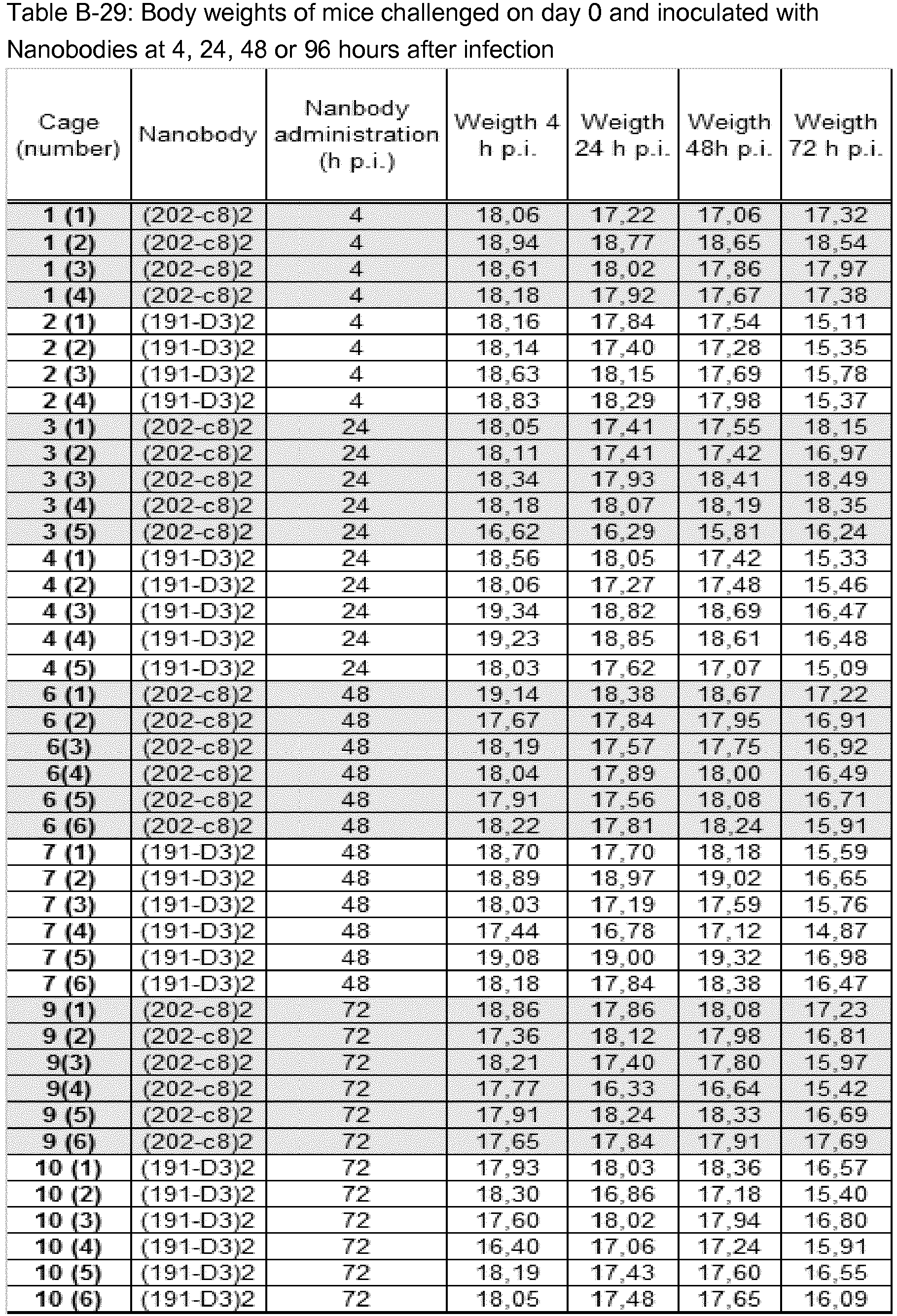

### Example 7: Use of nebulizer device for pulmonary delivery of P23IL0075 for systemic delivery during pre-clinical efficacy study.

| **Construct names** | **SEQ ID NO** | **Ref. SEQ ID NO** | **Amino Acid Sequence** |
|---|---|---|---|
| P23IL0075 or 119A3v16-9GS-ALB8-9GS-81A12v5 (81A12v5 is equal 81A12v4 with a (S49A) replaceme nt) | 5 | For 119A3v16 and 81a12v4 compare SEQ ID NO: 2578 and SEQ ID NO: 2584 in WO 2009/0686 27 | EVQLLESGGGLVQPGGSLRLSCAASGRIFSLPAS GNIFNLLTIAWYRQAPGKGRELVATINSGSRTYYA DSVKGRFTISRDNSKKTLYLQMNSLRPEDTAVYYC QTSGSGSPNFWGQGTLVTVSSGGGGSGGGSEV QLVESGGGLVQPGNSLRLSCAASGFTFSSFGMS WVRQAPGKGLEWVSSISGSGSDTLYADSVKGRF TISRDNAKTTLYLQMNSLRPEDTAVYYCTIGGSLS RSSQGTLVTVSSGGGGSGGGSEVQLLESGGGLV QPGGSLRLSCAASGRTLSSYAMGWFRQAPGKGR EFVARISQGGTAIYYADSVKGRFTISRDNSKNTLYL QMNSLRPEDTAVYYCAKDPSPYYRGSAYLLSGSY DSWGQGTLVTVSS |

This study was designed to evaluate the ability of a Nanobody to reach the systemic circulation in therapeutic amounts when delivered via the lung using a nebulizer device and to provide protection against a systemic inflammatory disease. The Nanobody tested was P23IL0075, which consists of 2 anti-IL23 Nanobody building blocks and 1 anti-serum albumin Nanobody building block (SEQ ID 5). The in vivo efficacy of P23IL0075 in acute in vivo mouse splenocyte model following pulmonary delivery was compared to the efficacy following subcutaneous delivery.

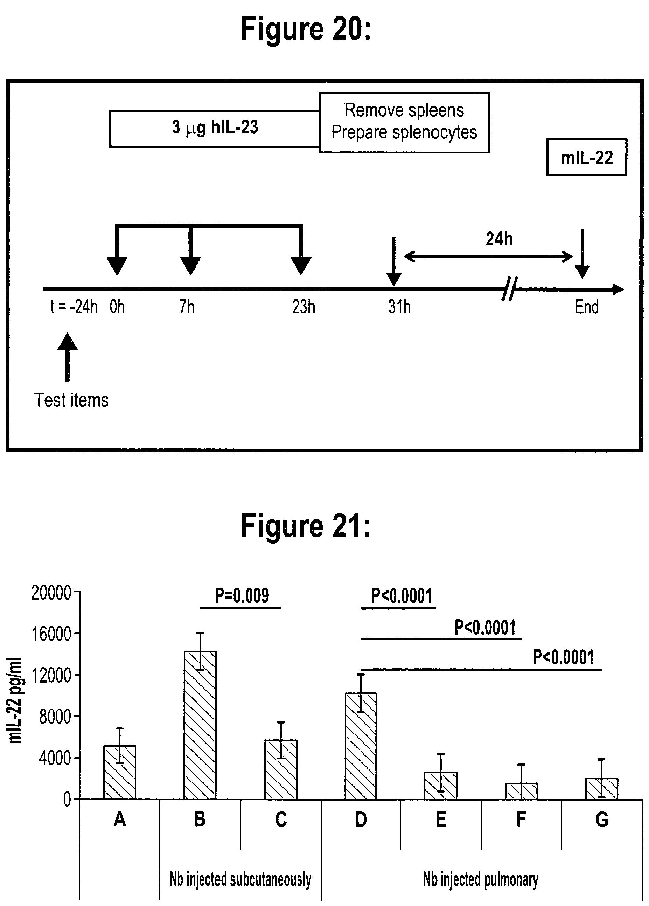

In short, C57BL/6J female mice (purchased from Charles River), 10-12 weeks old and weighing about 25 g, were used. An acclimatization period of at least one week was incorporated before the start of the experiment. Test items were administrated 24 hours before the first of three hIL-23 injections on t = 0h, 7h and 23h. Spleens were removed on t = 31h. Splenocytes were prepared and ex vivo stimulated for 24h after which mIL-22 levels were measured in the supernatant. The outline of the study is summarized below in Figure 20.
Drug was delivered to the animals at a 0.3 mg/kg dose subcutaneously, or at a 3 mg/kg or 7.8 mg/kg dose via the pulmonary route using the PennCentury MicroSprayer® device (Penn-Century MicroSprayer - Model IA-1C; 1.25" tip after the bend; FMJ-250 high pressure syringe). The subcutaneous 3 mg/kg dose was delivered as a 100 µL sample, whilst the pulmonary delivered 3 mg/kg and 7.8 mg/kg doses were delivered intratracheally in a total volume of 50 µL using the Microsprayer device. All P23IL0075 Nanobody samples were formulated in D-PBS + 0.01% Tween20. In addition, the 7.8 mg/kg P23IL0017 Nanobody sample was formulated in 10mM Histidine pH 6, 10% sucrose and administered intratracheally using the Microsprayer device.
24 hours after administration of the drug, the animals received an intraperitoneal injection of 3 µg human IL-23 (hIL23, full human IL23, i.e. composed of alpha subunit p19 (GenBank locus: NM_016584) and the p40 subunit of interleukin 12 (GenBank Locus: NM_002187) in a volume of 100 µL (t=0). 7 hours and 23 hours after this first injection, the animals received an additional intraperitoneal injection of 3µg hIL-23.
The mice from group A received PBS instead of hIL-23 at the same time points.

The test groups are shown in Table B-30.

**Table B-30: Overview of the test groups.**

| Group | Test Article + route | Induction |
|---|---|---|
| A | - | 3 x PBS intraperitoneally 100 µl |
| B | PBT administered subcutaneously (s.c.) 100 µl | 3 x 3 µg H IL-23 intraperitoneally 100 µl |
| C | 0.30 mg/kg P231L0075 administered subcutaneously (s.c.) 100 µl | 3 x 3 µg H IL-23 intraperitoneally 100 µl |
| D | PBT administered intratracheally (i.t.) 50 µl | 3 x 3 µg H IL-23 intraperitoneally 100 µl |
| E | 3.0 mg/kg P231L0075 administered intratracheally (i.t.) 50 µl | 3 x 3 µg H IL-23 intraperitoneally 100 µl |
| F | 7.8 mg/kg P231L0075 administered intratracheally (i.t.) 50 µl | 3 x 3 µg H IL-23 intraperitoneally 100 µl |
| G | 7.8 mg/kg P231L0075 administered intratracheally (i.t.) 50 µl | 3 x 3 µg H IL-23 intraperitoneally 100 µl |

Exactly 31h after the first hIL-23 injection, mice were bled for serum preparation and subsequently sacrificed. Spleens were removed and further processed for the *ex vivo* experiments. Briefly, splenocytes were isolated by homogenizing the spleens between frosted glass slides. Subsequently, splenocytes were washed and resuspended at a concentration of 10⁶ cells/ml in RPMI1640 supplemented with 10% FCS, 10U/ml penicillin, 100 µg/ml streptomycin, 1% non-essential amino acids, 1% sodium pyruvate, 2.5 mM HEPES and 0.00035% 2-mercapto ethanol. The cells were seeded at 200,000 cells/200µl/well in a 96-well culture plate pre-coated with hamster anti-mouse CD3e antibody (5 µg/mL in PBS, overnight at 4°C). For each spleen, 6 wells were seeded. The 96-well plates were incubated for 24 hours in a humidified CO₂ incubator. A commercial sandwich ELISA kit for mouse IL-22 (Antigenix) was used to measure the amount of mIL-22 in each of the 6 splenocyte supernatants. For each mouse, the mean mIL-22 concentration was calculated from the 6 replicate measurements.

The results are shown in Figure 21, which is a graph showing the results obtained in this Example 7 for the inhibition of the mIL-22 synthesis in a mouse splenocyte assay upon pulmonary administration of P231L0075 using the PennCentury Microsprayer device and compared with the inhibition of the mIL-22 synthesis upon subcutaneous administration. The results from group C were normalized to the mean mIL-22 concentration of group B, which was injected with hIL-23 only. The results from groups E, F and G were normalized to the mean mIL-22 concentration of group D, which was injected with hIL-23 only.
Subcutaneous delivery of 0.3 mg/kg P231L0075 Nanobody significantly blocked synthesis of mIL-22 to basal levels (P=0.009). Surprisingly, pulmonary delivery of 7.8 mg/mL P231L0075 Nanobody was also shown to significantly inhibit synthesis of mIL-22 to basal levels (P<0.0001), demonstrating that P231L0075 Nanobody is systemically released after pulmonary delivery. There was also no difference in the therapeutic efficacy of P231L0075 Nanobody formulated in D-PBS + 0.01% Tween20 (P<0.0001) as compared with P231L0075 Nanobody formulated in 10mM Histidine pH 6, 10% sucrose (P<0.0001). Interestingly, pulmonary delivery of a 3 mg/kg dose of P231L0075 also significantly inhibited mIL-22 synthesis (P<0.0001). There was no clear difference in the inhibition of mIL-22 synthesis provided by the 3 mg/kg (P<0.0001) and 7.8 mg/kg pulmonary delivered doses.

### Example 8: Systemic circulation and functionality of pulmonary administered and systemically delivered nanobodies/nanobody construct

### Sequences used:

| Construct name | SEQ ID NO | Ref. SEQ ID NO | Amino Acid Sequence |
|---|---|---|---|
| 4.10-Alb11 | 39 | SEQ ID NO: 113 in WO2009080714 | MAQVQLQESGGGLVQAGGSLRLSCAASGFTLGY YAIGWFRQAPGNEREGLSVITSGGGAIYYADSVK GRFTISRDNVKNTVSLQMNSLKPEDTAVYYCARV RAAFTSTTWTSPKWYDYWGQGTQVTVSSGGGG SGGGSEVQLVESGGGLVQPGNSLRLSCAASGFT FRSFGMSWVRQAPGKEPEWVSSISGSGSDTLYA DSVKGRFTISRDNAKTTLYLQMNSLKPEDTAVYYC TIGGSLSRSSQGTQVTVSSAAAEQKLISEEDLNGA AHHHHHH |
| IL6R202 | 40 | SEQ ID NO: 568 in WO20080 20079 | EVQLVESGGGLVQPGGSLRLSCAASGFTFSDYDI GWFRQAPGKGREGVSGISSSDGNTYYADSVKGR FTISRDNAKNTLYLQMNSLRPEDTAVYYCAAEPPD SSWYLDGSPEFFKYWGQGTLVTVSSGGGGSGG GSEVQLVESGGGLVQPGNSLRLSCAASGFTFSSF GMSWVRQAPGKGLEWVSSISGSGSDTLYADSVK GRFTISRDNAKTTLYLQMNSLRPEDTAVYYCTIGG SLSRSSQGTLVTVSS |

### Example 8.1: Intratracheal administration of 2 doses of 250 µg 4.10 Nanobodies increases circulating levels of leptin in mice.

A first group of 10 mice received two doses of 250 µg 4.10-Alb11 Nanobody construct through intra-tracheal inoculation. A second group of 10 mice received two doses of 250 µg IL6R202 Nanobody construct through intra-tracheal inoculation (i.t.). A third group of 7 mice received two doses of 250 µg 4.10-Alb11 Nanobody construct through intra-peritoneal injection (i.p.). A fourth group of 7 mice received two doses of 250 µg IL6R202 Nanobody construct through intra-peritoneal injection. The first dose was given on day 0, the second dose on day 2. Blood was taken one day before the first dose was given and one day after the second dose was given. Levels of leptin and Nanobody were determined. Data is represented as average ± SD.

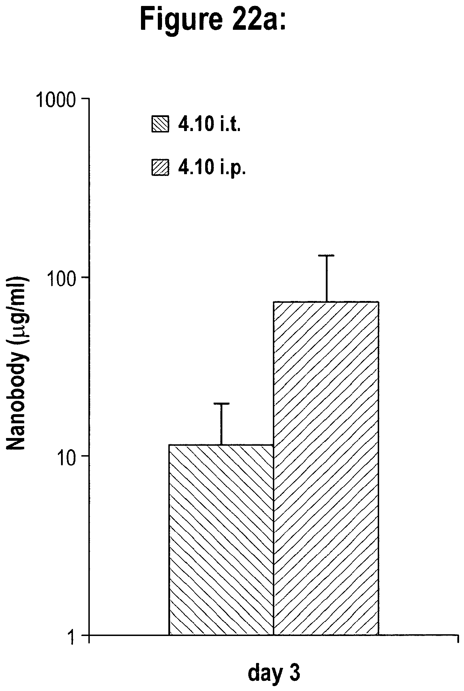

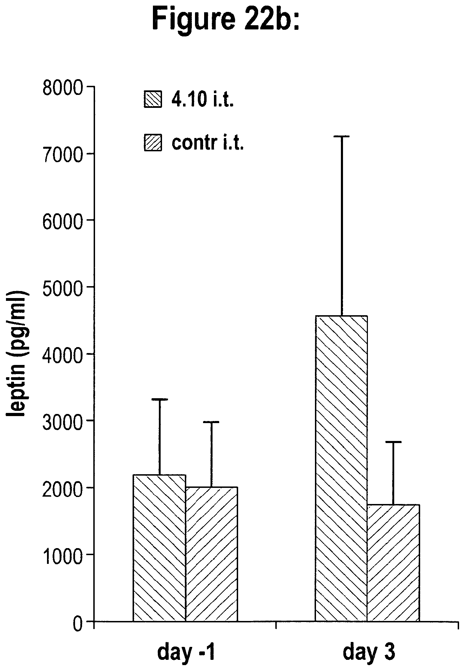

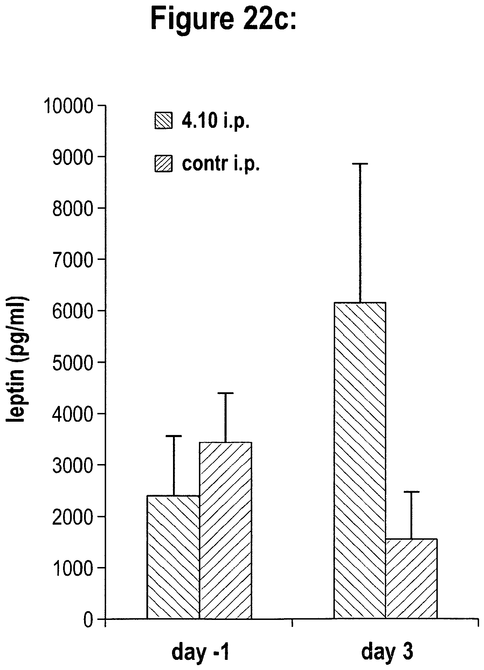

As shown in Figure 22, following i.t. injection of two doses of 4.10-Alb1, this Nanobody construct 4.10-Alb1 was clearly detected in blood (11.7 ± 8.08 µg /ml). This is -16% of what was detected following the i.p. injections (73.8 ± 61.5 µg /ml). Because the Elisa assay used to quantify this Nanobody depends on the interaction with a leptin receptor fragment, this data indicates that the Nanobodies present in circulation after i.t. and i.p. administration were intact, functional Nanobodies. This was further supported as also increased concentrations of leptin were detected in blood of mice treated with this Nanobody irrespective of the route of inoculation (Figure 22b (i.t.) and figure 22c (i.p.)).

### Example 8.2: Dose dependent increase of circulating leptin levels following i.t. administration of 4 increasing amounts of 4.10-Alb1 Nanobody constructs

Mice were given increasing amounts of 4.10-Alb-1 nanobody construct at day 0, 3, 6 and 9. On day 0, mice received 25 µg, on day 3 mice received 50 µg, on day 6 mice received 125 µg and on day 9 mice received 250 µg of 4.10-Alb-1 nanobody construct. Nanobodies were given i.p. or i.t. The i.t. groups consisted of 10 mice, the i.p. treated groups consisted of 7 mice. One day after each 4.10-Alb-1 nanobody construct administration blood was collected.

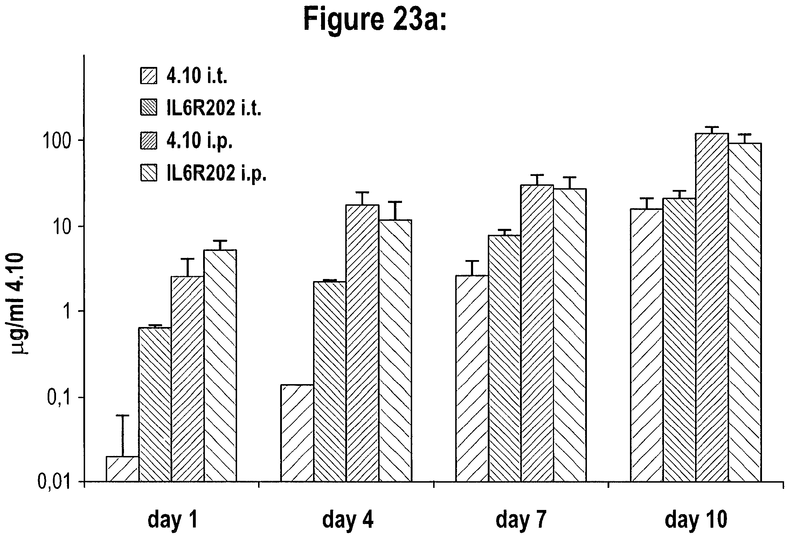

As shown in figure 23a. Nanobody constructs 4.10-Alb1 and IL6R202 were detected in blood following each i.t. inoculation. Inoculation of a higher amount resulted in higher concentrations present in blood. As expected, concentrations of Nanobody constructs in blood were higher following i.p. injections. Because the Elisa assays used to quantify these Nanobody constructs depend on the interaction with a leptin receptor fragment or IL6 receptor fragment, this data indicates that the Nanobody constructs present in circulation after i.t. and i.p. administration were intact, i.e. functional Nanobody constructs.

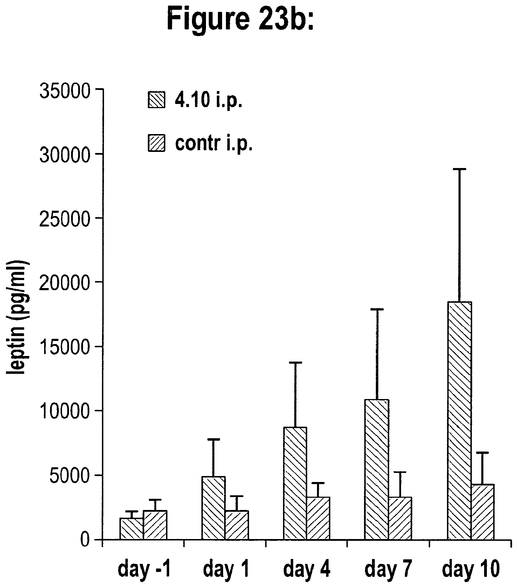

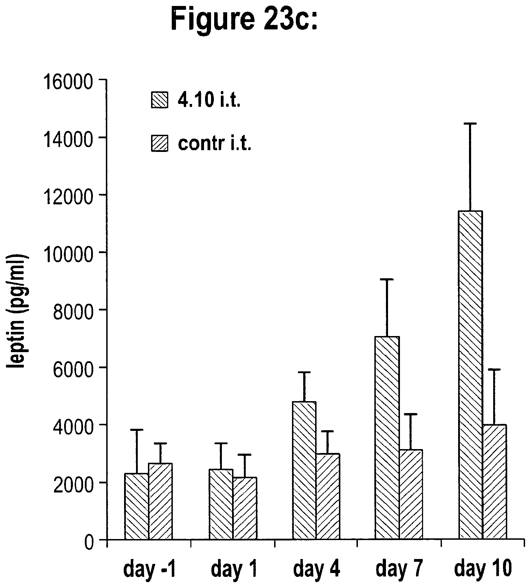

One day after the i.p injection of 25 µg leptin levels were already increased when compared to IL6R202 injected animals (Figure 23b). Leptin levels increased further after each additional injection with the 4.10-Alb-1 nanobody construct. After intratracheal inoculation increased leptin levels were detected for the first time after the inoculation of the second dose of 50 µg (Figure 23c). Levels further increased after inoculation of the 125 and 250 µg doses.

### Example 8.3: Increase in body weight following i.t. administration of 4 increasing amounts of 4.10 Nanobodies

During the Nanobody treatment as described in example 8.2, body weight of all animals was determined daily. As expected, the body weight of mice that received 4.10-Alb1 via i.p. injections clearly gained weight (Figure 24a) while control mice did not (figure 24b). Also for the mice that were treated i.t. with the 4.10-Alb1 Nanobody construct body weight showed a tendency to increase more than the body weight of control treated animals (figure 24c and figure 24d)).

To model bodyweight as a function of time, while incorporating the different treatment groups as well as the intra mouse variation we fit a mixed model in SAS using the following code:

| |
|---|
| proc mixed data=Bodyweight; |
| class muis group day; |
| model Bodyweight=dag dag*group/s ; |
| repeated day/subject=muis type=un rcorr r; |
| run; |

The line that starts with model defines how the fixed structure of the model looks like. We include a term that corresponds to the time (day) and we include the interaction term of time with treatment group (group) because we assume that the effect on the bodyweight may be different for each treatment group. Note that the main effect of group is not in the model anymore. This term appears to be not significant (p=0.125) which means that the bodyweight at day -1 is the same for each treatment group. The latter makes sense because at day -1 no treatment has been administered to the mouse yet. The intra mouse variation is covered by the repeated statement in the sas code above. For this variation we should find the most appropriate correlation structure for the different time point. By comparing the AIC criteria of several models with different correlation structures we obtained that the *unstructured* was the most appropriate correlation structure. This means that the correlation between all the different time points has been estimated, as well as the variance at each time point. The resulting correlation structure holds for every mouse and represents the intra mouse variation.

The residuals of the mixed model (see Figure 24e) look normally distributed and homogeneous hence no violation of the assumptions is present. We agree that this model is a good model for the bodyweight levels.

From this model we can derive the p-values for the comparison between the different treatment groups. In Table B-31 we present the results of the statistical tests to compare bodyweight increase for different treatment groups.

Table B-31 Statistical tests to compare bodyweight increase for different treatment groups.

| Table B-31 Estimates | | | | | | | | |
|---|---|---|---|---|---|---|---|---|
| Label | Estimate | Standard Error | DF | t Value | Pr > \|t\| | Alpha | Lower | Upper |
| test i.t. 4.10-Alb1 vs IL6R202 | 0.07239 | 0.02090 | 39 | 3.46 | 0.0013 | 0.05 | 0.03010 | 0.1147 |
| test i.t. 4.10-Alb1 vs IL6R202 | 0.2364 | 0.02437 | 39 | 9.70 | <.0001 | 0.05 | 0.1871 | 0.2857 |
| test i.t. 4.10-Alb1 vs i.p 4.10-Alb1 | -0.1482 | 0.02294 | 39 | -6.46 | <.0001 | 0.05 | -0.1946 | -0.1018 |

The first row in Table B-31 represents the test that compares the two i.t. treatment groups with respect to their bodyweight increase. From the p-value (0.0013) we may conclude that increase in bodyweight is significantly larger for the 4.10-Alb1 group compared to the control group (IL6R202). The estimated difference in bodyweight increase is 0.07239. The second row in Table B-31 represents the test that compares the two ip treatment groups with respect to their bodyweight increase. From the p-value (<0.0001) we may conclude that increase in bodyweight is significantly larger for the 4.10-Alb1 group compared to the control group (IL6R202). The estimated difference in bodyweight increase is 0.2364. The third row in Table B-31 represents the test that compares the 4.10-Alb1 i.t. treatment group with the 4.10-Alb1 i.p. treatment group with respect to their bodyweight increase. From the p-value (<0.0001) we may conclude that increase in bodyweight is significantly larger for the i.p. group compared to the i.t. group. The estimated difference in bodyweight increase is -0.1482. The predicted bodyweight model with corresponding confidence bands is presented in Figure 24f&g.

### Preferred aspects or particular embodiments described in the application:

### Method aspects:

1. Method of providing and/or delivering an effective amount of an immunoglobulin single variable domain and/or construct thereof to a mammal, e.g. human; wherein said immunoglobulin single variable domain and/or construct thereof is directed against at least one target; and wherein the method comprises the step of administering said immunoglobulin single variable domain and/or construct thereof to the pulmonary tissue.
2. Method of aspect 1, wherein the process of administering comprises the step of forming an aerosol comprising said immunoglobulin single variable domain and/or construct thereof by an appropriate inhaler device such as e.g. nebulizer, metered dose liquid inhalers and/or dry powder inhalers, preferably mesh nebulizer.
3. Method of aspect 1 or aspect 2, wherein an effective amount of an immunoglobulin single variable domain and/or construct thereof to the systemic circulation of said mammal, e.g. human, is provided.
4. Method of aspect 3, wherein the method is able to deliver said immunoglobulin single variable domain and/or construct thereof to the systemic circulation with a substantial absolute bioavailability, e.g. with an absolute bioavailability that is at least 10%, preferably 20%, more preferably 30%, more preferably 40%, more preferably 50% after administration of a single dose of said immunoglobulin single variable domain and/or construct thereof.
5. Method of aspect 3, wherein the half life or terminal half life of said immunoglobulin single variable domain and/or construct thereof in the systemic circulation is longer than 5 hours, preferably 6 hours or more, more preferably 7, 8 or 9 hours or more, even more preferably is 10 hours, 15 hours, or 20 hours or more, more preferred is 1, 2, 3, 4, 5, 6 or more days.
6. Method of any previous aspects, wherein said immunoglobulin single variable domain and/or construct thereof is a Nanobody and/or construct thereof.
7. Method of any previous aspects, wherein said immunoglobulin single variable domain and/or construct thereof is selected from the group consisting of a Nanobody, a construct comprising or essentially consisting of two Nanobodies directed against the same or different antigens optionally connected by a linker; and a construct comprising or essentially consisting of 3 Nanobodies directed against the same or different antigens optionally connected by a linker.
8. Method of any previous aspects, wherein said immunoglobulin single variable domain and/or construct thereof is selected from the group consisting of a Nanobody, and a construct comprising or essentially consisting of two Nanobodies directed against the same or different antigens optionally connected by a linker.
9. Method of any previous aspects, wherein said immunoglobulin single variable domain and/or construct thereof is selected from the group consisting of a construct comprising or essentially consisting of two Nanobodies directed against the same or different antigens optionally connected by a linker; and a construct comprising or essentially consisting of 3 Nanobodies directed against the same or different antigens optionally connected by a linker.
10. Method of any previous aspects, wherein said immunoglobulin single variable domain and/or construct thereof is selected from the group consisting of a construct comprising or essentially consisting of two Nanobodies directed against the same or different antigens optionally connected by a linker.
11. Method of aspect 1, wherein the method additionally comprises the step of using an intranasal delivery device in order to administer said immunoglobulin single variable domain and/or construct thereof to the pulmonary tissue of the mammal.
12. Method of any previous aspects, wherein the antigen is a antigen in the pulmonary tissue.
13. Method of any previous aspects, wherein the antigen is an antigen in the pulmonary tissue and is derived from a microorganism such as a virus, e.g. RSV such as e.g. RSV407 and variants thereof, e.g. functional variants of RSV407 that have up to 30, preferably 25, 20, 15, 10, or 5 mutated amino acid residues, or avian influenza virus, a fungi, a parasite or a bacterium and also allergic entities like house dust mite/protein.
14. Method of any previous aspects, wherein the antigen is a druggable antigen primarily expressed in the mammal but expressed also outside the pulmonary tissue of said mammal, e.g. is a) RANK-L, and the immunoglobulin single variable domain is e.g. RANKL008AA and variants thereof, e.g. functional variants of RANKL008AA that have up to 30, preferably 25, 20, 15, 10, or 5 mutated amino acid residues; or b) van Willebrand Factor and the immunoglobulin single variable domain is e.g. ALX-0081 and variants thereof, e.g. functional variants of ALX-0081 that have up to 30, preferably 25, 20, 15, 10, or 5 mutated amino acid residues; or c)leptin and the immunoglobulin single variable domain is 4.10-Alb1 and variants thereof, e.g. functional variants of 4.10-Alb1 that have up to 30, preferably 25, 20, 15, 10, or 5 mutated amino acid residues.
15. Method of any previous aspects, wherein an effective amount of said immunoglobulin single variable domain and/or construct thereof is administered once daily or once every 2 to 7 days, preferably once daily.
16. Method of any previous aspects, wherein an effective amount of said immunoglobulin single variable domain and/or construct thereof is administered once daily or once every 2 to 7 days, preferably once daily and wherein the construct is preferably administered locally.
17. Method of any previous aspects, wherein an effective amount of said immunoglobulin single variable domain and/or construct thereof is delivered to the systemic circulation when administered once daily or once every 2 to 7 days, preferably once daily, wherein none of said construct is directed against a serum protein.
18. Method of any previous aspects, wherein about 10, 20, 30, 40, 50, 60, 70, 80% or less of said immunoglobulin single variable domain and/or construct thereof is stable in the pulmonary tissue for at least 24 hours after administration of said construct.
19. Method of any previous aspects, wherein said construct comprises in addition an immunoglobulin single variable domain against a serum protein, e.g. human serum protein such as human serum albumin or human Fc-IgG1.
20. Method of aspect 16, wherein the systemic bioavailability of said construct is up to about 10 to 50%, preferably up to 20%, more preferably up to 30%, even more preferably up to 40%, most preferred up to 50%.
21. Method of any previous aspects, wherein the in vivo terminal half life of the immunoglobulin single variable domain and/or construct thereof in the systemic circulation of e.g. rats and/or humans is at least 5 times higher compared to the in vivo half life of the same immunoglobulin single variable domain and/or construct thereof when administered intravenously, more preferably 6 to 10 times, most preferred about 10 times higher.
22. Method of any previous aspects, wherein at least one of the antigen is involved or plays a part in respiratory diseases, e.g. COPD, asthma and respiratory viruses infection.
23. Method of any previous aspects wherein the mammal is a human, e.g. a human with a disease.

### Use aspect:

1. Use of an immunoglobulin single variable domain and/or construct thereof for delivering an effective amount of said immunoglobulin single variable domain and/or construct thereof to a mammal, e.g. human; wherein said immunoglobulin single variable domain and/or construct thereof is directed against at least one antigen; and wherein the said immunoglobulin single variable domain and/or construct thereof is administered to the pulmonary tissue.
2. Use of aspect A, wherein the process of administering comprises the step of forming an aerosol comprising said immunoglobulin single variable domain and/or construct thereof by an appropriate inhaler device such as e.g. nebulizer, metered dose liquid inhalers and/or dry powder inhalers.
3. Use of aspect A or aspect B, wherein an effective amount of an immunoglobulin single variable domain and/or construct thereof to the systemic circulation of said mammal, e.g. human, is provided.
4. Use of aspect C, wherein the delivery of said immunoglobulin single variable domain and/or construct thereof to the systemic circulation is achieved with a substantial absolute bioavailability, e.g. with an absolute bioavailability that is at least 10%, preferably 20%, more preferably 30%, more preferably 40%, more preferably 50% after administration of a single dose of said immunoglobulin single variable domain and/or construct thereof.
5. Use of aspect C, wherein the half life or terminal half life of said immunoglobulin single variable domain and/or construct thereof in the systemic circulation is longer than 5 hours, preferably 6 hours or more, more preferably 7, 8 or 9 hours or more, even more preferably is 10 hours, 15 hours, or 20 hours or more, more preferred is 1, 2, 3, 4, 5, 6 or more days.
6. Use of any previous aspects, wherein said immunoglobulin single variable domain and/or construct thereof is a Nanobody and/or construct thereof.
7. Use of any previous aspects, wherein said immunoglobulin single variable domain and/or construct thereof is selected from the group consisting of a Nanobody, a construct comprising or essentially consisting of two Nanobodies directed against the same or different antigens optionally connected by a linker; and a construct comprising or essentially consisting of 3 Nanobodies directed against the same or different antigens optionally connected by a linker.
8. Use of any previous aspects, wherein said immunoglobulin single variable domain and/or construct thereof is selected from the group consisting of a Nanobody, and a construct comprising or essentially consisting of two Nanobodies directed against the same or different antigens optionally connected by a linker.
9. Use of any previous aspects, wherein said immunoglobulin single variable domain and/or construct thereof is selected from the group consisting of a construct comprising or essentially consisting of two Nanobodies directed against the same or different antigens optionally connected by a linker; and a construct comprising or essentially consisting of 3 Nanobodies directed against the same or different antigens optionally connected by a linker.
10. Use of any previous aspects, wherein said immunoglobulin single variable domain and/or construct thereof is selected from the group consisting of a construct comprising or essentially consisting of two Nanobodies directed against the same or different antigens optionally connected by a linker.
11. Use of any previous aspects, wherein the immunoglobulin single variable domain and/or construct thereof is administered to the mammal, e.g. human, by using an intranasal delivery device.
12. Use of any previous aspects, wherein the antigen is a antigen in the pulmonary tissue.
13. Use of any previous aspects, wherein the antigen is not an antigen in the pulmonary tissue.
24. Use of any previous aspects, wherein the antigen is an antigen in the pulmonary tissue and is derived from a microorganism such as a virus, e.g. RSV such as e.g. RSV407 and variants thereof, e.g. variants of functional RSV407 that have up to 30, preferably 25, 20, 15, 10, or 5 mutated amino acid residues, or avian flu virus, a fungi, a parasite or a bacterium and also allergic entities like house dust mite/protein.
14. Use of any previous aspects, wherein the antigen is a druggable antigen primarily expressed in the mammal but expressed outside the pulmonary tissue of said mammal, e.g. RANK-L such as e.g. RANKL008AA and variants thereof, e.g. functional variants of RANKL008AA that have up to 30, preferably 25, 20, 15, 10, or 5 mutated amino acid residues, or van Willebrand Factor such as e.g. ALX-0081 and variants thereof, e.g. functional variants of RANKL008AA that have up to 30, preferably 25, 20, 15, 10, or 5 mutated amino acid residues.
15. Use of any previous aspects, wherein an effective amount of said immunoglobulin single variable domain and/or construct thereof is delivered to the systemic circulation when administered once daily or once every 2 to 7 days, preferably once daily.
16. Use of any previous aspects, wherein an effective amount of said immunoglobulin single variable domain and/or construct thereof is administered once daily or once every 2 to 7 days, preferably once daily and wherein the immunoglobulin single variable domain and/or construct thereof is preferably delivered in the pulmonary tissue.
17. Use of any previous aspects, wherein an effective amount of said immunoglobulin single variable domain and/or construct thereof is administered once daily or once every 2 to 7 days, preferably once daily, wherein none of said immunoglobulin single variable domain and/or construct thereof is directed against a serum protein.
18. Use of any previous aspects, wherein about 10, 20, 30, 40, 50, 60, 70, 80% or less of said immunoglobulin single variable domain and/or construct thereof is stable in the pulmonary tissue for at least 24 hours after administration of said immunoglobulin single variable domain and/or construct thereof.
19. Use of any previous aspects, wherein said immunoglobulin single variable domain and/or construct thereof comprises in addition an immunoglobulin single variable domain against a serum protein, e.g. human serum protein such as human serum albumin or human Fc-IgG1.
20. Use of aspect Q, wherein the systemic bioavailability of said immunoglobulin single variable domain and/or construct thereof is up to about 10 to 50%, preferably up to 50%.
21. Use of any previous aspects, wherein the in vivo terminal half life of the immunoglobulin single variable domain and/or construct thereof in e.g. rats is at least 5 times higher compared to the in vivo half life of the same immunoglobulin single variable domain and/or construct thereof when administered intravenously, more preferably 6 to 10 times, most preferred about 10 times higher.
22. Use of any previous aspects, wherein at least one of the antigen is involved or plays a part in respiratory diseases, e.g. COPD, asthma and respiratory viruses infection.
   i. Pharmaceutical compositions and devices of the description:
   ii. Pharmaceutical composition suitable for pulmonary administration according to a use or method as described in the above aspects.
   iii. Pharmaceutical composition of aspect i, wherein the composition comprises a) a construct comprising at least one immunoglobulin single variable domain and/or construct thereof directed against at least one antigen or essentially consisting of at least one immunoglobulin single variable domain and/or construct thereof directed against at least one antigen; and b) optionally comprising suitable excipients such as e.g. buffers, stabilizers and/or propellants.
   iv. Pharmaceutical composition of aspect I or ii that is administered once daily or once every 2 to 7 days, preferably once daily.
   v. Pharmaceutical composition of aspect I to iii that is a liquid.
   vi. Pharmaceutical composition of aspect i to iii that is a dry powder.
   vii. Pharmaceutical device suitable in the methods and/or uses as described above and/or suitable in the use with a pharmaceutical composition of aspects I to v.
   viii. Pharmaceutical device of claim vi that is an inhaler for liquids such as e.g. a suspension of fine solid particles or liquid droplets in a gas.
   ix. Pharmaceutical device of claim vi that is dry powder inhaler.

### Dosing interval:

a) Method of administering an immunoglobulin single variable domain and/or construct thereof, e.g. a Nanobody, to the pulmonary tissue as described above; wherein said administration is once a day, once every 2, 3, 4, 5, 6, or once every week, preferably once every day.
b) Method of aspect a); wherein said immunoglobulin single variable domain and/or construct thereof, e.g. a Nanobody, is delivered to the systemic circulation in an effective amount.
c) Use of an agent of the invention for administration once a day, once every 2, 3, 4, 5, 6, or once every week, preferably once every day.
d) Use of aspect c); wherein said immunoglobulin single variable domain and/or construct thereof, e.g. a Nanobody, is delivered to the systemic circulation in an effective amount.

Dosing interval for an anti-viral immunoglobulin single variable domain and/or construct thereof directed against said virus wherein said virus can cause respiratory tract infections:
e) Method of treating respiratory tract infections caused by a virus optionally after the therapeutic window for conventional anti-viral medications is closed with a single effective dose of a pharmaceutical composition comprising an immunoglobulin single variable domain and/or construct thereof; wherein said immunoglobulin single variable domain is directed against said virus.
f) Method of aspect e); wherein said treating is done after the therapeutic window for conventional anti-viral medications is closed.
g) Method of aspect e) or f); wherein said immunoglobulin single variable domain is a Nanobody.
h) Method of aspect e), f) or g); wherein said respiratory tract infections caused by a virus is selected from the group of influenza, viral bronchiolitis caused by respiratory syncytial virus (RSV), and respiratory diseases caused by an adenovirus.
i) Method of aspect e), f), g) or h) ; wherein said therapeutic window for conventional anti-viral medications is closed after 1 or more days after first infections, preferably 2 or more days after first infections, more preferably 3 or more days after first infections.
j) Method of aspect e), f), g), h) or i); wherein said therapeutic window for conventional anti-viral medications is closed after 1 or more days after first disease symptoms, preferably 2 or more days after first disease symptoms, more preferably 3 or more days after first disease symptoms.
k) Use of an agent of the invention for treating respiratory tract infections caused by a virus optionally after the therapeutic window for conventional anti-viral medications is closed with a single effective dose of a pharmaceutical composition comprising an immunoglobulin single variable domain and/or construct thereof; wherein said immunoglobulin single variable domain is directed against said virus.
l) of aspect k); wherein said treating is done after the therapeutic window for conventional anti-viral medications is closed.
m) Use of aspect k) or l); wherein said immunoglobulin single variable domain is a Nanobody.
n) Use of aspect k), l) or m); wherein said respiratory tract infections caused by a virus is selected from the group of influenza, viral bronchiolitis caused by respiratory syncytial virus (RSV), and respiratory diseases caused by an adenovirus.
o) Use of aspect k), l), m) or n); wherein said therapeutic window for conventional anti-viral medications is closed after 1 or more days after first infections, preferably 2 or more days after first infections, more preferably 3 or more days after first infections.
p) Use of aspect k), l), m), n) or o); wherein said therapeutic window for conventional anti-viral medications is closed after 1 or more days after first disease symptoms, preferably 2 or more days after first disease symptoms, more preferably 3 or more days after first disease symptoms.

### Particularly preferred aspects:

1. Method of providing and/or delivering an effective amount of an immunoglobulin single variable domain and/or construct thereof to a mammal, e.g. human; wherein said immunoglobulin single variable domain and/or construct thereof is directed against at least one target; and wherein the method comprises the step of administering said immunoglobulin single variable domain and/or construct thereof to the pulmonary tissue; wherein the delivery of said immunoglobulin single variable domain and/or construct thereof to the systemic circulation is achieved with a substantial bioavailability, i.e.
   a. in case the immunoglobulin single variable domain and/or construct thereof consists of essentially not more than 150, more preferably 140, even more preferably 130, most preferred not more than 120 amino acid residues (e.g. consists of a monovalent nanobody) the delivery of said immunoglobulin single variable domain and/or construct thereof to the systemic circulation is achieved with a bioavailability (compared to i.v. injection) that is at least 10%, preferably 15%, most preferably 20% after administration of a single dose of said immunoglobulin single variable domain and/or construct thereof; or
   b. in case the immunoglobulin single variable domain and/or construct thereof consists of essentially not more than 300, more preferably 280, even more preferably 260, most preferred not more than 240 amino acid residues (e.g. consists of two monovalent nanobodies and a linker) the delivery of said immunoglobulin single variable domain and/or construct thereof to the systemic circulation is achieved with a bioavailability (compared to i.v. injection) that is at least 5%, preferably 7.5%, most preferably 10% after administration of a single dose of said immunoglobulin single variable domain and/or construct thereof; or
   c. in case the immunoglobulin single variable domain and/or construct thereof consists of essentially not more than 450, more preferably 420, even more preferably 390, most preferred not more than 360 amino acid residues (e.g. consists of three monovalent nanobodies and two linkers) the delivery of said immunoglobulin single variable domain and/or construct thereof to the systemic circulation is achieved with a bioavailability (compared to i.v. injection) that is at least 5% after administration of a single dose of said immunoglobulin single variable domain and/or construct thereof.
2. Method of delivering an effective amount of an immunoglobulin single variable domain and/or construct thereof to a human; wherein said immunoglobulin single variable domain and/or construct thereof is directed against at least one antigen; and wherein the method comprises the step of administering said immunoglobulin single variable domain and/or construct thereof to the pulmonary tissue.
3. Method of aspects 1 or 2, wherein the process of administering comprises the step of forming an aerosol comprising said immunoglobulin single variable domain and/or construct thereof by an appropriate inhaler device such as e.g. a mesh nebulizer.
4. Method of previous aspects, wherein an effective amount of an immunoglobulin single variable domain and/or construct thereof to the systemic circulation of said human is provided.
5. Method of aspect 4, wherein the method is able to deliver said immunoglobulin single variable domain and/or construct thereof to the systemic circulation with an absolute bioavailability that is at least 10% after administration of a single dose administration of said immunoglobulin single variable domain and/or construct thereof.
6. Method of aspect 4, wherein the terminal half life of said immunoglobulin single variable domain and/or construct thereof in the systemic circulation is longer than 5 hours.
7. Method of any previous aspects, wherein said immunoglobulin single variable domain and/or construct thereof is a Nanobody and/or construct thereof.
8. Method of any previous aspects, wherein said immunoglobulin single variable domain and/or construct thereof is selected from the group of a Nanobody, a construct essentially consisting of two Nanobodies directed against the same or different antigens optionally connected by a linker; and a construct essentially consisting of 3 Nanobodies directed against the same or different antigens optionally connected by a linker.
9. Method of administering an immunoglobulin single variable domain and/or construct thereof to the pulmonary tissue according to aspects 1 to 8; wherein said administration is once a day.

## Claims

1. Immunoglobulin single variable domain and/or construct thereof directed against at least one antigen for use in a method of treatment by delivering an effective amount of an immunoglobulin single variable domain and/or construct thereof to the systemic circulation of a human via the pulmonary route; wherein the method comprises the step of administering said immunoglobulin single variable domain and/or construct thereof to the pulmonary tissue at dosage intervals of once a day or longer.

2. Immunoglobulin single variable domain and/or construct thereof for use according to claim 1, wherein the process of administering comprises the step of forming an aerosol comprising said immunoglobulin single variable domain and/or construct thereof by an appropriate inhaler device.

3. Immunoglobulin single variable domain and/or construct thereof for use according to claim 1 or claim 2, wherein delivery of said immunoglobulin single variable domain and/or construct thereof to the systemic circulation is achieved with a bioavailability:
a) in case the immunoglobulin single variable domain and/or construct thereof consists of not more than 150 amino acid residues, the delivery of said immunoglobulin single variable domain and/or construct thereof to the systemic circulation is achieved with a bioavailability (compared to i.v. injection) that is at least 10% after administration of a single dose administration of said immunoglobulin single variable domain and/or construct thereof;
b) in case the immunoglobulin single variable domain and/or construct thereof consists of not more than 300 amino acid residues, the delivery of said immunoglobulin single variable domain and/or construct thereof to the systemic circulation is achieved with a bioavailability (compared to i.v. injection) that is at least 7.5% after administration of a single dose of said immunoglobulin single variable domain and/or construct thereof; or
c) in case the immunoglobulin single variable domain and/or construct thereof consists of not more than 450 amino acid residues, the delivery of said immunoglobulin single variable domain and/or construct thereof to the systemic circulation is achieved with a bioavailability (compared to i.v. injection) that is at least 5% after administration of a single dose of said immunoglobulin single variable domain and/or construct thereof.

4. Immunoglobulin single variable domain and/or construct thereof for use according to claim 1 or claim 2, wherein the terminal half life of said immunoglobulin single variable domain and/or construct thereof in the systemic circulation is longer than 5 hours.

5. Immunoglobulin single variable domain and/or construct thereof for use according to any of claims 1 to 4, wherein said immunoglobulin single variable domain and/or construct thereof is a V_{HH}, a humanized V_{HH}, and/or construct thereof.

6. Immunoglobulin single variable domain and/or construct thereof for use according to any of claims 1 to 5, wherein said immunoglobulin single variable domain and/or construct thereof is selected from the group of a V_{HH}, a humanized V_{HH}, a construct consisting of two V_{HH}'s or humanized V_{HH}'s directed against the same or different antigens optionally connected by a linker; and a construct consisting of 3 V_{HH}'s or humanized V_{HH}'s directed against the same or different antigens optionally connected by a linker.

## Patentansprüche

1. Variable Immunglobulin-Einzeldomäne und/oder Konstrukt davon, die/das gegen wenigstens ein Antigen gerichtet ist, zur Verwendung in einem Verfahren zur Behandlung durch Zuführen einer wirksamen Menge einer variablen Immunglobulin-Einzeldomäne und/oder eines Konstrukts davon an den systemischen Kreislauf eines Menschen auf dem pulmonalen Weg; wobei das Verfahren den Schritt des Verabreichens der variablen Immunglobulin-Einzeldomäne und/oder des Konstrukts davon an das pulmonale Gewebe in Dosisintervallen von einmal täglich oder länger umfasst.

2. Variable Immunglobulin-Einzeldomäne und/oder Konstrukt davon zur Verwendung gemäß Anspruch 1, wobei das Verfahren der Verabreichung den Schritt des Bildens eines Aerosols, umfassend die variable Immunglobulin-Einzeldomäne und/oder das Konstrukt davon, durch eine geeignete Inhalatorvorrichtung umfasst.

3. Variable Immunglobulin-Einzeldomäne und/oder Konstrukt davon zur Verwendung gemäß Anspruch 1 oder Anspruch 2, wobei die Zuführung der variablen Immunglobulin-Einzeldomäne und/oder des Konstrukts davon an den systemischen Kreislauf mit einer Bioverfügbarkeit erzielt wird:
a) falls die variable Immunglobulin-Einzeldomäne und/oder das Konstrukt davon aus nicht mehr als 150 Aminosäureresten besteht, wird die Zuführung der variablen Immunglobulin-Einzeldomäne und/oder des Konstrukts davon an den systemischen Kreislauf mit einer Bioverfügbarkeit (im Vergleich zu i.v.-Injektion) erzielt, die nach Verabreichung einer Einzeldosisverabreichung der variablen Immunglobulin-Einzeldomäne und/oder des Konstrukts davon wenigstens 10% beträgt;
b) falls die variable Immunglobulin-Einzeldomäne und/oder das Konstrukt davon aus nicht mehr als 300 Aminosäureresten besteht, wird die Zuführung der variablen Immunglobulin-Einzeldomäne und/oder des Konstrukts davon an den systemischen Kreislauf mit einer Bioverfügbarkeit (im Vergleich zu i.v.-Injektion) erzielt, die nach Verabreichung einer Einzeldosis der variablen Immunglobulin-Einzeldomäne und/oder des Konstrukts davon wenigstens 7,5% beträgt; oder
c) falls die variable Immunglobulin-Einzeldomäne und/oder das Konstrukt davon aus nicht mehr als 450 Aminosäureresten besteht, wird die Zuführung der variablen Immunglobulin-Einzeldomäne und/oder des Konstrukts davon an den systemischen Kreislauf mit einer Bioverfügbarkeit (im Vergleich zu i.v.-Injektion) erzielt, die nach Verabreichung einer Einzeldosis der variablen Immunglobulin-Einzeldomäne und/oder des Konstrukts davon wenigstens 5% beträgt.

4. Variable Immunglobulin-Einzeldomäne und/oder Konstrukt davon zur Verwendung gemäß Anspruch 1 oder Anspruch 2, wobei die terminale Halbwertszeit der variablen Immunglobulin-Einzeldomäne und/oder des Konstrukts davon im systemischen Kreislauf länger als 5 Stunden ist.

5. Variable Immunglobulin-Einzeldomäne und/oder Konstrukt davon zur Verwendung gemäß irgendeinem der Ansprüche 1 bis 4, wobei die variable Immunglobulin-Einzeldomäne und/oder das Konstrukt davon eine V_{HH}, eine humanisierte V_{HH} und/oder ein Konstrukt davon ist.

6. Variable Immunglobulin-Einzeldomäne und/oder Konstrukt davon zur Verwendung gemäß irgendeinem der Ansprüche 1 bis 5, wobei die variable Immunglobulin-Einzeldomäne und/oder das Konstrukt davon ausgewählt ist aus der Gruppe einer V_{HH}, einer humanisierten V_{HH}, eines Konstrukts, bestehend aus zwei V_{HH}s oder humanisierten V_{HH}s, gerichtet gegen die gleichen oder unterschiedliche Antigene, optional verknüpft durch einen Linker; und eines Konstrukts, bestehend aus 3 V_{HH}s oder humanisierten V_{HH}s, gerichtet gegen die gleichen oder unterschiedliche Antigene, optional verknüpft durch einen Linker.

## Revendications

1. Domaine variable unique d'immunoglobuline et/ou construction de celui-ci, dirigé(e) contre au moins un antigène pour son utilisation dans un procédé de traitement par la distribution d'une quantité efficace d'un domaine variable unique d'immunoglobuline et/ou d'une construction de celui-ci dans la circulation systémique d'un être humain par voie pulmonaire; dans lequel le procédé comprend l'étape d'administration dudit domaine variable unique d'immunoglobuline et/ou de ladite construction de celui-ci dans les tissus pulmonaires à des intervalles posologiques d'une fois par jour ou des intervalles plus longs.

2. Domaine variable unique d'immunoglobuline et/ou construction de celui-ci pour son utilisation selon la revendication 1, dans lequel le procédé d'administration comprend l'étape de formation d'un aérosol comprenant ledit domaine variable unique d'immunoglobuline et/ou ladite construction de celui-ci par un dispositif inhalateur approprié.

3. Domaine variable unique d'immunoglobuline et/ou construction de celui-ci pour son utilisation selon la revendication 1 ou la revendication 2, dans lequel la distribution dudit domaine variable unique d'immunoglobuline et/ou de ladite construction de celui-ci dans la circulation systémique est obtenue avec une biodisponibilité :
a) dans le cas où le domaine variable unique d'immunoglobuline et/ou la construction de celui-ci consiste en 150 résidus d'acides aminés au maximum, la distribution dudit domaine variable unique d'immunoglobuline et/ou de ladite construction de celui-ci dans la circulation systémique est obtenue avec une biodisponibilité (par rapport à une injection i.v.) qui est d'au moins 10 % après l'administration d'une dose unique dudit domaine variable unique d'immunoglobuline et/ou de ladite construction de celui-ci ;
b) dans le cas où le domaine variable unique d'immunoglobuline et/ou la construction de celui-ci consiste en 300 résidus d'acides aminés au maximum, la distribution dudit domaine variable unique d'immunoglobuline et/ou de ladite construction de celui-ci dans la circulation systémique est obtenue avec une biodisponibilité (par rapport à une injection i.v.) qui est d'au moins 7,5% après l'administration d'une dose unique dudit domaine variable unique d'immunoglobuline et/ou de ladite construction de celui-ci ; ou
c) dans le cas où le domaine variable unique d'immunoglobuline et/ou la construction de celui-ci consiste en 450 résidus d'acides aminés au maximum, la distribution dudit domaine variable unique d'immunoglobuline et/ou de ladite construction de celui-ci dans la circulation systémique est obtenue avec une biodisponibilité (par rapport à une injection i.v.) qui est d'au moins 5% après l'administration d'une dose unique dudit domaine variable unique d'immunoglobuline et/ou de ladite construction de celui-ci.

4. Domaine variable unique d'immunoglobuline et/ou construction de celui-ci pour son utilisation selon la revendication 1 ou la revendication 2, dans lequel la demi-vie terminale dudit domaine variable unique d'immunoglobuline et/ou de ladite construction de celui-ci dans la circulation systémique est de plus de 5 heures.

5. Domaine variable unique d'immunoglobuline et/ou construction de celui-ci pour son utilisation selon l'une quelconque des revendications 1 à 4, dans lequel ledit domaine variable unique d'immunoglobuline et/ou ladite construction de celui-ci est un V_{HH}, un V_{HH} humanisé et/ou une construction de ceux-ci.

6. Domaine variable unique d'immunoglobuline et/ou construction de celui-ci pour son utilisation selon l'une quelconque des revendications 1 à 5, dans lequel ledit domaine variable unique d'immunoglobuline et/ou ladite construction de celui-ci est choisi(e) dans le groupe comprenant un V_{HH}, un V_{HH} humanisé, une construction consistant en deux V_{HH} ou V_{HH} humanisés dirigés contre les antigènes identiques ou différents reliés éventuellement par un segment de liaison ; et une construction consistant en 3V_{HH} ou V_{HH} humanisés dirigés contre les antigènes identiques ou différents éventuellement reliés par un segment de liaison.
